# EUROPEAN PATENT APPLICATION

(11) **EP 4 850 629 A2**
(43) Date of publication of application: **09.12.2026**
(21) Application number: 26213240.0
(22) Date of filing: 22.06.2023
(51) Int. Cl.: A61B 90/00

(54) **SURGICAL SAGITTAL BLADE CARTRIDGE**

(30) Priority: 22.06.2022 US 202263354477 P
(62) Document number(s) of the earlier application(s) in accordance with Art. 76 EPC: 23741463.6 / 4 543 322
(71) Applicant: Stryker European Operations Limited, Carrigtwohill, Co. Cork T45 HX08 (IE)
(72) Inventor: MEEHAN, Brian, Dublin 9, D09A2W4 (IE); GILHOOLEY, Séamus, Athenry, H65YF10 (IE); MCEWAN, Gregory Craig, Mattawan, MI, 49071 (US); SHIELS, Paul, Albuquerque, New Mexico, 87110 (US); TIMMER, Jeffrey, Kalamazoo, MI, 49009 (US)
(74) Representative: Röthinger, Rainer

(57) **Abstract**

A saw blade cartridge (110, 210, 310) for use with a surgical device (14) guided by a navigation system is presented. The saw blade cartridge (110, 210, 310) comprises a guide bar (111, 211, 311) including a distal portion and a proximal portion. The guide bar (111, 211, 311) comprises a first plate (112, 212, 312) defining a first aperture (130A, 330A, 330B, 330C); a second plate (114, 214, 314) defining a second aperture (130B, 330D, 330E, 330F); and an inner plate (116, 216, 316) disposed partially between the first and the second plates (112, 212, 312, 114, 214, 314), the inner plate defining a pivot surface (158, 258, 358). The first and second apertures (130A, 330A, 330B, 330C, 130B, 330D, 330E, 330F) are configured to facilitate determination of a position and/or orientation of the guide bar (111, 211, 311) with a navigation system (32). A blade (170, 270, 370) extends distally from the distal portion of the guide bar (111, 211, 311) and comprises a blade body (172, 272, 372) including a proximal end configured to abut the pivot surface (158, 258, 358). The first plate and second plate (112, 212, 312, 114, 214, 314) are positioned such that the first aperture and second aperture (130A, 330A, 330B, 330C, 130B, 330D, 330E, 330F) are concentrically aligned.

## Description

### CROSS-REFERENCE TO RELATED APPLICATIONS

The present application which claims priority to and all the benefits of U.S. Provisional Patent Application No. 63/354,477, filed on June 22, 2022, the entire contents of which are expressly incorporated herein by reference.

### BACKGROUND

A sagittal saw blade is a surgical saw with a head that pivots around an axis that is perpendicular to the blade. PCT Pub. No. WO 2006/017066A2/US Pat. No. 7,497,860, and PCT Pub. No. WO2016/182981A2/ US Pat. No. 10,687,823, the contents of which are each incorporated herein by reference, each disclose a sagittal saw blade cartridge. A sagittal saw blade cartridge includes a static guide bar and a blade head. The guide bar is an elongated member that is releasably attached to the handpiece, the saw that actuates the cartridge. The blade head is pivotally mounted to the guide bar and has teeth that extend forward from the guide bar. One or more drive links extend from the blade head towards the proximal end of the guide bar. The drive links are reciprocated back and forth by a drive assembly internal to the saw. The reciprocation of the drive links causes the blade head to pivot back and forth. The pivoting of the blade head is what enables the teeth to cut the tissue against which the blade head is pressed. Sometimes, this type of cartridge is referred to as an oscillating tip saw blade cartridge.

An advantage of the sagittal blade cartridge is that the only portion of the cartridge that pivots is the distally located blade head. By way of comparison, a conventional sagittal saw blade pivots from its point of attachment to the saw to which the blade is attached. A cartridge, when actuated, vibrates less in the hands of the surgeon holding the handpiece and/or a robotic arm. Also, it is common practice to use a cutting guide to properly position a sagittal saw blade relative to the tissue the blade is intended to cut. When a conventional blade is actuated, the oscillating movement of the blade imposes significant wear on the surfaces of the cutting guide defining the slot in which the blade is seated. The guide bar of a surgical sagittal blade cartridge only minimally moves in this slot. Thus, by using a cartridge, instead of a conventional blade, less of the material forming the cutting guide is rubbed off the guide.

When either a conventional sagittal blade or a sagittal blade cartridge advances through bone, the blade head is exposed to resistance and/or curved, angled, or shape surfaces. This resistance can be appreciable when the cut has a depth of 5 cm or more. Often the bone located adjacent the underside of the cartridge is more resistive to cutting than the bone located immediately above the cartridge. Similarly, the curve, angle, and/or shape of the bones surface can cause the sagittal blade cartridge to deflect in a certain direction. The cartridge, like most mechanical devices, will when advanced forward, advance along the path of least resistance. Since the bone above the cartridge can be less resistance to cutting than the bone below the cartridge, a cartridge when advanced, can flex upwardly out of the plane of the desired cut. This upwardly flexing or drifting of the blade is known as skiving. There can also be situations when owing to the density of the bone and to the shape of the bone as the point that the device enters, the cartridge, when advanced, flexes below the plane of the cut. This type of flexure or drifting of the blade is known as diving.

Regardless of the direction the blade flexes or drifts, it is undesirable. This is because a sagittal saw blade cartridge is typically used to remove bone so an artificial implant can be fitted in the space previously occupied by the removed bone. An implant is formed with surfaces designed to precisely seat against the complementary surfaces of the bone against which the implant is mounted. If the cut does not leave the bone with surfaces that have the desired shape, the results of the implant fitting procedure may be less than optimal.

In theory, one could increase the rigidity of a surgical sagittal blade cartridge by increasing the thickness of the guide bar. It should be appreciated that the slots of cutting guide through which the cartridge is inserted tend to be relatively narrow. Often the height of this slot is around 1.5 mm or less. This height limit imposes a limit of the thickness of the cartridge guide bar that can be inserted in this slot. Furthermore, if thickness of the guide bar is increased, by extension it is necessary to increase the thickness of the cut that will be formed by the cartridge. Increasing cut thickness can also lead to the cartridge leaving a cut surface that does not have the desired degree of planar smoothness. Increasing guide bar thickness is therefore typically not a viable solution for reducing the incidence of cartridge flexure. In robotic application cut guide thickness is not a limiting factor, but increasing the guide bar thickness thus requires an increase in the blade tip thickness, which also increase the amount of bone to be removed (more work to be done) which can be undesirable.

### SUMMARY OF THE INVENTION

This invention is related to a new and useful surgical sagittal blade cartridge. The surgical sagittal blade cartridge includes a guide bar designed to resist deformation when subjected to uneven resistance.

The surgical sagittal blade cartridge of this invention includes a guide bar that is formed with three plates. There is a first plate (top plate) and a second plate (bottom plate) opposite the top plate. Between the first and second plates there is an third plate (inner plate). The inner plate is formed to have a curved head. The curved head functions as the boss around which the cartridge blade head pivots
This Summary introduces a selection of concepts in a simplified form that are further described below in the Detailed Description below. This Summary is not intended to limit the scope of the claimed subject matter nor identify key features or essential features of the claimed subject matter.

In one aspect, a saw blade cartridge for use with a surgical device is disclosed. The saw blade cartridge also includes a guide bar may include:. The cartridge also includes a first plate and a second plate, each of the first plate and the second plate having a distal portion and a proximal portion. The cartridge also includes an inner plate at least partially disposed between the first and the second plates, the inner plate defining a pivot surface. The cartridge also includes a blade at least partially disposed between the first and the second plates, the blade may include:. The cartridge also includes a blade body including a proximal end and a distal end. The cartridge also includes a blade head disposed on the distal end of the blade body, the blade head formed with teeth, where the proximal end of the blade body abuts the pivot surface. The cartridge also includes where the proximal portion of each of the first and second plates each define a perimeter edge. The cartridge also includes where a portion of the inner plate extends beyond the perimeter edge defined by the proximal portion of at least one of the first and second plates.

In a second aspect, a saw blade cartridge of a surgical device is disclosed. The saw blade cartridge also includes a guide bar may include:. The cartridge also includes a first plate and a second plate, each of the first plate and the second plate having a distal portion and a proximal portion. The cartridge also includes an inner plate disposed partially between the first and the second plates. The cartridge also includes where the guide bar defines a pivot surface. The cartridge also includes a blade at least partially disposed between the first and the second plates, the blade may include:. The cartridge also includes a blade body including a proximal end and a distal end. The cartridge also includes a blade head disposed on the distal end of the blade body, the blade head formed with teeth, where the proximal end of the blade body abuts the pivot surface. The cartridge also includes where the proximal portion of each of the first and second plates each define a perimeter edge. The cartridge also includes where a portion of the inner plate extends beyond the perimeter edge defined by the proximal portion of at least one of the first and second plates.

In a third aspect, a saw blade cartridge a surgical device and a navigation system is disclosed. The saw blade cartridge also includes a guide bar including a distal portion and a proximal portion, the guide bar may include:. The cartridge also includes a first plate. The cartridge also includes a second plate. The cartridge also includes an inner plate positioned between the first and the second plates. The cartridge also includes a blade at least partially disposed between the first and the second plates of the guide bar, the blade may include:. The cartridge also includes a blade body including a proximal end and a distal end, the proximal end of the blade body abuts a pivot defined by the guide bar. The cartridge also includes a blade head disposed on the distal end of the blade body, the blade head formed with teeth. The cartridge also includes at least three indicia disposed on an outer surface of one of the first plate or the second plate to define a plane of the guide bar within a coordinate system of the navigation system.

In a fourth aspect, a saw blade cartridge a surgical device and a navigation system is disclosed. The saw blade cartridge also includes a guide bar including a distal portion and a proximal portion, the guide bar may include: a first plate; a second plate; and an inner plate disposed partially between the first and the second plates, the inner plate defining a pivot surface. The cartridge also includes a blade at least partially disposed between the first and the second plates, the blade may include: a blade body including a proximal end and a distal end, the proximal end of the blade body abuts the pivot surface; and a blade head disposed on the distal end of the blade body, the blade head formed with teeth. The cartridge also includes at least three openings defined in one of the first plate or the second plate, the at least three of the openings to provide access to an outer surface of the inner plate at the location of each of the at least three openings.

In a fifth aspect, a saw blade cartridge for a surgical device is disclosed. The saw blade cartridge also includes a guide bar including a distal end and a proximal end, the guide bar may include: a first plate; a second plate; and an inner plate disposed partially between the first and the second plates, the inner plate defining a pivot surface; a recess defined in the proximal end of at least one of the first plate, the second plate, or the inner plate. The cartridge also includes a blade at least partially disposed between the first and the second plates, the blade may include: a blade body including a proximal end and a distal end, the proximal end of the blade body abuts the pivot surface; and a blade head disposed on the distal end of the blade body, the blade head formed with teeth.

In a sixth aspect, a surgical saw system is disclosed. The surgical saw system also includes a surgical device including a mount, the mount defining a mount surface, and the mount may include: a first wall and a second wall extending above the mounting surface, the first and second wall positioned on opposing sides of a longitudinal axis of the mount; the first and second wall are tapered relative to the longitudinal axis of the mount; at least one tab disposed on each of the first and second walls, each of the at least one tabs positioned above and extending over the mounting surface. The system also includes a saw blade cartridge removably couplable to the mount of the surgical device, the saw blade cartridge may include: a guide bar including a distal portion and a proximal portion, the guide bar may include: a first plate; a second plate; and an inner plate disposed partially between the first and the second plates, where the proximal portion of each of the first and second plates each define a perimeter edge, where a portion of the proximal portion of the inner plate extends beyond the perimeter edge defined by the proximal portion of at least one of the first and second plates. The system also includes where each of the first and second walls engage the perimeter edge of at least one of the first plate or the second plate to facilitate alignment of the saw blade cartridge along the longitudinal axis and each of the at least one tabs engages a surface of the portion of the proximal portion of the inner plate that extends beyond the perimeter edge defined by the proximal portion of at least one of the first and second plates.

In a seventh aspect, a method of confirming a position and/or orientation of a surgical saw blade with a navigation system is disclosed. The method also includes positioning an instrument including a first tracking device such that distal end of the instrument is disposed within a first opening of the plurality of openings and abuts the surface of the inner plate. The method also includes repositioning the instrument such that distal end of the instrument is disposed within a second opening of the plurality of openings and abuts the surface of the inner plate. The method also includes determining an actual position and orientation of the surgical saw blade based on at least the position of the first and second openings as identified using the instrument. The method also includes determining an expected position and orientation of the surgical saw blade based on predetermined geometrical data corresponding to the relationship between a mounting feature of a surgical manipulator and a position of the plurality of openings in either the first plate or the second plate of the surgical saw blade. The method also includes comparing the expected position and orientation of the plurality of openings with the actual position and orientation of the plurality of openings.

In an eighth aspect, a method of confirming a position and/or orientation of a surgical saw blade within a known coordinate system defined by a navigation system is disclosed. The method also includes manipulating an instrument including a first tracking device such that a distal end of the instrument contacts each of the plurality of markings on the outer surface of the guide bar. The method also includes repositioning the instrument such that distal end of the instrument contacts a marking of the plurality of markings on the outer surface of the guide bar. The method also includes determining an actual position and orientation of each of the markings within the known coordinate system based on at least the pose of the instrument when contacting each of the markings. The method also includes determining an expected position and orientation of the markings within the known coordinate system based on predetermined geometrical data. The method also includes comparing the expected position and orientation of each of the plurality of markings with the actual position and orientation of each of the plurality of markings. The method also includes assigning a modified position based on the deviation between the expected position and orientation of each of the plurality of markings based on the predetermined geometrical data and the actual position. The method also includes defining a plane of the surgical saw blade within the known coordinate system based on the modified position.

In a ninth aspect, a method of operating a saw blade with a navigation system is disclosed. The method also includes mounting a surgical saw blade to a surgical manipulator, the surgical manipulator including a first tracking device. The method also includes contacting each of the at least three indicia on the surgical saw blade with a distal end of an instrument, the instrument including a second tracking device. The method also includes tracking the position of the first tracking device and the second tracking device with a localizer of the navigation system as the distal end of the instrument contacts each of the at least three indicia on the surgical saw blade to define an actual position of each of the at least three indicia relative to a known coordinate system. The method also includes performing a mathematical fit of the actual position of each of the at least three indicia and an expected position for each of the at least three indicia relative to the known coordinate system. The method also includes determining a hybrid position within a known coordinate system based on the product of the mathematical fit for each of the at least three indicia. The method also includes defining a plane of the surgical saw blade based on the hybrid position for each of the at least three indicia within the known coordinate system.

In a tenth aspect, a method of registering a saw blade within a known coordinate system of a navigation system is disclosed. The method also includes mounting a surgical saw blade to a surgical manipulator, the surgical manipulator including a first tracking device. The method also includes manipulating an instrument including a second tracking device such that a distal end of the instrument pass through each of the at least three apertures and contacts the inner plate exposed by each of the apertures. The method also includes tracking the position of the first tracking device and the second tracking device with a localizer of the navigation system as the distal end of the surgical instrument contacts the inner plate through each of the at least three aperture on the surgical saw blade to define an actual position of each of the at least three apertures relative to the first tracking device of the surgical manipulator. The method also includes defining an actual position of a portion of the inner plate exposed by each of the at least three apertures within the known coordinate system based on the tracked position of the instrument when contacting the inner plate through each of the at least three apertures. The method also includes comparing an expected position of the portion of the inner plate exposed by each of the at least three apertures within the known coordinate system to the actual position of the portion of the inner plate exposed by each of the at least three apertures within the known coordinate system. The method also includes modifying the position of the portion of the inner plate exposed by each of the at least three apertures within the known coordinate system based on the deviation between the actual position and the expected position of the portion of the inner plate exposed by each of the at least three apertures within the known coordinate system. The method also includes defining a plane of the surgical saw blade based on the modifying the position of the portion of the inner plate exposed by each of the at least three apertures within the known coordinate system.

In an eleventh aspect, a method of registering a saw blade within a known coordinate system of a navigation system is disclosed. The method also includes mounting a surgical saw blade to a surgical manipulator, the surgical manipulator including a first tracking device. The method also includes contacting each of the at least three reference features on the surgical saw blade with a distal end of a surgical instrument, the surgical instrument including a second tracking device. The method also includes tracking the position of the first tracking device and the second tracking device with a localizer of the navigation system as the distal end of the surgical instrument contacts each of the at least three reference features on the surgical saw blade to define an actual position of each of the at least three reference features relative to the first tracking device of the surgical manipulator. The method also includes performing a mathematical fit based on the actual position of each of the at least three reference features and to an expected position for each of the at least three reference features relative to the known coordinate system. The method also includes determining a hybrid position of the at least three reference features within the known coordinate system based on the mathematical fit. The method also includes defining a plane of the surgical saw blade based on the hybrid position of the at least three reference features within the known coordinate system.

In a twelfth aspect, a method of controlling a hand-held medical robotic system for use with a surgical saw blade is disclosed. The method also includes registering the surgical saw blade to a navigation system, the step of registering the surgical saw blade may include manipulating an instrument including a first tracking device to insert a distal end of the instrument within each of the plurality of openings to contact the surface of the inner plate at each location of the plurality of openings to define a plane of the surgical saw blade within a first coordinate system relative to a second tracking device that is coupled to the hand-held medical robotic system. The method also includes navigating the surgical saw blade based on the position and orientation of the second tracking device.

In a thirteenth aspect, a method of controlling a hand-held medical robotic system for use with a surgical saw blade is disclosed. The method also includes registering the surgical saw blade to a navigation system, the step of registering the surgical saw blade may include manipulating an instrument including a first tracking device to use a distal end of the instrument to contact the surface of the guide bar to trace the optical marking extending between the two reference features to define a plane of the surgical saw blade within a first coordinate system relative to a second tracking device that is coupled to the hand-held medical robotic system. The method also includes navigating the surgical saw blade based on the position and orientation of the second tracking device.

In a fourteenth aspect, a surgical saw system is disclosed. The surgical saw system also includes a surgical device including a mount, the mount defining a mount surface, and the mount may include:. The system also includes a first wall and a second wall extending above the mounting surface, the first and second wall positioned on opposing sides of a longitudinal axis of the mount. The system also includes the first and second wall, each having a proximal portion and a distal portion, the proximal portion oriented at a first interior taper angle relative to the longitudinal axis of the mount and the distal portion orient at a second interior taper angle relative to the longitudinal axis of the mount. The system also includes where the second interior taper angle is greater than the first interior taper angle relative to the longitudinal axis of the mount rather than the first taper. The system also includes at least one tab disposed on each of the first and second walls, each of the at least one tabs positioned above and extending over the mounting surface. The system also includes a saw blade cartridge removably couplable to the mount of the surgical device, the saw blade cartridge may include:. The system also includes a guide bar including a distal portion and a proximal portion, the guide bar may include:. The system also includes a first plate. The system also includes a second plate. The system also includes an inner plate disposed partially between the first and the second plates. The system also includes where one of the first and second plates defines an outer edge of a proximal portion of the guide bar. The system also includes where the proximal portion of each of the first and second walls engage the outer edge of the proximal portion of the guide bar to facilitate alignment of the saw blade cartridge along the longitudinal axis. The system also includes where the distal portion of the wall is spaced away from the outer edge of the proximal portion of the guide bar. The system also includes an under surface of each of the at least one tabs is disposed over a top surface of the inner plate. In some of the implementations described above, the guide bar may further defines a longitudinal axis extending between the distal portion and the proximal portion of the guide bar; and wherein the outer perimeter of the proximal portion of the guide bar defines a first taper angle relative to the longitudinal axis between a first point and a second point on the outer perimeter; and wherein the outer perimeter defines a second taper angle relative to the longitudinal axis between the second point and a third point on the outer perimeter, the first point being closer to a proximal end of the cartridge than the third point and the second point being disposed between the first point and the third point.

In some of the implementations described above, the outer edge of the proximal portion of the guide bar may define a third taper angle relative to the longitudinal axis.

In some of the implementations described above, the pivot surface may comprise an arcuate surface, and the proximal end of the blade body defining a recess that abuts the pivot surface, such that the blade body is configured to pivot about the pivot surface defined by the inner plate when the blade body is actuated.

In some of the implementations described above, at least one of the plates may define a reference feature configured to facilitate determination of a position and/or orientation of the surgical saw blade cartridge within a known coordinate system of a navigation system.

In some of the implementations described above, the reference feature is selected from the group comprising an optical marking, a divot, or an aperture, the reference feature comprises a plurality of laser markings, and wherein at least one of the plurality of laser markings is positioned on each side of the longitudinal axis of the surgical blade cartridge.

In some of the implementations described above, at least one of the first plate or the second plate is shaped to provide access to the reference feature of the inner plate, and each of the reference features is disposed on a surface of the inner plate that is exposed by an aperture in one of the outer plates.

In some of the implementations described above, the location of the indicia and/or reference features defined on the guide bar may be configured to identify the saw blade cartridge to the navigation system, including one or more characteristics of the saw blade cartridge, the characteristic including at least one of the following: a type of the blade, a blade thickness of the blade head, a tooth configuration of the blade head, a length of the guide bar, and/or a width of the guide bar.

In some of the implementations described above, the surgical manipulator and/or the surgical device is one of a hand-held tool, a robotic arm, or a hand- held robot.

In some of the implementations described above, the guide bar is further formed such that a portion of the inner plate is exposed by one of the first and second outer plates, the exposed portion configured to engage the mount of the surgical manipulator.

In some of the implementations described above, the proximal portion of the surgical saw blade cartridge may define an outer perimeter, a portion of the outer perimeter may serve a contact point to removably engage the surgical manipulator such that the contact point assists in aligning the saw blade cartridge with the surgical device.

In some of the implementations described above, the blade head may define one or more apertures or cut-outs for allowing debris to exit and/or to be used as a reference point when manufacturing the blade.

In a fifteenth aspect, a surgical saw system is disclosed. The surgical saw system also includes a surgical device including a mount, the mount defining a mount surface, and the mount may include: a first wall and a second wall extending above the mounting surface, the first and second wall positioned on opposing sides of a longitudinal axis of the mount; the first and second wall are tapered relative to the longitudinal axis of the mount; at least one tab disposed on each of the first and second walls, each of the at least one tabs positioned above and extending over the mounting surface. The system also includes a saw blade cartridge removably couplable to the mount of the surgical device, the saw blade cartridge may include:. The system also includes a guide bar including a distal portion and a proximal portion, the guide bar may include:. The system also includes a first plate and a second plate, at least one of the first and second plate defining a first outer edge of the proximal portion of the guide bar. The system also includes an inner plate disposed partially between the first and the second plates, the inner plate defining a pivot surface and a second outer edge of a portion of the proximal portion of the guide bar. The system also includes where the second outer edge of the inner plate extends beyond the first outer edge defined by the first plate, the second outer edge configured to engage the first and second walls of the surgical device to assist in aligning the saw blade cartridge within the mount of the surgical device.

In a sixteenth aspect, a saw blade cartridge for use with a surgical device is disclosed. The saw blade cartridge also includes a guide bar including a distal portion and a proximal portion, the guide bar may include:. The cartridge also includes a first plate. The cartridge also includes an second plate disposed adjacent to the first plate, the second plate defining a pivot surface. The cartridge also includes a blade extending distally from the distal portion of the guide bar, the blade may include:. The cartridge also includes a blade body including a proximal end and a distal end. The cartridge also includes a blade head disposed on the distal end of the blade body, the blade head formed with teeth, where the proximal end of the blade body abuts the pivot surface. The cartridge also includes where the proximal portion defines a proximal end. The cartridge also includes where the first plate defines a first outer edge. The cartridge also includes where the second plate defines a second outer edge, the second outer edge extends beyond the first outer edge defined by the first plate and where the second outer edge tapers outwardly moving distally from the proximal end of the proximal portion toward the distal end of the proximal portion such that the second outer edge assists in aligning the saw blade cartridge with the surgical device.

In a seventeenth aspect, a saw blade cartridge for use with a surgical device is disclosed. The saw blade cartridge also includes a guide bar including a distal portion and a proximal portion, the guide bar may include:. The cartridge also includes a first plate defining a first outer edge of the proximal portion. The cartridge also includes a second plate disposed adjacent to the first plate, the second plate defining a pivot surface and a second outer edge of the proximal portion of the guide bar. The cartridge also includes where the second outer edge extends beyond the first outer edge defined by the first plate, the second outer edge configured to engage the surgical device to assist in aligning the saw blade cartridge with the surgical device. The cartridge also includes a blade extending distally of the distal portion of the guide bar, the blade may include:. The cartridge also includes a blade body including a proximal end configured to abut the pivot surface.

In a eighteenth aspect, a saw blade cartridge for use with a surgical device guided by a navigation system is disclosed. The saw blade cartridge also includes a guide bar including a distal portion and a proximal portion, the guide bar may include:. The cartridge also includes a first plate defining a first aperture. The cartridge also includes a second plate defining a second aperture. The cartridge also includes an inner plate disposed partially between the first and the second plates, the inner plate defining a pivot surface. The cartridge also includes where the first and second apertures are configured to facilitate determination of a position and/or orientation of the guide bar with a navigation system. The cartridge also includes a blade extending distally from the distal portion of the guide bar, the blade may include:. The cartridge also includes a blade body including a proximal end configured to abut the pivot surface. The cartridge also includes where the proximal portion defines a proximal end. The cartridge also includes where the first plate and second plate are positioned such that the first aperture and second aperture are concentrically aligned.

In a nineteenth aspect, a saw blade cartridge for use with a surgical device including a tracker identifiable by/associated with a navigation system is disclosed. The saw blade cartridge also includes a guide bar having a distal portion and a proximal portion, the guide bar may include: a first plate defining a first reference feature in the distal portion and a second reference feature in the proximal portion; a second plate defining a third reference feature in the distal portion and a fourth reference feature in the proximal portion; and an inner plate at least partially disposed between the first and the second plates, where the combination of reference features are usable to register the saw blade cartridge within the known coordinate system defined by the navigation system relative to the tracker of the surgical device. The cartridge also includes a blade at least partially disposed between the first and the second plates, the blade may include:. The cartridge also includes a blade body including a proximal end and a distal end. The cartridge also includes a blade head disposed on the distal end of the blade body, the blade head formed with teeth.

In a twentieth aspect, a surgical saw assembly for use with a navigation system is disclosed. The surgical saw assembly also includes a surgical instrument/device including an instrument reference feature. The assembly also includes a tracker disposed on the surgical instrument, the tracker identifiable by and/or associated with the navigation system. The assembly also includes a saw blade cartridge removably couplable to the surgical device, the saw blade cartridge may include: a guide bar having a distal portion and a proximal portion, the guide bar may include: a first plate defining a first reference feature in the distal portion and a second reference feature in the proximal portion; a second plate defining a third reference feature in the distal portion and a fourth reference feature in the proximal portion; and an inner plate at least partially disposed between the first and the second plates, the inner plate defining a pivot surface. The assembly also includes where the combination of reference features are usable to facilitate registration of the saw blade cartridge within a known coordinate system defined by the navigation system relative to the tracker of the surgical device. a blade extending from the distal portion of the guide bar.

In a twenty-first aspect, a method of registering a saw blade within a known coordinate system of a navigation system is disclosed. The method also includes mounting a surgical saw blade to a surgical manipulator, the surgical manipulator including a first tracking device. The method also includes contacting each of the at least three reference features on the surgical saw blade with a distal end of a surgical instrument, the surgical instrument including a second tracking device. The method also includes tracking the position of the first tracking device and the second tracking device with a localizer of the navigation system as the distal end of the surgical instrument contacts each of the at least three reference features on the surgical saw blade to define an actual position of each of the at least three reference features relative to the first tracking device of the surgical manipulator. The method also includes defining a plane of the surgical saw blade based on the actual position of the at least three reference features within the known coordinate system.

In a twenty-second aspect, a method of registering a saw blade within a known coordinate system of a navigation system is disclosed. The method also includes mounting a surgical saw blade to a surgical manipulator, the surgical manipulator including a first tracking device. The method also includes contacting each of at least two reference features on a first surface of the surgical saw blade with a distal end of a surgical instrument, the surgical instrument including a second tracking device. The method also includes contacting each of at least two reference features on a second surface of the surgical saw blade with the distal end of the surgical instrument. The method also includes tracking a position of the first tracking device and the second tracking device with a localizer of the navigation system as the distal end of the surgical instrument contacts each of the reference features on both the first and the second surfaces of the surgical saw blade to define an actual position of each of the reference features of the first and the second surfaces relative to the first tracking device of the surgical manipulator. The method also includes comparing the actual position of each of the reference features and a position of a manipulator reference feature within the known coordinate system to verify whether the surgical saw blade is properly mounted to the surgical manipulator.

In a twenty-third aspect, a method of registering a saw blade within a known coordinate system of a navigation system is disclosed. The method also includes mounting a surgical saw blade cartridge to a surgical manipulator, the surgical saw blade cartridge including a guide bar having a first plate disposed adjacent a second plate and a blade at least partially disposed between first and second plates and extending from a distal end of the guide bar, and the surgical manipulator including a first tracking device. The method also includes contacting each of at least two reference features on the first plate of the guide bar with a distal end of a surgical instrument, the surgical instrument including a second tracking device. The method also includes contacting each of at least two reference features on the second plate of the guide bar with the distal end of the surgical instrument, each of the reference features of the first plate being concentric with one of the reference features of the second plate. The method also includes tracking the position of the first tracking device and the second tracking device with a localizer of the navigation system as the distal end of the surgical instrument contacts each of the reference features on both the first and the second plates of the surgical saw blade cartridge to define an actual position of each of the reference features of the first and the second plates relative to the first tracking device of the surgical manipulator. The method also includes determining a thickness of the cartridge based on the actual position of the reference features on the first plate and the second plate. The method also includes providing an indication to the user based on the determined thickness of the cartridge.

In a twenty-fourth aspect, a method of registering a saw blade within a known coordinate system of a navigation system is disclosed. The method also includes mounting a surgical saw blade to a surgical manipulator, the surgical manipulator including a first tracking device. The method also includes contacting each of at least two reference features on a first surface of the surgical saw blade with a distal end of a surgical instrument, the surgical instrument including a second tracking device. The method also includes contacting each of at least two reference features on a second surface of the surgical saw blade with the distal end of the surgical instrument. The method also includes contacting at least one reference feature on the surgical manipulator. The method also includes tracking a position of the first tracking device and the second tracking device with a localizer of the navigation system as the distal end of the surgical instrument contacts each of the reference features on both the surgical saw blade and surgical manipulator to define an actual position of each of the reference features of the surgical saw blade relative to the reference features of the surgical manipulator. The method also includes defining a hybrid position for each of the at least one reference features of the surgical manipulator by performing a mathematical fit between the actual position of each of the reference features of the surgical saw blade relative to the at least one reference features of the surgical manipulator.

In a twenty-fifth aspect, a method of registering a surgical saw blade within a known coordinate system of a navigation system is disclosed. The method also includes mounting the surgical saw blade to a surgical manipulator, the surgical manipulator including a first tracking device. The method also includes contacting each of at least two reference features on a first surface of the surgical saw blade with a distal end of a surgical instrument, the surgical instrument including a second tracking device. The method also includes contacting each of at least two reference features on a second surface of the surgical saw blade with the distal end of the surgical instrument. The method also includes tracking a position of the first tracking device and the second tracking device with a localizer of the navigation system as the distal end of the surgical instrument contacts each of the reference features on both the first and the second surfaces of the surgical saw blade to define an actual position of each of the reference features of the first and the second surfaces relative to the first tracking device of the surgical manipulator. The method also includes defining a position and/or orientation of the surgical saw blade within the known coordinate system in one or more degrees of freedom based on a mathematical fit of the position of the at least two reference features of each of the first surface and the second surface.

In a twenty-sixth aspect, a method of registering a surgical saw assembly within a known coordinate system of a navigation system is disclosed. The method also includes mounting a surgical saw blade to a surgical manipulator, the surgical manipulator including a first tracking device. The method also includes contacting each of at least two reference features on a first surface of the surgical saw blade with a distal end of a surgical instrument, the surgical instrument including a second tracking device. The method also includes contacting each of at least two reference features on a second surface of the surgical saw blade with the distal end of the surgical instrument. The method also includes contacting a manipulator reference feature defined on the surgical manipulator with the distal end of the surgical instrument. The method also includes tracking a position of the first tracking device and the second tracking device with a localizer of the navigation system as the distal end of the surgical instrument contacts each of the reference features on both the first and the second surfaces of the surgical saw blade to define an actual position of each of the reference features of the first and the second surfaces and the manipulator reference feature relative to the first tracking device of the surgical manipulator. The method also includes defining a hybrid position of the manipulator reference feature within known coordinate system based on a comparison of the actual position of each of the reference features of the first and the second surfaces relative to the manipulator reference feature. The method also includes defining a position and/or orientation of the surgical saw assembly within the known coordinate system in at least one degree of freedom based on a mathematical fit of the position of the at least two reference features of each of the first surface and the second surface and the hybrid position of the manipulator reference feature.

In a twenty-seventh aspect, a method of registering a saw assembly within a known coordinate system of a navigation system is disclosed. The method also includes mounting a surgical saw blade to a surgical manipulator, the surgical manipulator including a first tracking device. The method also includes contacting each of at least two reference features on a first surface of the surgical saw blade with a distal end of a surgical instrument, the surgical instrument including a second tracking device. The method also includes contacting each of at least two reference features on a second surface of the surgical saw blade with the distal end of the surgical instrument. The method also includes contacting a manipulator reference feature defined on the surgical manipulator with the distal end of the surgical instrument. The method also includes tracking a position of the first tracking device and the second tracking device with a localizer of the navigation system as the distal end of the surgical instrument contacts each of the reference features on both the first and the second surfaces of the surgical saw blade to define an actual position of each of the reference features of the first and the second surfaces and the manipulator reference feature relative to the first tracking device of the surgical manipulator. The method also includes applying a first mathematical fit to the actual position of the reference features of the first and the second surfaces and the manipulator reference feature relative to one another. The method also includes defining a hybrid position of the manipulator reference feature based on the first mathematical fit. The method also includes applying a second mathematical fit to the actual position of the reference features of the first and the second surfaces and the hybrid position of the manipulator reference feature relative to one another. The method also includes defining position and/or orientation in one or more degrees of freedom of the surgical saw assembly within the known coordinate system based on the second mathematical fit.

In a twenty-eighth aspect, a method of verification of mounting of a surgical saw blade to a surgical manipulator is disclosed. The method also includes mounting a surgical saw blade to a surgical manipulator, the surgical manipulator including a first tracking device. The method also includes contacting each of at least two reference features on a first surface of the surgical saw blade with a distal end of a surgical instrument, the surgical instrument including a second tracking device. The method also includes contacting a manipulator reference feature defined on the surgical manipulator with the distal end of the surgical instrument. The method also includes applying a mathematical fit to the actual position of the reference features of the first surface and the manipulator reference feature relative to one another. The method also includes comparing an output of the mathematical fit to a threshold, where the output of the mathematical fit relative to the threshold is indicative of whether the surgical saw blade is properly mounted to the surgical manipulator.

In a twenty-ninth aspect, a method of verifying a registration of a surgical saw blade within a known coordinate system of a navigation system is disclosed. The method also includes mounting a surgical saw blade to a surgical manipulator, the surgical manipulator including a first tracking device. The method also includes contacting a first reference feature located on a proximal portion of a first plate of the surgical saw blade and contacting a second reference feature located on a distal portion of the first plate with a distal end of a surgical instrument, the surgical instrument including a second tracking device. The method also includes contacting a third reference feature located on a proximal portion of a second plate of the surgical saw blade and contacting a fourth reference feature located on a distal portion of the second plate with the distal end of the surgical instrument. The method also includes defining a first blade thickness based on a comparison of the location of the first reference feature of the first plate and the third reference feature of the second plate. The method also includes defining a second blade thickness based on a comparison of the location of the second reference feature of the first plate to the fourth reference feature of the second plate. The method also includes computing a deviation between the first blade thickness to the second blade thickness. The method also includes where the deviation the first blade thickness to the second blade thickness is indicative of deflecting the distal portion of the first and/or second plate when contacting either of the second or fourth reference features.

Implementations of one or more of the above aspects may optionally include one or more of the following features. The method where the step of defining the hybrid position for the at least one reference feature of the surgical manipulator within the known coordinate system based on the mathematical fit is such that the hybrid position for each of the reference features is different from the actual position in only one degree of freedom. The step of defining the hybrid position for the at least one reference feature of the surgical manipulator within the known coordinate system based on the mathematical fit cause the actual position of each of the at least one reference features of the surgical manipulator to be moved in only one degree of freedom. The method of any one to 123, may include: comparing the actual position of the at least one reference features of the manipulator to the actual position of each of the reference features of the surgical saw blade; and determining whether the surgical saw blade is properly mounted to the surgical manipulator based on a deviation the actual position of the at least one reference features of the manipulator to the actual position of each of the reference features of the surgical saw blade being within an accepted threshold/range. The method of any one to 123, may include: rejecting the registration of the surgical saw blade within the known coordinate system of a navigation system based on a deviation the actual position of the at least one reference features of the manipulator to the actual position of each of the reference features of the surgical saw blade being within an accepted threshold/range. The method of any one to 123, may include: comparing the hybrid position of the at least one reference features of the manipulator to the actual position of each of the reference features of the surgical saw blade; and determining whether the surgical saw blade is properly mounted to the surgical manipulator based on a deviation the hybrid position of the at least one reference features of the manipulator to the actual position of each of the reference features of the surgical saw blade being within an accepted threshold/range. The method of any one to 123, may include: rejecting the registration of the surgical saw blade within the known coordinate system of a navigation system based on a deviation the hybrid position of the at least one reference features of the manipulator to the actual position of each of the reference features of the surgical saw blade being within an accepted threshold/range. The accepted threshold for deviation between the hybrid position of the at least one reference features of the manipulator to the actual position of each of the reference features of the surgical saw blade is plus or minus one millimeter (+/- 1 mm.).

Implementations of one or more of the above aspects may also optionally include one or more of the following features. The method may include the step of contacting a manipulator reference feature defined on the surgical manipulator with the distal end of the surgical instrument; and defining a hybrid position of the manipulator reference feature within known coordinate system based on a comparison of the actual position of each of the reference features of the first and the second surfaces relative to the manipulator reference feature; and where the tool center point is further defined based on a mathematical fit of the position of the at least two reference features of each of the first surface and the second surface and the hybrid position of the manipulator reference feature. The step of defining the position and/or orientation of the surgical saw blade within the known coordinate system in one or more degrees of freedom may include defining the position and/or orientation of the surgical saw blade within the known coordinate system in up to six (6) degrees of freedom.

Any of the above aspects can be combined in full or in part. Any features of the above aspects can be combined in full or in part. Any of the above implementations for any aspect can be combined with any other aspect. Any of the above implementations can be combined with any other implementation whether for the same aspect or different aspect.

### BRIEF DESCRIPTION OF THE DRAWINGS

The invention is pointed out with particularity in the claims. The above and further features and benefits of this invention are understood from the following Detailed Description taken in conjunction with the accompanying drawings in which:
Figure 1 is a perspective view of a surgical system including a navigation system and a surgical saw blade coupled to a surgical manipulator.
Figure 2A is a perspective view of a first configuration of a surgical saw blade cartridge for use with the surgical system of Figure 1 and/or the surgical system of Figure 1.
Figure 2B is a top view of the first configuration of a surgical saw blade cartridge of Figure 2A.
Figure 2C is a bottom view of the first configuration of a surgical saw blade cartridge of Figure 2A.
Figure 2D is a side view of the first configuration of a surgical saw blade cartridge of Figure 2A.
Figure 2E is a zoomed perspective view of a proximal portion of the first configuration of a surgical saw blade cartridge of Figure 2A.
Figure 2F is a partially exploded view of the first configuration of a surgical saw blade cartridge of Figure 2A.
Figure 3A is a perspective view of a second configuration of a surgical saw blade cartridge for use with the surgical system of Figure 1.
Figure 3B is a top view of the second configuration of a surgical saw blade cartridge of Figure 3A.
Figure 3C is a bottom view of the second configuration of a surgical saw blade cartridge of Figure 3A.
Figure 3D is a side view of the second configuration of a surgical saw blade cartridge of Figure 3A.
Figure 3E is a top view of the second configuration of a surgical saw blade cartridge of Figure 3A, the surgical saw blade cartridge including additional reference features and/or markings.
Figure 3F is a partially exploded view of the second configuration of a surgical saw blade cartridge of Figure 3A.
Figure 4A is a top view of a saw blade cartridge including a first configuration of a blade head.
Figure 4B is a top view of a saw blade cartridge including a second configuration of a blade head.
Figure 5A is a top view of the distal portion of the saw blade cartridge of Figure 4A with the top plate removed to illustrate the first configuration of the blade head within of the surgical saw blade cartridge.
Figure 5B is a top view of the distal portion of the saw blade cartridge of Figure 4B with the top plate removed to illustrate the second configuration of the blade head within of the surgical saw blade cartridge.
Figure 6A is a perspective view of a surgical manipulator and a pointer of the surgical system of Figure 1, the surgical manipulator including a surgical saw blade cartridge with a plurality of reference features and a tool tracker, and the pointer including a pointer tracker.
Figure 6B is a partial sectional view of a distal end of the pointer of Figure 6A disposed in an aperture of the surgical saw blade cartridge, the distal end of the pointer contacting a reference feature on an inner layer of the surgical saw blade cartridge that is exposed by the aperture.
Figure 6C is a perspective view of a perspective view of a surgical manipulator and a pointer of the surgical system of Figure 1, the surgical manipulator including a surgical saw blade cartridge with an alternative configuration of a reference feature and a tool tracker, and the pointer including a pointer tracker.
Figure 6D is a partially exploded view of the surgical manipulator of Figure 6C including the surgical saw blade cartridge separated from a mount of the surgical manipulator to illustrate the alternative configuration of the reference feature on the surgical saw blade cartridge.
Figure 7A is a partially exploded view of a mount and a surgical saw blade cartridge of the surgical manipulator of Figure 6A.
Figure 7B is a top view of the surgical saw blade cartridge coupled to the mount of the surgical manipulator of Figure 6A.
Figure 7C is an enhanced top view of the surgical saw blade cartridge coupled to the mount of the surgical manipulator of Figure 7B, illustrating how the surgical saw blade cartridge engages the mount of the surgical manipulator.
Figure 7D is a rear view of the surgical saw blade cartridge coupled to the mount of the surgical manipulator of Figure 7B, illustrating how the surgical saw blade cartridge engages the mount of the surgical manipulator.
Figure 8 is a perspective view of a third configuration of a surgical saw blade cartridge for use with the surgical system of Figure 1 and/or the surgical manipulator of Figure 1.
Figure 9 is a partially exploded view of the third configuration of the surgical saw blade cartridge of Figure 8.
Figure 10 is a top view of the third configuration of the surgical saw blade cartridge of Figure 8.
Figure 11 is a bottom view of the third configuration of the surgical saw blade cartridge of Figure 8.
Figure 12 is a sectional view of the third configuration of the surgical saw blade cartridge of Figure 8.
Figure 13A is a zoomed sectional view of the third configuration of the surgical saw blade cartridge of Figure 12 illustrating an example reference feature and example configurations of a surgical instrument for touching off on the reference feature.
Figure 13B is a zoomed sectional view of the third configuration of the surgical saw blade cartridge of Figure 13A illustrating one of the surgical instruments touching off on the reference feature of the surgical blade.
Figure 14 is a perspective view of the surgical blade of Figure 8 including an alternate configuration of the reference features.
Figure 15 is a top view of the surgical saw blade cartridge of Figure 14.
Figure 16 is a bottom view of the surgical saw blade cartridge of Figure 14.
Figure 17 is a partially exploded view of the surgical saw blade cartridge of Figure 14.
Figure 18 is a perspective view of a fourth configuration of a surgical saw blade cartridge for use with the surgical system of Figure 1 and/or the surgical manipulator of Figure 1.
Figure 19 is a top exploded view of the fourth configuration of the surgical saw blade cartridge of Figure 18.
Figure 20 is a bottom view of the fourth configuration of the surgical saw blade cartridge of Figure 18.
Figure 21 is a partially exploded view of the fourth configuration of the surgical saw blade cartridge of Figure 18.
Figure 22 is a sectional view of the fourth configuration of the surgical saw blade cartridge of Figure 18.
Figure 23A is a zoomed sectional view of the fourth configuration of the surgical saw blade cartridge of Figure 18 illustrating an example reference feature and example configurations of a surgical instrument for touching off on the reference feature.
Figure 23B is a zoomed sectional view of the fourth configuration of the surgical saw blade cartridge of Figure 23A illustrating one of the surgical instruments touching off on the reference feature of the surgical blade.
Figure 24 is a flow chart illustrating a method of registering a surgical instrument with a navigation system.
Figure 25 is a flow chart illustrating an alternative method of registering a surgical instrument with a navigation system.
Figure 26 is a flow chart illustrating an alternative method of registering a surgical instrument with a navigation system.
Figure 27A is a flow chart illustrating an alternative method of registering a surgical saw blade with a navigation system including defining a plane of the surgical saw blade.
Figure 27B is a continuation of the flow chart of Figure 27A.
Figure 28 is a flow chart illustrating an alternative method of registering a surgical saw blade with a navigation system.
Figure 29 is a flow chart illustrating an alternative method of registering a surgical saw blade with a navigation system including defining a plane of the surgical saw blade.
Figure 30 is a flow chart illustrating an alternative method of registering a surgical saw blade with a navigation system.
Figure 31 is a flow chart illustrating a method of verifying a registration of a surgical saw blade with a navigation system.
Figure 32 is a flow chart illustrating an alternative method of registering a surgical saw blade with a navigation system.
Figure 33 is a flow chart illustrating an alternative method of registering a surgical saw blade with a navigation system.
Figure 34 is a flow chart illustrating an alternative method of registering a surgical saw blade with a navigation system.
Figure 35A is a flow chart illustrating an alternative method of registering a surgical saw blade with a navigation system.
Figure 35B is a continuation of the flow chart of Figure 35A.
Figure 36 is a flow chart illustrating a method of verifying a proper mounting of a surgical saw blade to a surgical manipulator.
Figure 37 is a flow chart illustrating a method of verifying a registration of a surgical saw blade with a navigation system.

### DETAILED DESCRIPTION

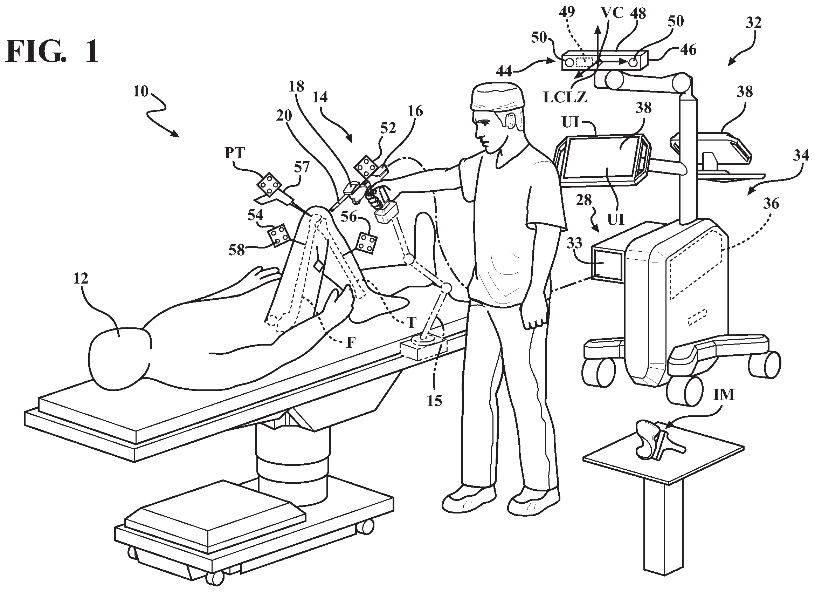

Referring to Figure 1, a surgical system 10 is illustrated. The surgical system 10 is shown performing a total knee procedure on a patient 12 to resect portions of a femur F and tibia T of the patient 12 so that the patient 12 can receive a total knee implant IM. The surgical system 10 may be used to perform other types of surgical procedures, including procedures that involve hard/soft tissue removal, or other forms of treatment. For example, treatment may include cutting tissue, coagulating tissue, ablating tissue, stapling tissue, suturing tissue, or the like. In some examples, the surgical procedure involves knee surgery, hip surgery, shoulder surgery, spine surgery, and/or ankle surgery, and may involve removing tissue to be replaced by surgical implants, such as knee implants, hip implants, shoulder implants, spine implants, and/or ankle implants. The surgical system 10 and techniques disclosed herein may be used to perform other procedures, surgical or non-surgical, and may be used in industrial applications or other applications where surgical systems are utilized.

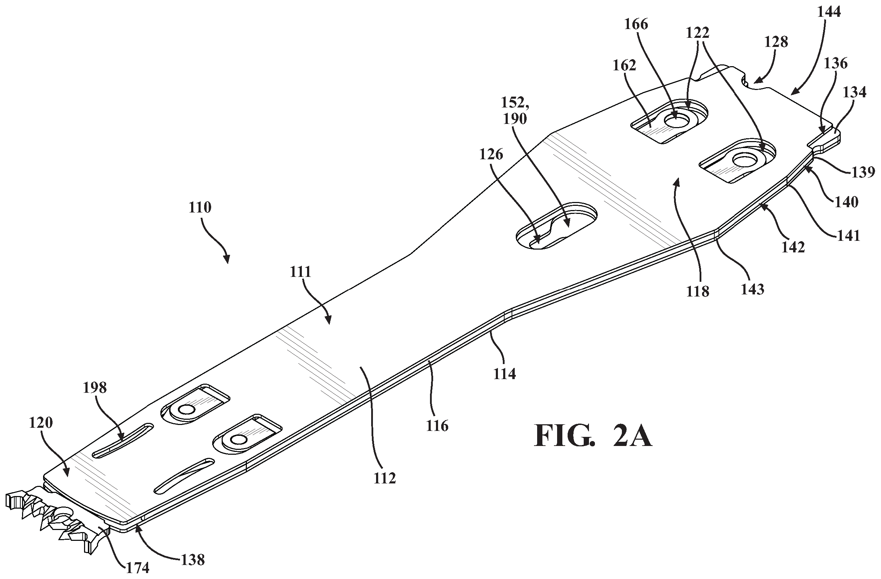

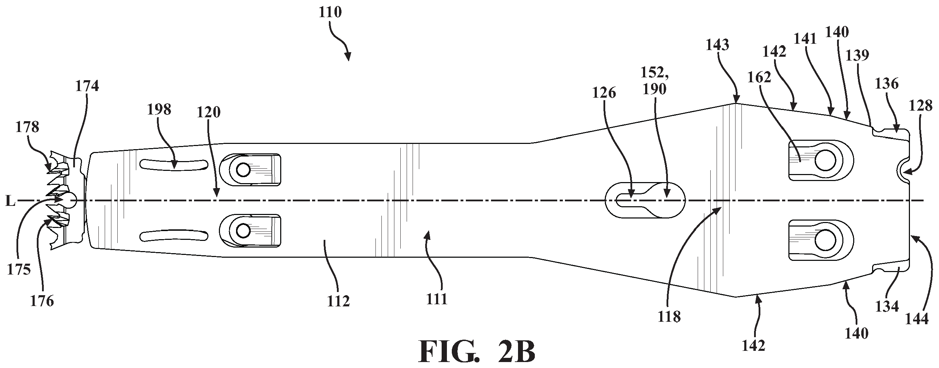

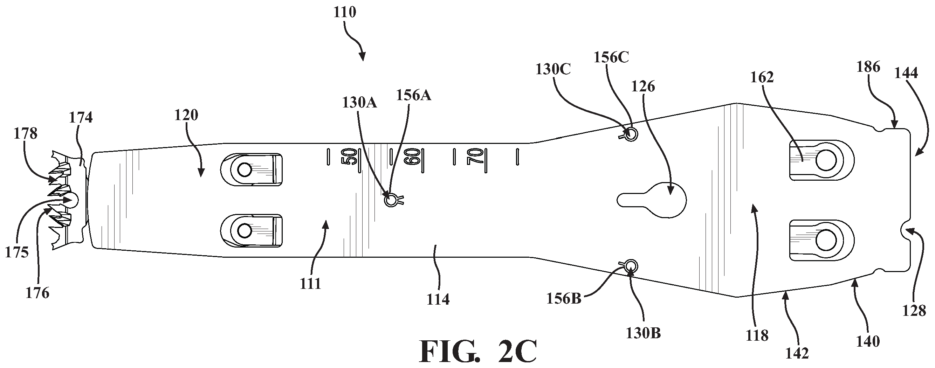

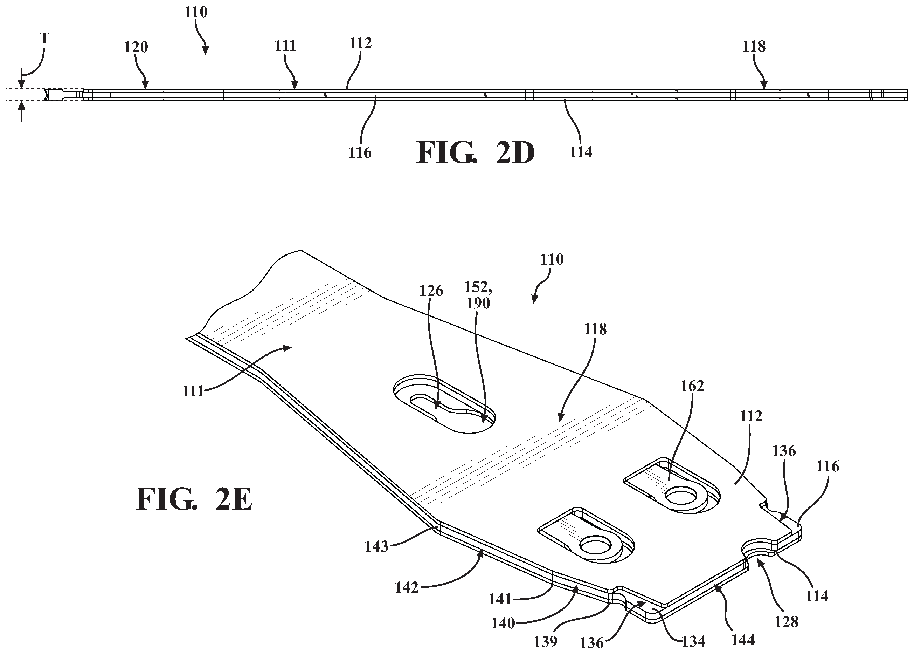

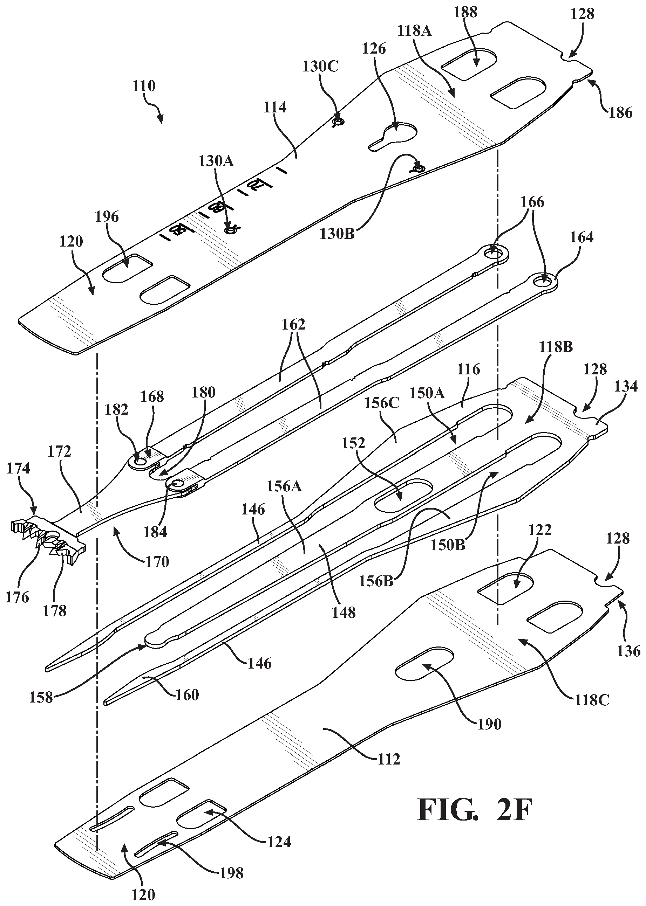

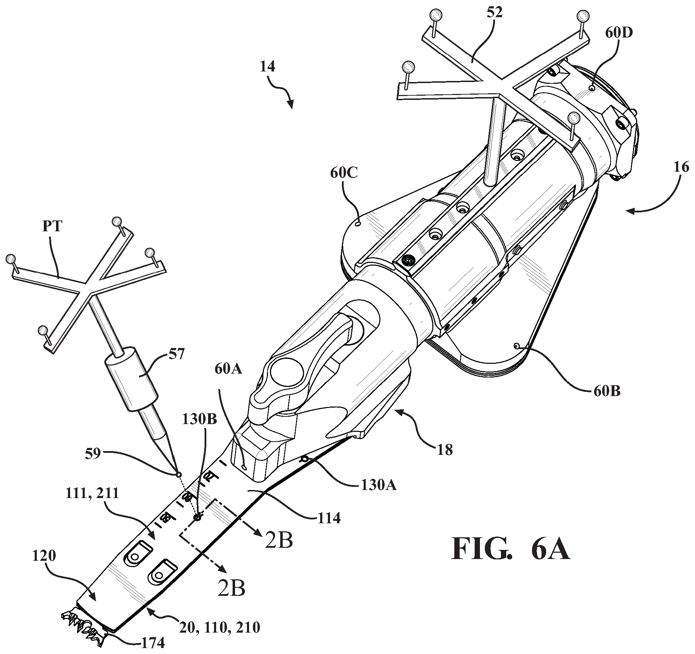

Referring to Figures 1 and 2, the surgical system 10 includes an surgical manipulator 14. The surgical manipulator may include a hand-held instrument, hand-held medical robotic system, a surgical robotic, or the like. The surgical manipulator 14 may also be referred to as a surgical instrument. An exemplary hand-held robotic instrument is shown in PCT/US2020/042128, PCT/US2022/013108 and PCT/US2022/013115, and PCT/US2022/054115, all of which are hereby incorporated by reference in their entirety. An exemplary non-robotic instrument is shown ins U.S. Patent No. 7,704,254, which is also incorporated by reference in its entirety. In some examples, a user manually holds and supports the surgical instrument 14 (as shown in Figure 1). In some examples, the user may manually hold the surgical instrument 14 while the instrument is being at least partially, or fully, supported by an assistive device, such as a passive arm (e.g., linkage arm with locking joints, weight-balancing arm), an active arm, and/or the like. As best shown in Figures 1 and 6A, the surgical instrument 14 comprises a tool platform 16.

The surgical instrument 14 may be freely moved and supported by a user without the aid of a guide arm, e.g., configured to be held by a human user while effecting physical removal of material such that the weight of the tool is supported solely by a hand of the user during the procedure. Put another way, the surgical instrument 14 may be configured to be held such that the user's hand is supporting the instrument 14 against the force of gravity. The surgical instrument 14 may weigh 8 lbs. or less, 6 lbs. or less, 5lbs. or less, or even 3lbs. or less. The surgical instrument 14 may have a weight corresponding to ANSI/AAMI HE75:2009. The instrument 14 also comprises a tool mount 18 for receiving a tool 20. In some examples, when the tool 20 is a saw blade 110, the tool mount 18 may be referred to as a blade mount.

The tool 20 couples to the tool mount 18 to interact with the anatomy in certain operations of the surgical system 10 described further below. The tool 20 may also be referred to as an end effector. The tool 20 may be removable from the tool mount 18 such that new/different tools 20 can be attached when needed. The tool 20 may also be permanently fixed to the tool mount 18. The tool 20 may comprise an energy applicator designed to contact the tissue of the patient 12. In some examples, the tool 20 may be a saw blade 110, as shown in Figures 2A to 2D, or other type of cutting accessory. In such instances, the tool support may be referred to as a blade support or tool mount. It should be appreciated that in any instance where blade support is referred to, it may be substituted for the term 'tool support' and vice-versa. The tool 20 may comprise the blade assembly and drive motor to cause oscillatory motion of the blade as shown in U.S. Patent No. 9,820,753 to Walen et al. or U.S. Patent No. 10,687,823, hereby incorporated herein by reference. Alternative rotary driving motors are contemplated. Such driving components may comprise a transmission TM coupled to the drive motor DM to convert rotary motion from the drive motor DM into oscillating motion of the tool 20.

The surgical system 10 further includes a navigation system 32. One example of the navigation system 32 is described in U.S. Patent No. 9,008,757, filed on September 24, 2013, entitled, "Navigation System Including Optical and Non-Optical Sensors," hereby incorporated herein by reference. The navigation system 32 tracks movement of various objects. Such objects include, for example, the instrument 14, the tool 20 and the anatomy, e.g., the femur F and tibia T. The navigation system 32 tracks these objects to gather state information of each object with respect to a (navigation) localizer coordinate system LCLZ. As used herein, the state of an object includes, but is not limited to, data that defines the position and/or orientation of the tracked object (e.g., coordinate systems thereof) or equivalents/derivatives of the position and/or orientation. For example, the state may be a pose of the object, and/or may include linear velocity data, angular velocity data, and the like.

The navigation system 32 may include a cart assembly 34 that houses a navigation controller 36, and/or other types of control units. A navigation user interface UI is in operative communication with the navigation controller 36. The navigation user interface UI includes one or more displays 38. The navigation system 32 is capable of displaying graphical representations of the relative states of the tracked objects to the user using the one or more displays 38. The navigation user interface UI further comprises one or more input devices to input information into the navigation controller 36 or otherwise to select/control certain aspects of the navigation controller 36. Such input devices include interactive touchscreen displays. However, the input devices may include any one or more of push buttons, pointer, foot switches, a keyboard, a mouse, a microphone (voice-activation), gesture control devices, and the like. In some examples, the user may use buttons located on the pointer to navigate through icons and menus of the user interfaces UI to make selections, configuring the surgical system 10 and/or advancing through the workflow.

The navigation system 32 also includes a localizer 44 coupled to the navigation controller 36. In one example, the localizer 44 is an optical localizer and includes a camera unit 46. The camera unit 46 has an outer casing 48 that houses one or more optical sensors 50. The localizer 44 may comprise its own localizer controller 49 and may further comprise a video camera VC.

The navigation system 32 includes one or more trackers. In some examples, the trackers include a pointer tracker PT, a tool tracker 52, a first patient tracker 54, and a second patient tracker 56. In the illustrated example of Figure 1, the tool tracker 52 is firmly attached to the instrument 14, the first patient tracker 54 is firmly affixed to the femur F of the patient 12, and the second patient tracker 56 is firmly affixed to the tibia T of the patient 12. In this example, the patient trackers 54, 56 are firmly affixed to sections of bone. The trackers 52, 54, 56 and pointer tracker are registered to their respective objects (e.g. bone, tool) and the navigation system 32 manually, automatically, or a combination thereof. In some examples, the pointer tracker PT is firmly affixed to a pointer 57 and used for registering the anatomy to one or more coordinate systems, including the localizer coordinate system LCLZ and/or used for other calibration and/or registration functions. In one example, the pointer 57 may be used to register the patient trackers 54, 56 to the bone which the tracker 54, 56 is attached, respectively, and the tool tracker 52 (and optionally 53) to the tool mount 18, the tool 20, the tool platform 16, or a combination thereof. In some examples, the pointer tracker PT may be used to register the TCP of the instrument 14 to the tracker 52 relative to a tracker coordinate system. This way, if the localizer 44 is moved from position to position, the registration of the instrument 14 is located relative to the tool tracker 52. However, other means of registration of the trackers 52, 54, 56 are contemplated and may be implemented together or separately with the pointer tracker PT. Other tracker locations are also contemplated.

The tool tracker 52 may be affixed to any suitable component of the instrument 14, and in some versions may be attached to the tool platform 16, the tool mount 18, directly to the tool 20, or a combination thereof. The trackers 52, 54, 56, PT may be fixed to their respective components in any suitable manner, such as by fasteners, clamps, or the like. For example, the trackers 52, 54, 56, PT may be rigidly fixed, flexibly connected (optical fiber), or not physically connected at all (ultrasound), as long as there is a suitable (supplemental) way to determine the relationship (measurement) of that respective tracker to the associated object. Any one or more of the trackers 52, 54, 56, PT may include active markers 58. The active markers 58 may include light emitting diodes (LEDs). Alternatively, the trackers 52, 54, 56, PT may have passive markers, such as reflectors, which reflect light emitted from the camera unit 46. Printed markers, or other suitable markers not specifically described herein, may also be utilized.

Various coordinate systems may be employed for purposes of tracking the objects. For instance, the coordinate systems may comprise the localizer coordinate system LCLZ, the tool mount coordinate system TCS, the base coordinate system BCS, coordinate systems associated with each of the trackers 52, 54, 56, PT, one or more coordinate systems associated with the anatomy, one or more coordinate systems associated with pre-operative and/or intra-operative images (e.g., CT images, MRI images, etc.) and/or models (e.g., 2D or 3D models) of the anatomy - such as the implant coordinate system, and a TCP (tool center point) coordinate system. In some examples, the surgical system 10 does not rely on pre-operative and/or intraoperative imaging to create the 2D or 3D models of the target bone. Rather, the surgical system may be used in an imageless system using the pointer tracker PT to register the target anatomy, capturing various anatomical landmarks, which is then processed by the control system 60 to morph a nominal bone model to match the captured data. In other examples, pre-operative and intraoperative imaging is used to image the target area of the patient and then transform the 2D and/or 3D images into a 3D model of the target bone. It is also contemplated that the surgical system 10 may use a combination of imaged and imageless procedures in creating a 3D model of the target surgical area. One exemplary system is described in U.S. Patent No. 8,617,174, which is hereby incorporated by reference. Coordinates in the various coordinate systems may be transformed to other coordinate systems using transformations upon establishing relationships between the coordinate systems, e.g., via registration, calibration, geometric relationships, measuring, etc.

In some examples, the TCP is a predetermined reference point or origin of the TCP coordinate system defined at the distal end of the tool 20. The geometry of the tool 20 may be defined relative to the TCP coordinate system and/or relative to the tool support coordinate system TCS. The tool 20 may comprise one or more geometric features, e.g., perimeter, circumference, radius, diameter, width, length, height, volume, area, surface/plane, range of motion envelope (along any one or more axes), etc. defined relative to the TCP coordinate system and/or relative to the tool support coordinate system TCS and stored in the non-volatile memory of the control boards 31 in the control housing 29 of the instrument 14, the navigation system 32, the instrument controller 28, or a combination thereof. The tool center point (TCP), in one example, is a predetermined reference point and corresponding coordinate system defined at the tool 20. The TCP has a known, or able to be calculated (i.e., not necessarily static), pose relative to other coordinate systems. The TCP coordinate system includes an origin point and a set of axes (e.g. x axis, y axis, z axis) which define the pose of the TCP. By tracking the TCP (or knowing the pose of the TCP), the system 10 may calculate the position and orientation of the instrument 14 based on the pose of the TCP and the known positional relationship between the TCP and the features of the instrument 14. In some examples, the tool 20 has a blade plane (e.g., for saw blades) that will be described for convenience and ease of illustration, but is not intended to limit the tool 20 to any particular form. In other examples, the tool 20 has an axis. Points, other primitives, meshes, other 3D models, etc., can be used to virtually represent the tool 20. The origin point of the TCP coordinate system may be located at the spherical center of the bur 25 of the tool 20, the tip of a drill bit, or at the distal end of the saw blade 27 such that the TCP coordinate system is tracked relative to the origin point on the distal tip of the tool 200. Alternatively, the TCP may be tracked using a plurality of tracked points. The TCP may be defined in various ways depending on the configuration of the tool 20. The instrument may employ the joint/motor encoders, or any other non-encoder position sensing method, so the control system 60 may determine a pose and/or position of the TCP relative to the hand-held portion 16 and BCS. The tool support 18 may use joint measurements to determine TCP pose and/or could employ techniques to measure TCP pose directly. The control of the tool 20 is not limited to a center point. For example, any suitable primitives, meshes, etc., can be used to represent the tool 20. It should be appreciated that the TCP may alternatively be defined as a point, as opposed to a coordinate system. The TCP coordinate system allows calculate any required reference points or geometry aspects of the tool once you have determined the pose of the saw blade or other tool.

The localizer 44 monitors the trackers 52, 54, 56, PT (e.g., coordinate systems thereof) to determine a state of each of the trackers 52, 54, 56, PT, which correspond respectively to the state of the object respectively attached thereto. The localizer 44 may perform known techniques to determine the states of the trackers 52, 54, 56, PT, and associated objects (such as the tool, the patient, the tool support, and the hand-held portion). The localizer 44 provides the states of the trackers 52, 54, 56, PT to the navigation controller 36. In some examples, the navigation controller 36 determines and communicates the states of the trackers 52, 54, 56, PT to the instrument controller 28 or console.

The navigation controller 36 may comprise one or more computers, or any other suitable form of controller. Navigation controller 36 has a central processing unit (CPU) and/or other processors, memory, and storage (not shown). The processors can be any type of processor, microprocessor or multi-processor system. The navigation controller 36 is loaded with software. The software, for example, converts the signals received from the localizer 44 into data representative of the position and/or orientation of the objects being tracked. The navigation controller 36 may additionally, or alternatively, comprise one or more microcontrollers, field programmable gate arrays, systems on a chip, discrete circuitry, and/or other suitable hardware, software, or firmware that is capable of carrying out the functions described herein. The term processor is not intended to limit any embodiment to a single processor.

Although one example of the navigation system 32 is shown to determine object states, the navigation system 32 may have any other suitable configuration for tracking the instrument 14, tool 20, and/or the patient 12. In another example, the navigation system 32 and/or localizer 44 are ultrasound-based. For example, the navigation system 32 may comprise an ultrasound imaging device coupled to the navigation controller 36. The ultrasound imaging device images any of the aforementioned objects, e.g., the instrument 14, the tool 20, and/or the patient 12, and generates state signals to the navigation controller 36 based on the ultrasound images. The ultrasound images may be 2D, 3D, or a combination of both. The navigation controller 36 may process the images in near real-time to determine states of the objects. The ultrasound imaging device may have any suitable configuration and may be different than the camera unit 46 as shown in Figure 1.

In another example, the navigation system 32 and/or localizer 44 are radio frequency (RF)-based. For example, the navigation system 32 may comprise an RF transceiver coupled to the navigation controller 36. The instrument 14, the tool 20, and/or the patient 12 may comprise RF emitters or transponders attached thereto. The RF emitters or transponders may be passive or actively energized. The RF transceiver transmits an RF tracking signal and generates state signals to the navigation controller 36 based on RF signals received from the RF emitters. The navigation controller 36 may analyze the received RF signals to associate relative states thereto. The RF signals may be of any suitable frequency. The RF transceiver may be positioned at any suitable location to track the objects using RF signals effectively. Furthermore, the RF emitters or transponders may have any suitable structural configuration that may be much different than the trackers 52, 54, 56, PT shown in Figure 1.

In yet another example, the navigation system 32 and/or localizer 44 are electromagnetically based. For example, the navigation system 32 may comprise an EM transceiver coupled to the navigation controller 36. The instrument 14, the tool 20, and/or the patient 12 may comprise EM components attached thereto, such as any suitable magnetic tracker, electro-magnetic tracker, inductive tracker, or the like. The trackers may be passive or actively energized. The EM transceiver generates an EM field and generates state signals to the navigation controller 36 based upon EM signals received from the trackers. The navigation controller 36 may analyze the received EM signals to associate relative states thereto. Again, such navigation system 32 examples may have structural configurations that are different than the navigation system 32 configuration shown in Figure 1.

The navigation system 32 may have any other suitable components or structure not specifically recited herein. Furthermore, any of the techniques, methods, and/or components described above with respect to the navigation system 32 shown may be implemented or provided for any of the other examples of the navigation system 32 described herein. For example, the navigation system 32 may utilize solely inertial tracking or any combination of tracking techniques, and may additionally or alternatively comprise, fiber optic-based tracking, machine-vision tracking, and the like.

Referring to Figures 2A to 2F, a first configuration of a blade cartridge 110 for use with the surgical instrument 14 of the surgical system of Figure 1 is illustrated. The sagittal blade cartridge 110 may also be referred to as a sagittal blade cartridge, surgical blade cartridge, or cartridge. The blade cartridge 110 may comprises a guide bar 111. The guide bar 111 may comprises a plurality of plates 112, 114, 116, including a first plate 112, a second plate 114, and a third plate 116. The guide bar 111 may be configured such that the third plate 116 may be disposed between the first plate 112 and the second 114. As such, the first plate 112 may also be referred to as the bottom plate, the second plate 114 may be referred to as a top plate, and the third plate 116 may be referred to as an inner plate, center plate, inner member, center layer, and/or center member. While the guide bar 111 is illustrated as only including three plates layered in a laminate type arrangement, it is contemplated that the guide bar 111 may comprise any number of plates and/or layers.

Collectively, the plates are formed so that the guide bar 111 has a proximal portion 118 that forms the proximal end 118A, 118B, 118C of the plates 112, 114, 116. The proximal portion 118 of the guide bar 111 may be configured to initially taper outwardly as you move distally along the guide bar 111, and then eventually taper inwardly as you move further distally along the guide bar 111. The outward taper of the proximal portion 118 of the guide bar 111 may configured to as a mounting feature to assist in mounting and/or securing the saw blade cartridge 110 to a surgical instrument 14, such as a surgical robot or handheld instrument. For example, as illustrated in Figures 2B and 2C, opposed outer edges of the proximal portion 118 of the guide bar 111 may define a contact surface with the tool mount 18. It is further contemplated that one or both of the proximal portion(s) 118A, 118C of the first and/or second plates 112, 114 may be shaped to define a recess 136 or include a recess or cutout 136 defined in the outer edge of the proximal portion 118A, 118C of the first and/or second plates 112, 114 that exposes a portion 134 of the proximal portion 118B of the third plate 116. The recess 136 may be sized and/or shaped to only expose a small portion 134 of the third plate, such as the tab 134 of the third plate 116 that is exposed by the recess 136 defined in the proximal portion 118 of the first plate 112 as illustrated in Figure 2B. Alternatively, the recess 136 of defined by the proximal portion of the first plate 112 may include an entire section of the proximal portion 118 of the first plate 112 that is removed exposed a distal ledge of the third plate along the proximal edge of the proximal portion of the guide bar 111. In either configuration, the portion 134 of the proximal end 18B of the third plate 116 that is exposed by the recess 136 defined in the outer edge of the first or the second plate 112, 114 may be configured to be one of the portions 134 of the guide bar 111 that contacts and/or interacts with the mount 18 of the surgical instrument 14 for the purpose of mounting and/or aligning of the saw blade cartridge 110 relative to the surgical instrument 14. This will be described in greater detail below.

Referring to Figure 2B, the opposed outer edges of the proximal portion 118 of the guide bar 111 may comprise a number of points 139, 141, 143 spaced along the perimeter edge of the proximal portion 118. The points 139, 141, 143 along the edge of the proximal portion 118 of the guide bar 111 may define a plurality of segments 140, 142 of the outer edge of the proximal portion 118 of the guide bar 111, each of which may comprise varying degrees of taper relative to a longitudinal axis L that extends from the proximal 118 to the distal end 120 of the guide bar 111. For example, the outer edge of the proximal portion 118 of guide bar 111 may comprise a first segment 140 defined between a first point 139 and a second point 141. The first segment 140 may comprise a first outward directed taper such that the second point 141 is further from the longitudinal axis L of the guide bar 111 than the first point 139. The outer edge of the proximal portion 118 of the guide bar 111 may comprise a second segment 142 defined between the second point 141 and a third point 143. The second segment 142 may comprise a second outward directed taper such that the third point 143 is further from the longitudinal axis L of the guide bar 111 than the second point 141. The proximal portion 118 of the guide bar 111 may be configured such that each of the first and second segments 140, 142 comprise a different taper relative to the longitudinal axis L of the guide bar 111. The taper and/or orientation of each of the segment 140, 142 of the opposed outer edges of the proximal portion 118 of the guide bar 111 may be correspond to and/or be matched to an angle and/or taper of a surface of the bracket 68 of a mount 18 for securing the saw blade cartridge 110 to the surgical instrument 14, as will be described in greater detail below.

The proximal portion 118 of the guide bar 111 may further comprise a proximal edge 144 that is oriented to be generally perpendicular to the longitudinal axis of the guide bar 111. A recess 128 may be defined in the proximal edge 144 of the guide bar 111. The recess 128 may be formed in the proximal edge 144 of the guide bar 111 one side or the other of the longitudinal axis L of the guide bar 111. The recess 128 may serve as an alignment and/or positioning aide when manufacturing the guide bar 111, including manufacturing of the individual plates 112, 114, 116 forming the guide bar. It is further contemplated that the recess 128 formed in each of the first, second, and/or third plates 112, 114, 116 may vary in size and/or shape. For example, as illustrated in Figures 2B and 2E, the recess 128 formed in the first plate and the third plate 112, 116 is cut deeper into the proximal portion the respective plates and has a larger radius that the recess 128 formed in the second plate 114. This may allow the manufacture of the guide bar 111 to distinguish between the first and second plates 112, 114, which may otherwise appear very similar to one another. The difference in the size and/or shape of the recess 128 formed in the first and second plates 112, 114 may also assist the user in properly mounting the guide bar to the mount of the surgical instrument 14.

Forward of the proximal portion 118 of the guide bar 111 is a middle portion and a distal portion 120. The plates 112, 114, 116 are formed so that the opposed sides of the guide bar that form the sides of the middle portion are generally parallel. The opposed sides of the distal portion 120 of the guide bar 111 may be tapered in some configurations. As illustrated in the figures, opposed edges 132, 199 of the distal portion 120 of the guide bar 111 may be tapered inwardly toward the center of the plates 112, 114, 116. It is also contemplated that the opposed edges of the distal portion 120 of the guide bar 111 may be generally parallel to one another. The width across the middle portion and/or the distal portion 120 of the guide bar 111 may be less that the width of the proximal portion 118 at the widest point between the opposed edges of the proximal portion 118 of the guide bar 111.

The proximal portion 118 of each of the plates 112, 114, 116 may further comprise two laterally spaced apart openings 122, 188 (one identified). As illustrated in the figures, the first plate may comprise a pair of openings 122, and the second plate 114 may comprise a pair of openings 188. The third plate 116, depending on the shape of the plate 116 may or may not define an opening. As illustrated in the figures, the third plate 116 is shaped to define a channel 150A, 150B that cooperates with the openings 122, 88 defined in the first and second plates 112, 114. While the plates are illustrated including a pair of openings 122, 188 defined in the first and second plates 112, 114, and a pair of channels 150A, 150B defined by the third plate 116, it is contemplated that the guide bar 111 may be formed with a single opening in each of the plates 122, 188, as well as with more than two opening 122, 188 defined in each of the plates 112, 114, 116. The openings 122, 188 may formed to have a semi-oval shape. Each opening 122, 188 has a curved portion towards the proximal end of the plate 112, 114, 116 and a straight portion towards the distal end of the guide bar 111.

Forward of the openings 122, 188, at least one of the plates 112, 114, 116 may be configured to define a keyhole shaped opening 126, 152, 190. As illustrated in the figures, the second plate 114 may define a keyhole shaped opening 126 and the first and third plates 112, 116 define openings 152, 190 that cooperate with the keyhole shaped opening 126 of the second plate 114. While the figures only show the second plate 114 with a keyhole shaped opening 126, it is contemplated that one, two, or all three of the plates 112, 114, 116 may define a keyhole shaped opening 126, 152, 190. For example, it is contemplated that the third plate 116 may define a keyhole shaped opening 152, and the first and second plates 112, 114 may define general openings 126, 190, such as openings in the shape of a square, circle, oval, or the like. Referring to Figure 2B, the keyhole shaped opening 126 is shaped so the widest width portion of the opening 126 can receive a fastening feature of a mount for the saw blade cartridge 110. The openings 126, 152, 190 defined in the respective plates 112, 114, 116 may be centered between the opposed edges of the guide bar 111, such that the openings 126, 152, 190 are positioned on the longitudinal axis L extending between the proximal end 118 and the distal end 120 of the guide bar 111 such that the longitudinal axis L bisects the guide bar 111. The keyhole shaped opening 126 may further be formed so the narrower portion of the opening 126 is located distally forward of the wide width portion of the opening to allow the fastening feature 62 of the tool mount 18 to slide within the opening 126.

Furthermore, at least one of the plates 112, 114, 116 of the guide bars 111 may comprise an opening 130 or aperture configured to provide access to and/or expose the reference feature 156 that is disposed one of the other plates 112, 114, 116 of the guide bar 111. For example, as illustrated in Figures 2C and 2F, the guide bar 111 may comprise a first, second, and third plate 112, 114, 116. Either the first or second plate 112, 114 may comprise an opening 130 to provide access for the pointer 57 to the reference feature 156 that is disposed the third plate 116.

Referring to Figure 2C, one configuration of the guide bar 111 may comprise a plurality of additional openings 130 formed in the first, and/or second plates 112, 114, configured for verification and/or registration of the saw blade cartridge 110 within a known coordinate system of the surgical navigation system 32. For example, at least one of the first and/or second plates 112, 114 may define at least three openings 130A, 130B, 130C. The openings 130A, 130B, 130C defined in the first and/or second plates 112, 114 may be configured to provide access to a reference feature 156A, 156B, 156C corresponding to each of the openings 130A, 130B, 130C and defined on the third plate 116. The reference feature 156 may comprise a portion of the surface of the third plate 116 that is exposed by the openings 130A, 130B, 130C. The openings 130A, 130B, 130C may further serve to define a contact point on the third plate 116 for verification and/or registration of the pose of the saw blade cartridge 110 with the navigation system 32. It is contemplated that the openings 130A, 130B, 130C may comprise an aperture in the shape of a circle, square, rectangle, or other similar polygon that provide access to the reference feature 156 on the surface of the third plate 116. While not illustrated in the figures, the reference feature 156 may further comprise a recess, indent, divot, indicia, or similar marking defined on the third plate 116 and capable of being accessed through the openings 130A, 130B, 130C defined in one of the first or second plates 112, 114. This may include a divot on surface of the third plate that is accessible through the openings 130A, 130B, 130C in one of the first plate 112 or the second plate 114. It is further contemplated that the reference feature 156 may comprise a marking, such as a laser marker (without any physical recess, divot or aperture). The marking may be realized as an etch, color, engraving, or similar optical marking.

Generally, the guide bar 111 may comprise three of more openings 130A, 130B, 130C corresponding to three or more reference features 156A, 156B, 156C to allow the navigation system 32 to define a plane of the surgical saw blade cartridge 110. However, additional openings 130A, 130B, 130C and corresponding reference features 156A, 156B, 156C may be defined on the guide bar 111. Furthermore, the openings 130A, 130B, 130C and corresponding reference features 156A, 156B, 156C are spaced apart from another such that a straight line cannot be drawn which intersects all reference features 156A, 156B, 156C.

The reference features 156A, 156B, 156C, and by extension the openings 130A, 130B, 130C, may be arranged on the guide bar 111 in known location both with regard the specific location of the reference features 156A, 156B, 156C on the guide bar 111, as well as the location of each of the reference features 156A, 156B, 156C with regard to each of the other the reference features 156A, 156B, 156C. The known location of the reference features 156A, 156B, 156C on the guide bar 111 will be utilized to define a position of the guide bar 111 and/or the surgical saw blade cartridge 110 within a known coordinate system of the navigation system 32. The known location of the reference features 156A, 156B, 156C will be utilized by the navigation system 32 to calculate an expected location of each reference features 156A, 156B, 156C based on the geometric properties and the mechanical stack up of each of the surgical instrument 14 and the surgical saw blade cartridge 110 as part of the registration process, which will be described in greater detail below.

The third plate 116, as seen best in Figure 2F, is formed to have a proximal portion 118B of the plate. A pair of laterally spaced apart tines 146 may extend forward from the proximal portion 118B of the third plate 116. The tines 146 may be referred to as the outer tines. The outer surfaces of tines 146 are the outer surfaces of the third plate 116 that form sections of the outer side surfaces of the guide bar 111. The outer tines 146 thus have proximal sections that extending forward from proximal portion 118B and taper first outwardly and then inwardly as you move distally along the third plate 116. The outer tines 146 are symmetrically located relative to the longitudinal axis L of the guide bar 111. Outer tines 146 are further shaped to be mirror images each other, mirrored about the longitudinal axis L. Furthermore, the outer tines 146 may be configured to couple the couple the first, second and third plates 112, 114, 116 together to form the guide bar 111. For example, the first and second plates 112, 114 may be welded or similarly coupled to the outer tines 146 of the third plate 116 to form the guide bar 111. Alternatively, it is also contemplated that the first and second plates 112, 114 may be welded or similarly coupled to the inner tine 148 of the third plate 116 to form the guide bar 111. By welding or similarly coupling the first and second plates 112, 114 to the inner tine 148 of the third plate 116, it may avoid any interference, unintended warping or similar disturbance to the edges of the proximal portion 118 of the third plate 116, which may be utilized in aligning and/or orienting the guide bar 111 relative to the surgical instrument 14.

Adjacent the distal end of each tine 146, is shaped to have a lobe 160, that extends inwardly towards the longitudinal axis L of the guide bar 11. Moving proximally to distally along the tine 146, the associated lobe 160 first curves inwardly toward the longitudinal axis L of the guide bar 111 and then curves outwardly.

The third plate 116 may further comprise a third tine 148, the inner tine, that is disposed between the outer tines 146 and is centered on the longitudinal axis L of the guide bar 111. As described above, the inner tine 148 may be formed to define an opening 152 which may be a keyhole shaped opening 152. The inner tine 148 may shaped so that opening 152 is identical in shape and positioned to be in registration with opening 126 and 190 of first and second plates 112, 114, respectively. The inner tine 148 is of constant width except for the distal end of the tine. At the most distal end, the inner tine 148 may comprise a head 158 with a distally directed face that is curved and configured to define a pivot surface. While not illustrated in the figures, it is also contemplated that the head 158 may be defined a recessed surface configured to receive a rocker or curved surface that may pivot within the recessed surfaced defined by the head 158. The head 158 may include a center, the center defining a radius of the distally directed face that is curved to define the pivot surface

While the figures illustrate an inner tine 148 disposed between the outer tines 146, it is contemplated that the third plate 116 may be formed without the inner tine 148, and only include the two outer tines 146 spaced apart from one another. In such a configuration, a pivot pin may be coupled adjacent the blade head. In other words, while the third plate is shown has having three tines formed as a single component, it is contemplated that the three tines may be formed separately and secured between the first and second plates.

The outer tines 146 are spaced apart from the inner tine 148, such that the third plate 116 defines a pair of elongated, closed end slot 150A, 150B between each of the outer tines 146 and the inner tine 148. The proximal end of the slot 150A, 150B is the closed end of the slot. When cartridge 110 is assembled, each slot 150A, 150B is disposed over a separate one of the openings 122, 188 in the first and second plates 112, 114, respectively. The third plate 116 is further formed so that the proximal end portion of each slot 150A, 150B has a shape substantially equal to the shape of openings 122, 188 in the first and second plates 112, 114. Thus, the lateral width of the proximal end portion of each slot 150A, 150B is greater than the width of the slot 150A, 150B distally forward of the proximal portion 118.

While the third plate 116 is generally depicted in the figures as being formed as a single solitary or unitary plate, it is contemplated that the third plate 116 may be configured as a plurality of separate pieces arranged to form the features and /or portions of the third plate 116 that are described above. For example, the proximal portion 118 of the third plate 116 may be formed as a unitary piece with the outer tines 146, and the inner tine 148 may be a separate piece. Alternatively, the inner tine 148 may be omitted. In yet another exemplary configuration, the proximal portion 118 of the third plate 116, the outer tines 146, and the inner tine 148 may all be formed as separate pieces.

Referring to Figures 2A and 2F, it is observed that first plate 112 is shaped to have an outer perimeter substantially identical to that of second plate 114. However, it is also contemplated that the one of the firs plate 112 or the second plate 114 may be longer/shorter and/or narrower/wider than the other plate. For example, the second plate 114 may be constructed such that the outer perimeter is narrower, i.e. the perimeter edge is closer to the longitudinal axis L, than that of the first plate 112. Forward of the proximal portion 118 of the first and second plates 112, 114 is a distal portion 120. The distal portion of the first and second plates 112, 114 is shaped to have two openings 124, 196. The openings 124, 196 are identical in shape and positioned such that the openings 124 of the first plate 112 are in registration with the openings 196 of the second plate 114.

The saw blade cartridge 110 may further comprise a saw blade 170 disposed between the first and second plates 112, 114 at the distal end of the saw guide bar 111. The saw blade 170 is formed to have a blade body 172 and a blade head 174. The saw blade 170 is formed so that blade body has a thickness that is no greater than the thickness of third plate 116. The blade body 172 of the saw blade 170, seen in Figure 2F, is generally rectangular in shape. However, it is also contemplated that the perimeter edges of the blade body 172 may be tapered and/or curved. The taper and/or curve may be matched to the taper or curve of the lobes 160 of the outer tines 146 of the inner plate 116. The shape of the blade body may be configured to allow the blade body 172 to pivot about the head 158 of the inner tine 148, oscillating between the lobes 160 of the outer tines 146.

When saw blade 170 is in the centered position within the guide bar 111 the major axis of the blade body 172 is collinear with the longitudinal axis L of the guide bar 111. The blade body 172 may be shaped to have two feet 184, one foot identified. The feet 184 have the same thickness of as the blade body 172. Each foot 184 extends to the open end of the adjacent slot 150A, 150B internal to the inner tine 148. Between the feet 184, the blade body 172 is formed to have a curved, proximally directed face 180. The blade body 172 is shaped so that face 180 can seat against and pivot around the head 158 integral with the inner tine 148. While the figures illustrate the third plate with an inner tine 148 defining a head 158 for the saw blade to pivot about, it is further contemplated that the guide bar may be configured with a pin disposed between the first and second plates 112, 114, and the saw blade 170 being configured to pivot about the pin. The blade feet 184 and blade body 172 are sometimes referred to as the base of the saw blade 170.

The distal end of the blade body 172 extends distally of the guide bar 111, such that the blade head 174 extends forward of the distal end of guide bar 111. The blade head 174 is formed with a plurality of teeth 176, 178. Referring to Figure 2D, the saw blade 170 is further formed so that blade head 174 has a thickness greater than that of the blade body 172. More particularly, the blade head 174 is formed to have a thickness equal to or greater than the thickness of the guide bar 111 so that the kerf formed by the cutting action of saw blade 170 is sufficient to receive the guide bar 111.

Referring to Figures 2A through 2C, the blade head 174 may comprise two type of teeth, base teeth 178 and one or more pilot teeth 176. For example, as illustrated in Figure 2C, the blade head 174 may comprise two pilot teeth 176. However, it is contemplated that the blade head 174 may comprise a single pilot tooth, or more than two pilot teeth. The pilot teeth 176 may be disposed proximate the center of the distal surface of the blade head 174. Furthermore, the pilot teeth 176 may be configured to extend a distance distally beyond the adjacent base teeth 178 of the blade head 174. In operation, with the pilot teeth 176 extending distally beyond the adjacent base teeth 178, the pilot teeth 176 are configured to oscillate along a first arc as the adjacent base teeth 178 oscillate about a second arc, such that the first arc is distal to the second arc. An exemplary surgical blade cartridge including pilot teeth is disclosed in International Patent Application No. PCT/US2021/064328 filed on December 20, 2021, which is incorporated herein by reference in its entirety.

Optionally, the blade head 174 may also include cut-outs or apertures 175. As illustrated in the Figures, the blade head is formed with a single cut-out 175. However, it is contemplated that the blade head 174 may be formed with any number of cut-outs or apertures 175. The cut-out 175 may be formed in tear-drop shape or generally circular shape. The cut-out 175 may be configured to allow for removal of debris while cutting. It is also contemplated that the apertures 175 may be configured to provide cooling of the blade 70 and/or blade head 74 while cutting. Furthermore, the cut-out 175 may be configured to assist in manufacturing the blade 170. For example, when manufacturing the blade 170, the cut-out 175 may be machined and/or formed in the blank piece of material from which the blade will be formed. The cut-out 175 may then be used during manufacturing as a reference point, such that the blade body 172, blade head 174, and/or teeth 176, 178 may be machined/formed suing the cut-out(s) 175 as a reference. For example, the manufacturing process may be setup such that the apertures 175 are identifiable, and the machining tool is programmed to cut and/or grind the blank piece of the material at a location based on the aperture(s) 175 to form the teeth.

A drive link 162 is disposed in each of the slots 150A, 150B internal to the guide bar 111. Each drive link 162 is in the form of an elongated flat strip of metal. The drive links 162 are formed so that, at the proximal end of each link, there is a foot 164. Each foot 164 is formed to have a center located through hole 166, one opening identified. Through holes 166 are dimensioned so that the associated drive link feet 164 can be fitted over the saw head drive pins. Each drive link 162 is shaped so that foot 164 has a thickness that is greater than the thickness of the metal strip forming the main body of the link 162. The thickness of the feet 164 is typically no greater than the thickness of the guide bar 111. When the cartridge 110 is assembled, the feet 164 generally seat in the proximal end portion of the associated slot 150A, 150B. The portions of the feet 164 that project outwardly from the main body of each link 162 seat in the openings 122, 188 of the first and/or second plates 112, 114.

The drive links 162 may further comprise two fingers 168 extend distally forward from the distal end of the main body of each drive link 162, one finger identified. Fingers 168 overlap and are spaced apart from each other. More particularly, fingers 168 are spaced apart from each a sufficient distance so that a blade foot 184 can seat between each pair of fingers. Each finger 168 is formed with a hole 182. The holes 182 of each pair of fingers 168 are in registration with each other.

Referring to Figures 2B and 2F, the distal portion of the guide bar 111 may comprises a pair of generally oval shaped opening 198 in the first plate 112. While two openings 198 are illustrated in the figures, it is contemplated that guide bar 111 may be constructed with only a single opening, or with more than two openings. Furthermore, while the openings are illustrated in the figures as being defined in the first plate 112, it is contemplated that the similar openings may also be defined in the second plate 114. The openings 198 may comprise a curve and/or bend that extends longitudinally along the length of the distal portion 120 of the first plate 112. The opening may be configured to assist with the removal of debris that may become trapped between the first and second plates 112, 114 when the saw blade cartridge 110 is utilized to cut biological tissue.

The distal portion 120 of the guide bar 111 may also be formed to define a cut-out or slot 138 between the first and second plates 112, 114. As illustrated in Figure 2A, the outer tine 146 of the inner plate 116 is formed so that the distal most point of the lobe 160 of the outer tine 146 is positioned proximal of the distal most edge of the first and second plates 112, 114. This arrangement defines a slot 138 along the side edge of the guide bar 111 that may assist the removal of debris that may become trapped between the first and second plates 112, 114 when the saw blade cartridge 110 is utilized to cut biological tissue. The slot 138 along the side edge of the guide bar 111 that may also assist to increase the range of motion of the saw blade 170 as it is pivoted. By shortening the length of the outer tines 146, increased clearance may be provided allowing the saw blade to pivot further, and thus increasing the distance the blade head 174 may oscillate.

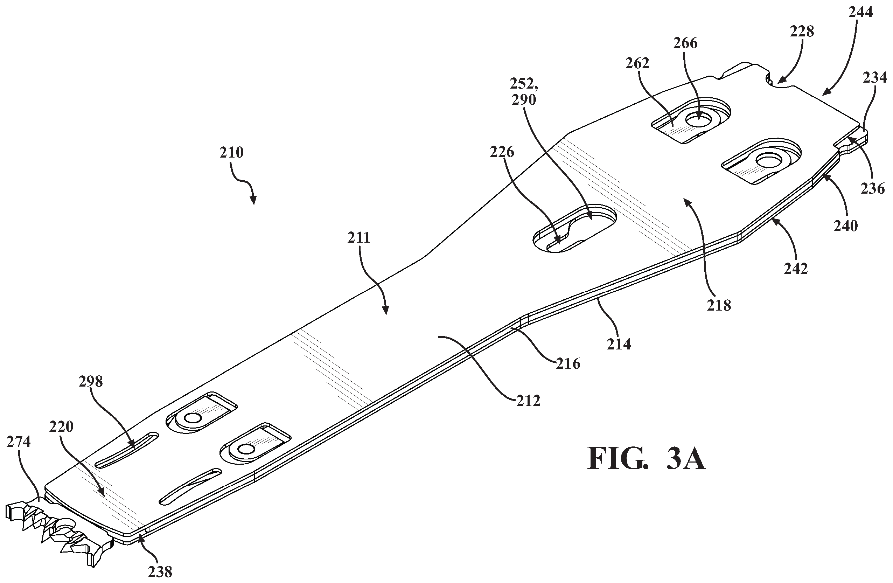

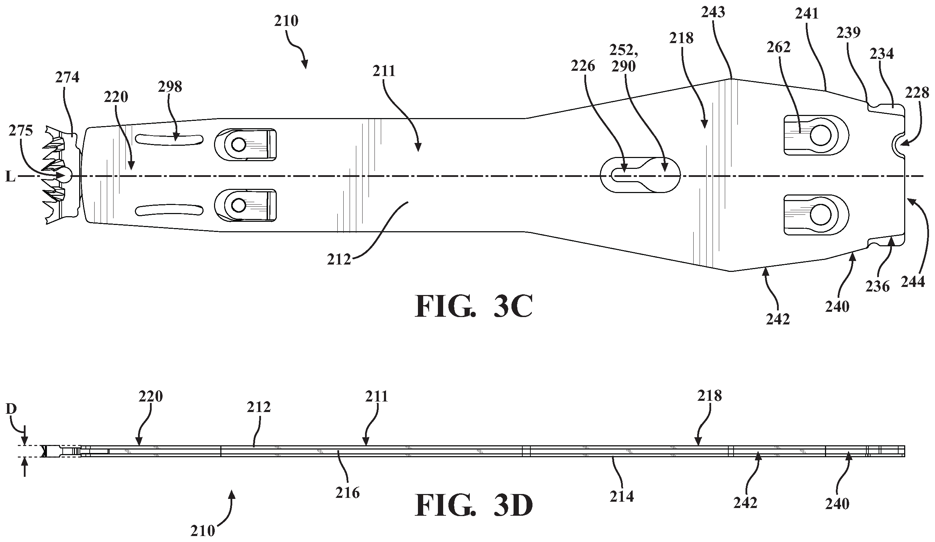

Referring to Figures 3A to 3G, a second configuration of the saw blade cartridge 210 is illustrated. It should be understood that components and/or features of the second configuration of the saw blade cartridge 210 including the same base reference number increased by one hundred, i.e. 110 and 210, may operate in a similar or the exact same manner as described above.

Similar to as described above, the second configuration of the saw blade cartridge 210 may comprises a guide bar 211 including a plurality of plates (212, 214, 216), including a first plate 212, a second plate 214, and a third plate 216. The guide bar 211 may be configured such that the third plate 216 may be disposed between the first plate 212 and the second 214. The first, second, and third plates, 212, 214, 216 are constructed in the same or similar fashion as those of the saw blade cartridge 110 described above. However, the second configuration of the saw blade cartridge 210 illustrated in Figures 3A to 3G is constructed with an alternative configuration of the reference features 256 disposed on the guide bar.

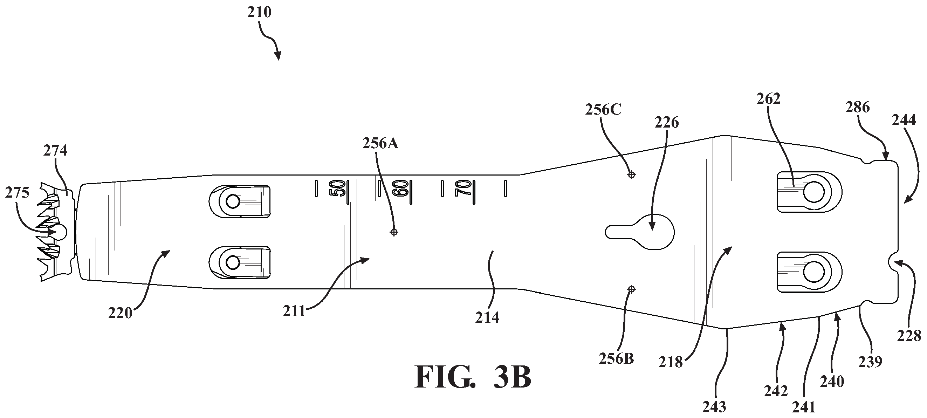

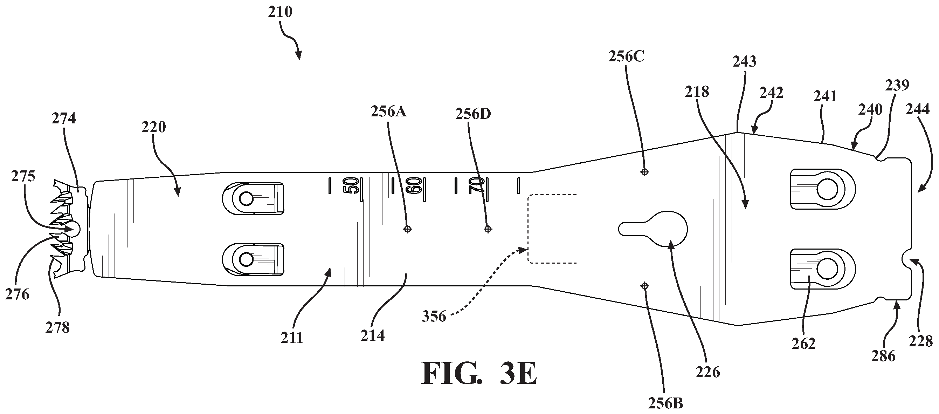

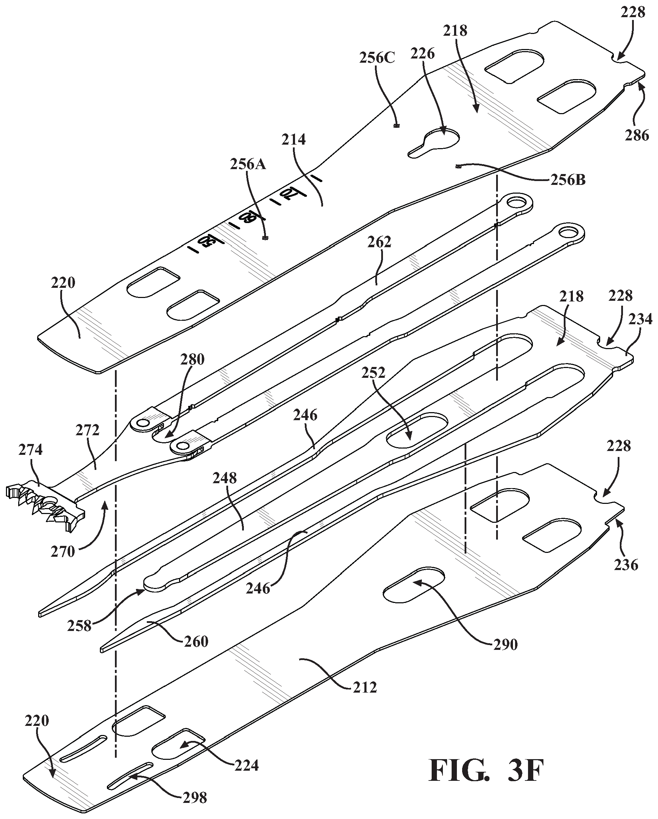

Referring to Figures 3A to 3G the saw blade cartridge 210 described above is illustrated including an alternative configuration of reference features 256A, 256B, 256C, 256D. As described above, the saw blade cartridge 210 may comprise a plurality of reference features 256A, 256B, 256C, 256D. Figures 3B, 3E, and 3F show the surgical saw blade cartridge 210 including an exemplary arrangement of reference features 256A, 256B, 256C, 256D. While the reference features 256A, 256B, 256C, 256D are illustrated in the figures as being disposed on the second plate 214, it is also contemplated that the plurality of reference features 256A, 256B, 256C, 256D may be disposed on the first or second plate 212, 214. Furthermore, the reference features 256A, 256B, 256C, 256D for a given saw blade cartridge may be placed on any combination of the first, second, and/or third plate(s) 212, 214, 216.

As described above, the reference features 256A, 256B, 256C, 256D may be formed as an aperture, divot, engraving, color marking, or similar marking or indicia. For example, as is illustrated in Figure 3E, the reference features 256A, 256B, 256C, 256D may comprise one or more indicia or similar visual marking disposed on the second plate 214. The reference features 256A, 256B, 256C, 256D may be defined as the indicia disposed on the surface of the second plate 214, such that the cross-hair markings illustrated in Figure 3E. While an example of the reference features 256A, 256B, 256C, 256D illustrated in Figure 3E are represented by a cross-hair marking, other shapes and/or forms of visual marking are contemplated. For example the reference features 256A, 256B, 256C, 256D may by defined by a circular-shaped marking, a triangle-shaped marking, a X-shaped marking, or the like. The markings representing the reference features 256A, 256B, 256C, 256D may be formed on the guide bar 211 via a laser marking, etching, painting, engraving, or similar marking process.

Alternatively, the reference features 256A, 256B, 256C, 256D may be defined as a divot or recess formed in the surface of the second plate 214. In yet another configuration, the reference features 256A, 256B, 256C, 256D may be defined by a combination of the features described above. For example, the reference features 256A, 256B, 256C, 256D may include a divot formed on the surface of the second plate 214 and an indicia applied to the divot to provide a visual identifier within the divot to define the reference features 256A, 256B, 256C, 256D.

The reference features 256A, 256B, 256C, 256D may be arranged on the guide bar 211 in known location both with regard the specific location of the reference features 256A, 256B, 256C, 256D on the guide bar 211, as well as the location of each of the reference features 256A, 256B, 256C, 256D with regard to each of the other the reference features 256A, 256B, 256C, 256D. The known location of the reference features 256A, 256B, 256C, 256D on the guide bar 211 will be utilized to define a position and/or orientation of the guide bar 211 and/or the surgical saw blade cartridge 210 within a known coordinate system of the navigation system 32. The known location of the reference features 256A, 256B, 256C, 256D will be utilized by the navigation system 32 to calculate an expected location of each reference features 256A, 256B, 256C, 256D based on the geometric properties and the mechanical stack up of each of the surgical instrument 14 and the surgical saw blade cartridge 210 as part of the registration process, which will be described in greater detail below.

Referring to Figure 3E, an alternative configuration of a reference feature 356 is illustrated. The reference feature 356 may include a visual marker disposed on a surface of at least one of the plates 112, 114, 116 of the guide bars 111. For example, the reference feature 356 may include a visual mark disposed on the second plate 114 of the guide bar 111. The reference feature 356 may comprise a line disposed on the guide bar 111 capable of being traced. The line may indicate the location of an outline of a portion of the tool mount 18. A pointer 57 may be utilized to trace the line representing the reference feature 356 to define the plane of the surgical saw blade cartridge 110 within the known coordinate system of the navigation system 32. While an example configuration of a path to be traced by the pointer 57 is illustrated using a dotted line in the Figures 3E, it is further contemplated that the reference feature 356 may not be physically and/or visually present on the one of the plates 112, 114, 116 of the guide bars 111. For example, as will be described in greater detail below, the process of registering the surgical saw blade cartridge 110 within the known coordinate system of the navigation system 32 may comprise tracing a path/line along an outline of the tool mount 18 on the guide bar 111 (similar to the path shown via the dotted-line) without having a visual or physical reference feature defined on the guide bar 111. The method may further include tracing a different portion of the tool mount other than what is shown.

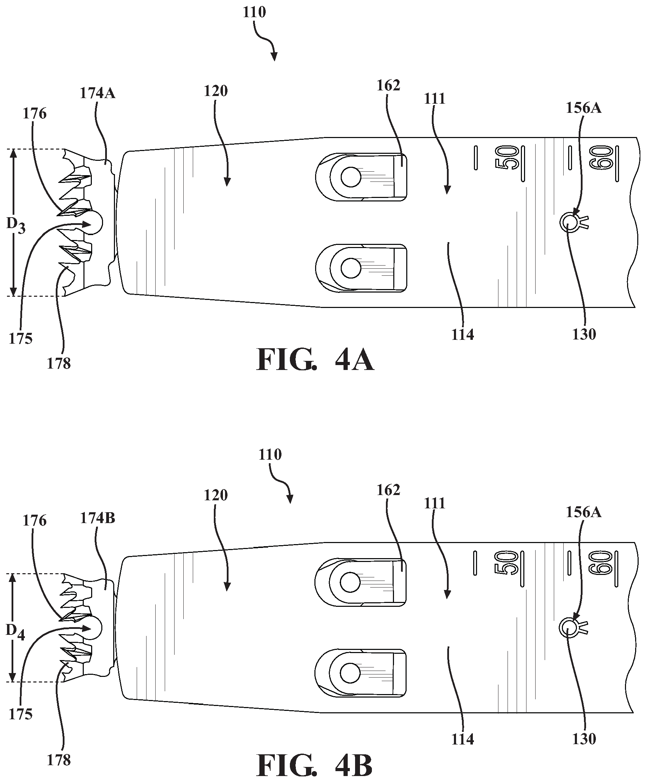

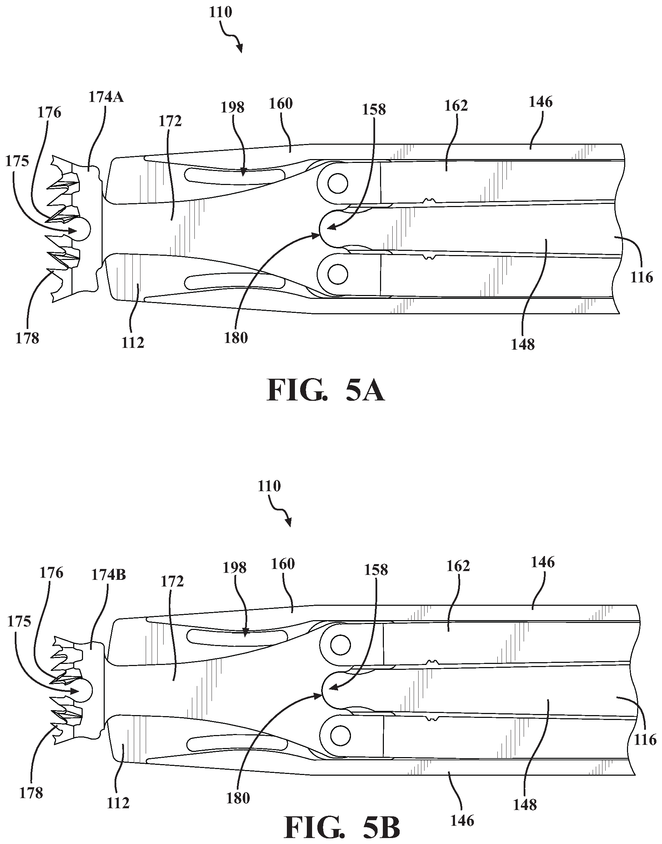

Referring to Figures 4A to 5B, a surgical saw blade cartridge 110 as described above is illustrated including alternative configurations of a blade head 174A, 174B. Similar to the blade head 174 described above, each the configurations of the blade head 174A, 174B illustrated in Figures 4A and 4B comprise two types of teeth, base teeth 178 and one or more pilot teeth 176. Optionally, each of the configurations of the blade head 174A, 174B may also including cut-out or aperture 175. As illustrated in the Figures, the blade head 174A, 174B is formed with a single cut-out 175 formed in a generally tear-drop shape. As described above, the blade head 174A, 174B may be formed with additional cut-out or apertures 175, and the cut-out or aperture 175 may be formed in alternative shapes.

A first configuration of the blade head 174A, as illustrated in Figures 4A and 5A, may comprise a first width D3. Alternatively, a second configuration of the blade head 174B, as illustrated in Figures 4B and 5B, may comprise a second width D4. The blade heads 174A, 174B may be configured such that the first width D3 of the first configuration of the blade head 174A is longer (wider) than the second width D4 of the second configuration of the blade head 174B. While each the blade heads 174A, 174B may generally comprise a similar shape, the first configuration of the blade head 174A and the second configuration of the blade head 174B may be better suited for different surgical procedures. For example, the narrower second configuration of the blade head 174B may be better suited for making smaller cuts and/or cutting in a more confined space. The narrower second configuration of the blade head 174B may also be better suited for making more precise cuts. Alternatively, the wider first configuration of the blade head 174A may be better suited for making larger cuts and/or cutting denser materials or biological tissue.

Likewise, not all configurations of the saw blade cartridge 110, 210 may have all the described features. For example, it is not required that all configurations of the saw blade cartridge 110, 210 include outer tines that extend forward of the inner tine. In yet another example, it may not be necessary that all outer tines include inwardly directed lobes.

Likewise, in some versions of the saw blade cartridge 110, 210, the inner plate 116, 216 may be wholly or partially formed with one or both of the first plate 112, 212 or the second plate 114, 214. Thus, in these versions of the saw blade cartridge 110, 210, once a blank is formed, the blank is machined to define the slots in which the drive rods are seated and that define the perimeter of the tines. Alternatively, the opening- and slot-defining portion of the guide bar may be formed by molding.

In some versions of the saw blade cartridge 110, 210, a single drive link may be all that is needed to pivot the blade head. In some versions of the saw blade cartridge 110, 210 the one or more drive links may not be parallel to the longitudinal axis through the cartridge. Thus, in these versions of the saw blade cartridge 110, 210, the openings in the guide bar through in which elements of the one or more drive links are seated likewise may not be aligned online that are parallel to the longitudinal axis through the cartridge.

In versions of the saw blade cartridge 110, 210 in which the at least one drive link and the blade are provided with complementary teeth, the guide bar may have a structure different from what has been described. Specifically, the guide bar may be formed so that head are which the blade head is not an integral part of one of the plates forming the bar. In these versions of the saw blade cartridge 110, 210, the structure around which the blade pivots may be a pin mounted to or integral with at least one of the plates that form the bar.

In some versions of the saw blade cartridge 110, 210 two or all three of the plates that form the guide bar may not be three distinct plates that are welded or otherwise secured together. In these alternative versions of the saw blade cartridge 110, 210, the plural plates are machined or molded together as a single unit.

Referring to Figures 6A through 6D, a configuration of a surgical instrument 14 and pointer 57 of the surgical system 10 described above is illustrated. The surgical instrument 14 includes a surgical saw blade cartridge 110 removably coupled to the tool platform 16 via a tool mount 18. The surgical instrument 14 may also comprise a tracker 52 coupled to and associated with the tool platform 16 of the surgical instrument 14, the tracker configured to allow the navigation system 32 to determine the pose of the tool platform 16 within the known coordinate system.

The tool platform 16 may further comprise one or more markers 60A, 60B, 60C, 60D. The markers 60A, 60B, 60C, 60D may also be referred to as fiducials or reference features. The markers 60A, 60B, 60C, 60D may be configured to register the pose of the tool platform 16 within the known coordinate system defined by the navigation system 32 using the tracked pointer 57 and touching off on one or more of the markers 60A, 60B, 60C, 60D while tracking the pointer tracker PT and the tracker of the surgical instrument 52.

A surgical saw blade cartridge 110, 210 that is removably coupled to the tool platform 16 of the surgical instrument 14. The surgical saw blade cartridge 110, 210 comprises a guide bar 111, 211 made up of a plurality of plates 112, 114, 116, 212, 214, 216. For example, as is described above, the guide bar 111, 211 may comprise three plates 112, 114, 116, 212, 214, 216 stacked on one another to form the guide bar 111, 211.

The plates 112, 114, 116, 212, 214, 216 of the guide bar 111, 211 may further comprise and/or define one or more reference feature 156, 256. The reference features 156, 256 may serve as a contact point for the pointer 57 for the purpose of registering the surgical saw blade cartridge 110, 210 within the known coordinate system of the navigation system 32. For example, the guide bar 111, 211 may comprise and/or define three reference features 156, 256, that when contacted by the pointer 57 may be utilized to define a plane of the surgical saw blade cartridge 110, 210 within the known coordinate system of the navigation system 32.

As described above, the surgical system may also comprise the pointer 57 for registering the pose of the tool platform 16 and/or the pose of the surgical saw blade cartridge 110, 210 within the known coordinate system of the navigation system 32. The pointer 57 may comprise a pointer tracker PT that is identifiable and trackable by the localizer 44 of the navigation system 32.

In operation, a distal end 59 of the pointer 57 may be touched to the markers 60A, 60B, 60C, 60D of the tool platform 16 and/or the reference feature 156, 256 of the surgical saw blade cartridge 110, 210 to identify the location of the tool platform 16 and/or the surgical saw blade cartridge 110, 210 within the known coordinate system of the navigation system 32 relative to the tracker 52 of the surgical instrument 14. This may be accomplished by the localizer tracking the pose of the pointer 57 as the pointer is moved from each of the markers 60A, 60B, 60C, 60D of the tool platform 16 and/or the reference feature 156, 256 of the surgical saw blade cartridges 110, 210 to the subsequent markers 60A, 60B, 60C, 60D and/or the reference feature 156, 256.

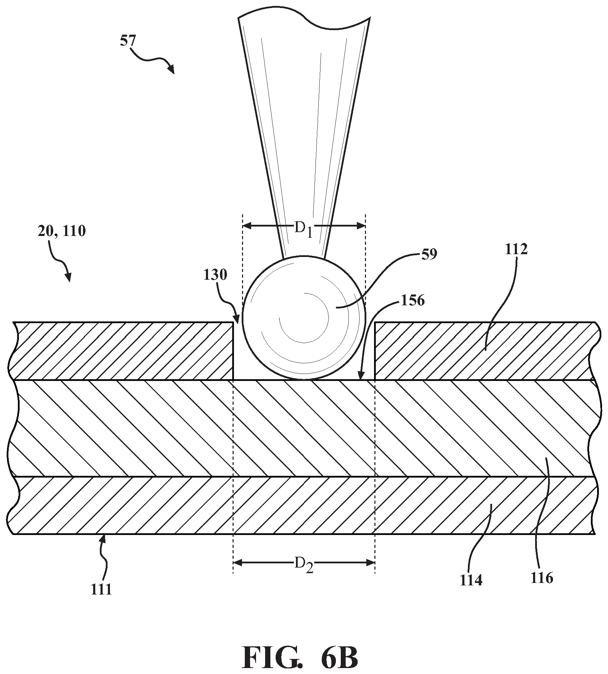

Referring to Figures 6A and 6B, the distal end 59 of the pointer 57 may be formed in the shape of a sphere or ball. As will is described above, the reference feature 156, 256 of the surgical saw blade cartridges 110, 210 may be defined as a recess, indent, divot, indicia, or similar marking on the guide bar 111, 211. As opposed to a physical marking such as a recess or divot, the reference feature 156, 256 may also be defined by a marking, such as a laser marking or similar visual marker on the guide bar 111, 211.

The distal end 59 of the pointer 57 may be sized and/or shaped to fit within the opening 130 defined in the either the first or second plate 112, 114 of the guide bar 111 described above, such that the distal end 59 of the pointer 57 is not inhibited and/or prevented from contacting the reference feature disposed on the third plate 116. For example, as illustrated in Figure 6B, the distal end includes a sphere-shaped tip including a first diameter D1. The opening may comprise a second dimension D2. The second dimension D2 may define a diameter of a circular or oval shaped opening. Alternatively, the second dimension D2 may comprise a length of a side of a triangle and/or the width of a square or rectangle. The combination of the distal end 59 of the pointer 57 and the opening 130 may be configured such that smallest second dimension D2 of the opening 130 is greater than the largest first diameter D1 of the distal end 59. The advantage of using a surgical saw blade cartridge 110, 210 that defines the reference feature 156, 256, 356 without using divots or similar physical alteration to the guide bar 111, 211 is that the manufacturing process is simplified. In this instance, only the opening 130 needs to be machined into the second plate 114, 214, without need to further machine a divot in the third plate 116, 216. Machining divots suitable for navigation verification and registration is a costly and complex process. The sphere-shaped tip on the distal end 59 of the pointer 57 abuts the flat portion of the third plate 114, 214. This abutment serves to define the plane of the third plate 116, 216, and by extension the plane of the surgical saw blade cartridge 110, 210. Because there is a hole in the second plate 114, 214 to provide access to the third plate 116, 216, the accuracy is further enhanced because the opening 130 serves as both a physical guide and a visual guide to ensure that the user places the distal end 59 of the pointer 57 at the appropriate location on the third plate 116, 216. Because the opening 130 in the outer plates 112, 114, 212, 214 is a physical guide, it provides a superior user experience that instills confidence in the registration process. As described above, this registration feature is sufficient as the robotic system primarily requires accuracy of the blade position and/or orientation in one plane, such as y-plane in the primary example.

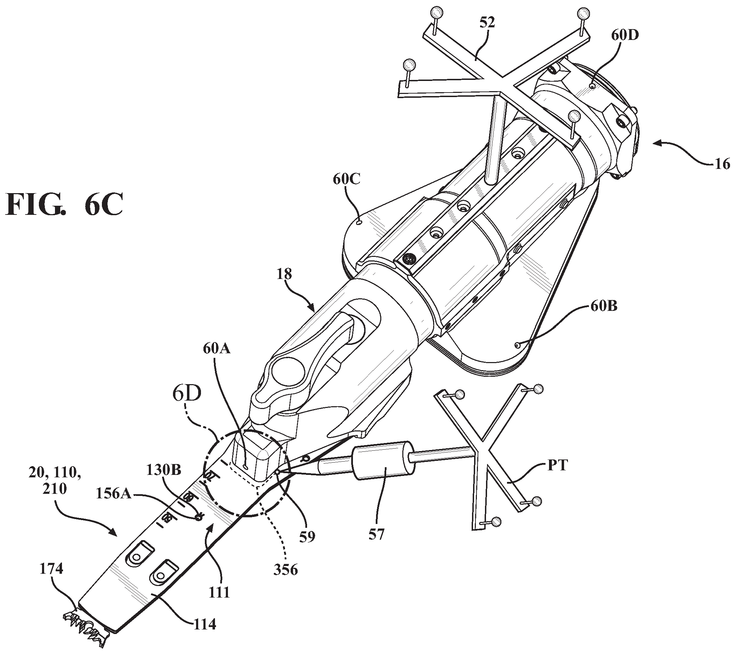

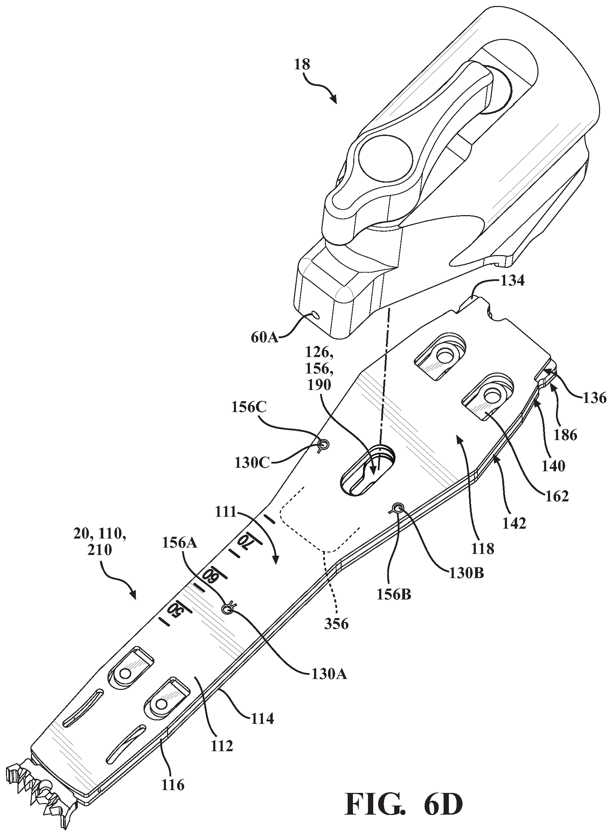

Referring to Figures 6C and 6D, one of the alternative configurations of the reference feature 356 described above for the surgical saw blade cartridge 110, 210 is illustrated. The reference feature 356 may also be defined by a marking, such as a laser marking or similar visual marker on the guide bar 111. As illustrated in Figures 6C and 6D, the alternative configuration of the reference feature 356 may include a visual maker disposed on a surface of one of at least one of the plates 112, 114, 116 of the guide bars 111. For example, the reference feature may include a visual mark disposed on the second plate 114 of the guide bar 111. The reference feature 356 may comprise a line that traces and/or outlines a portion of the tool mount 18, the line to be traced by the pointer 57 to define the plane of the surgical saw blade cartridge 110, 210 within the known coordinate system of the navigation system 32. While an example configuration of a path to be traced by the distal end 59 of the pointer 57 is illustrated using a dotted line in the Figures, it is further contemplated that the reference feature 356 may not be physically and/or visually present on the one of the plates 112, 114, 116 of the guide bars 111. For example, the process of register the surgical saw blade cartridge 110, 210 within the known coordinate system of the navigation system 32 may comprise tracing a path along an intersection of the tool mount 18 and the guide bars 111 (similar to the path shown via the dotted-line) without having a visual or physical reference feature defined on the guide bar 111.

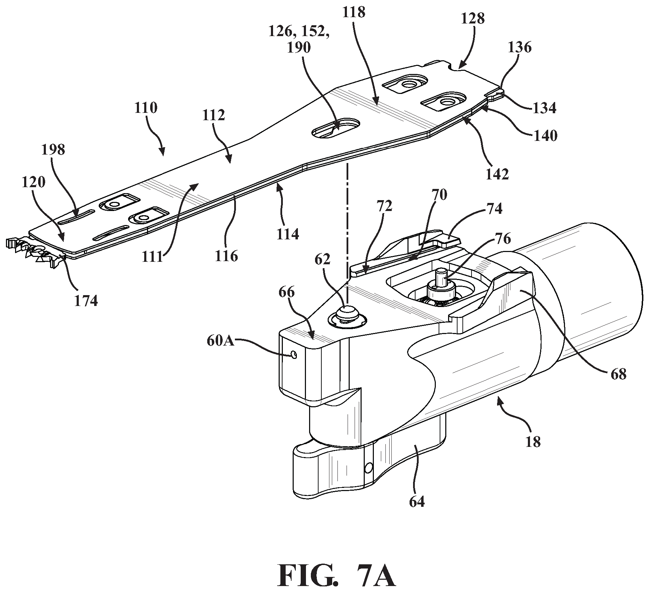

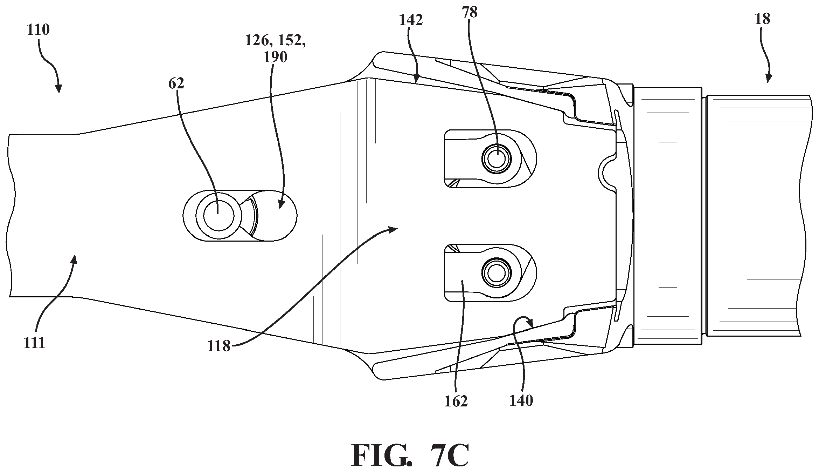

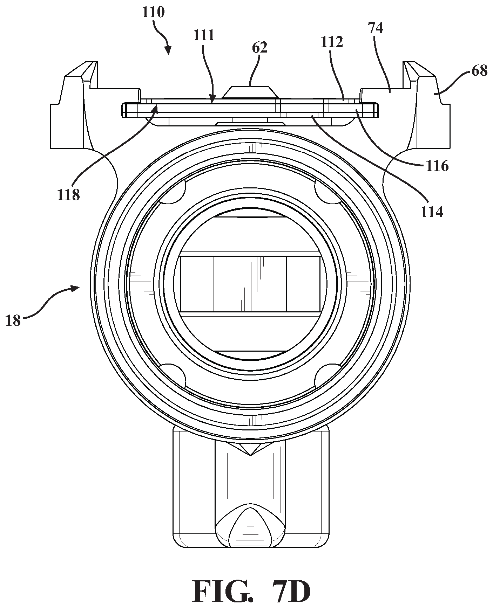

Referring to Figures 7A through 7D, a configuration of a tool mount 18 for securing either of the surgical saw blade cartridges 110, 210 described above to a surgical instrument 14 is illustrated. The mount 18 may comprise a coupling rod 62 configured to removably secure the saw blade cartridge 110, 210 to the mount 18. The coupling rod 62 may interact with the openings 126, 152, 190, 226, 252, 290 of the saw blade cartridge 110, 210, such as the key hole opening 126, 226 to removably couple the saw blade cartridge 110, 210 to the mount 18. The coupling rod 62 may comprise a head configured to fit thought the larger portion of the keyhole opening 126, 226, and the shaft of the coupling rod 62 may be configured to fit within the smaller portion of the keyhole opening 126, 226 after the head has been inserted through the larger portion. The larger portion may then be seated above the smaller portion and configured to removably secure the saw blade cartridge 110, 210 to the mount 18. An exemplary configuration of a mount for a saw blade cartridge is disclosed in International Publication No. WO2007/030793 and U.S. Patent No. 8,696,673, both of which are incorporated herein in their entirety.

The coupling rod 62 may be controlled by a coupling lever 64 configured to manipulate the coupling rod 62 between a locked and unlocked state. For example, the coupling lever 64 may be rotated in one direction to retract the coupling rod 62 to a locked stated configured to secure the saw blade cartridge 110, 210 to the mount 18. Alternatively, the coupling lever 64 may be rotated in the opposite direction to extend or advance the coupling rod 62 to an unlocked stated configured to allow for installation and/or removal of the saw blade cartridge 110, 210 from the mount 18.

The mount 18 may also comprise a lateral mount surface 66 configured to receive the saw blade cartridge 110, 210. When the saw blade cartridge 110, 210 is coupled to the mount, bottom surface of the proximal portion 118 of the first plate 112, 212 may seated adjacent the mounting surface 66. When the coupling rod 62 is in the locked state, the coupling rod 62 may be configured to press the saw blade cartridge 110, 210 against the mount surface 66 to hold the saw blade cartridge 110, 210 in place.

The mount 18 may also comprise a drive rod 76 for actuating the saw blade 170, 270 of the saw blade cartridge 110, 210. The drive rod 76 may comprise at least one drive pin 78 configured to be seated in the holes 166, 266 formed in a drive link 162, 262. While a pair of drive pins 78 are illustrated in the figures, it is contemplated that the mount 18 may be configured to actuate the saw blade 170, 270 of the saw blade cartridge 110, 210 with only a single drive pin 78. In operation, the drive rod 76 may be oscillated and/or partially rotated back and forth causing the drive pin(s) 78 to reciprocate, and in turn reciprocating the drive link 162, 262.

The drive rod 76 may also comprise a biasing member (not shown) configured to urge the drive rod 76 in a proximal direction toward a locked position. The force of biasing member may be overcome by pulling the drive rod 76 in a distal direction toward a loading position. In operation, the holes 166, 266 formed in a drive link 162, 262 may be seated over the drive pin(s) 78 while the drive rod 76 is in the locked position. The saw blade cartridge 110, 210 may then be pulled distally (forward) to overcome the force of the biasing member and urging the drive rod toward the loading position where the saw blade cartridge 110, 210 can be mounted to the mount 18. Once the saw blade cartridge 110, 210 is loaded, i.e. the coupling rod is inserted through the key hole opening 126, 226 and the proximal portion 118, 218, of the second plate 114, 214 is adjacent the mounting surface 66, the saw blade cartridge 110, 210 may be released and the biasing member will move the saw blade cartridge 110, 210 proximally and until it has reached the locked position.

The mount 18 may further comprise a bracket 68 positioned on opposing sides of the lateral mounting surface 66. The bracket 68 may extend vertically (i.e. upward) from the lateral mounting surface 66 and comprise a tab 74 that is configured to at least partially extend over a portion of a lateral surface of one of the plates 112, 114, 116, 212, 214, 216 of the guide bar 111, 211 when the saw blade cartridge 110, 210 is coupled to the mount 18. For example, as illustrated in Figure 7A, the proximal portion 118 of the guide bar 111, 211 may be configured such that one of the first plate or the second plate 112, 114 defines a recess 136 configured to expose a portion 134 of the third plate 116. The recess 136 in one of the first plate or the second plate 112, 114 is such that a portion 134 of the third plate 116 extends beyond the outer perimeter edge 136 of the respective first plate or second plate 112, 114.

The purpose of the pulling the saw blade cartridge 110, 210 distally (forward) to load the saw blade cartridge 110, 210 on the mount 18 is to allow the proximal portion 118, 218 of the saw blade cartridge 110, 210 to clear the tabs 74 of the brackets 68. Then when the biasing member moves the saw blade cartridge 110, 210 proximally, a portion of the proximal portion 118, 218 of the saw blade cartridge 110, 210 is seated between the tab 74 and the lateral mounting surface 66 of the mount 18.

The bracket 68 may also define an interior vertical surface 70, 72. The interior vertical surface 70, 72 may be oriented to provide a vertical surface that is tapered outwardly from the longitudinal axis of the later mounting surface 66. The tapered surface 325 may be configured to match the various tapers of the opposing edges 140, 142, 240, 242 of the proximal portion 118, 218 of the guide bar 111, 211 for the purpose of orienting and/or securing the saw blade cartridge 110, 210 to the mount 18. The tapered surface 70, 72 may be configured to match the various tapers of the opposing edges 140, 142, 240, 242 of the proximal portion 118, 218 of the guide bar 111, 211 such that the biasing member urging the saw blade cartridge 110, 210 proximally will cause the proximal portion 118, 218 of the guide bar 111, 211 to be wedged between the opposed tapered surface 70, 72 of the bracket 68 to axially align the surgical saw blade 110, 210 relative to the surgical instrument 14. It is also contemplated that the proximal portion 118, 218 of the guide bar 111, 211 may be wedged between the opposed tapered surface 70, 72 of the bracket 68 to transversely align the surgical saw blade 110, 210 relative to the surgical instrument 14. The opposing edges 140, 142, 240, 242 of the proximal portion 118, 218 of the guide bar 111, 211 may be formed with various segments having different taper angles (which will be described in greater detail below) and the tapered surface 70, 72 of bracket 68 being formed to match can provide improved alignment of the saw blade cartridge 110, 210 with the mount 18, improving the accuracy of the navigation of the saw blade cartridge 110, 210.

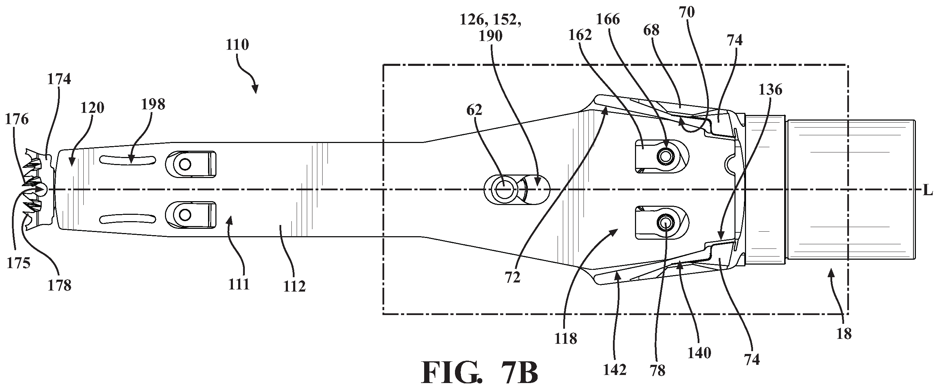

Alternatively, referring to Figure 7B, is also contemplated that only one of the opposed tapered surfaces 70, 72 of the bracket 68 are configured to engage the opposing edges 140, 142, 240, 242 of the proximal portion 118, 218 of the guide bar 111, 211. As illustrated in Figures 7A and 7B, the opposing edges 140, 142, 240, 242 of the proximal portions 118, 218 of the guide bar 111, 211 may comprise a first segment 140, 240 and a second segment 142, 242. The first segment 140, 240 may be oriented a first outward taper angle relative to the longitudinal axis of the guide bar 111, 211 and the second segment 142, 242 may be oriented a second outward taper angle relative to the longitudinal axis of the guide bar 111, 211, with the first segment 140, 240 being oriented at a first outward taper angle that is greater than the second outward taper angle the second segment 142, 242. The first tapered surface 70 of the bracket 68 may be oriented at a third outward taper angle relative to the longitudinal axis of the later mounting surface 66 and the second tapered surface 72 of the bracket 68 may be oriented at a fourth outward taper angle relative to the longitudinal axis of the later mounting surface 66. The third outward taper angle of the first tapered surface 70 of the bracket 68 may be less than or equal to the fourth outward taper angle of the second tapered surface 72 of the bracket 68. Furthermore, the third outward taper angle of the first tapered surface 70 of the bracket 68 configured to match the first outward taper angle of the first segment 140, 240 of the opposing edges 140, 240 of the proximal portions 118, 218 of the guide bar 111, 211 to allow the first segment 140, 240 of the opposing edges 140, 240 of the proximal portions 118, 218 of the guide bar 111, 211 to wedge between the first tapered surface 70 of the bracket 68. The matching tapers of the opposing edges 140, 240 of the proximal portions 118, 218 of the guide bar 111, 211 and the first tapered surface 70 of the bracket 68 may configured as an alignment feature, such that the engagement of the opposing edges 140, 240 of the proximal portions 118, 218 of the guide bar 111, 211 and the first tapered surface 70 of the bracket 68 axially aligns the guide bar 111, 211 within the mount 18. The fourth outward taper angle of the second tapered surface 72 of the bracket 68 may similarly be configured to match the second outward taper angle of the first segment 142, 242 of the opposing edges 142, 242 of the proximal portions 118, 218 of the guide bar 111, 211 and similarly act as an alignment feature to axially aligns the guide bar 111, 211 within the mount 18. However, it is also contemplated that the proximal portion of the guide bar 111 may configured such that the fourth outward taper angle of the second tapered surface 72 of the bracket 68 may be greater than the second outward taper angle of the first segment 142, 242 of the opposing edges 142, 242 of the proximal portions 118, 218 of the guide bar 111, 211, thus creating a gap between the tapered surface 72 of the bracket 68 the first segment 142, 242 of the proximal portions 118, 218 of the guide bar 111, 211. This configuration is illustrated in Figure 7B. This configuration of the proximal portion 118, 218 of the guide bar 111, 211 may be utilized to avoid over constraining the guide bar 111, 211 within the mount 18 and creating additional interferences between the proximal portions 118, 218 of the guide bar 111, 211 and the mount 18 that may negatively impact alignment of the guide bar 111, 211 within the mount 18.

In operation, the saw blade cartridge 110, 210 may be coupled to a surgical instrument 14, such as a robot arm, or a hand-held power tool, including a hand-held robotic instrument. The saw blade cartridge 110, 210 may be fitted to a mount 18 of the surgical instrument 14. To accomplish this, each drive pin 78 of the mount 18 is seated in a separate one of the holes 166, 266 formed in a drive link 162, 262, and the saw blade cartridge 110, 210 may be pulled forward pulling the drive pins 78 and the driver mechanism 76 forward. The saw blade cartridge 110, 210 may then be lowered such that the bottom surface of the first plate 112, 212 is positioned adjacent the mounting surface 66 of the mount 18. In doing so, the coupling rod 62 of the mount 18 may be inserted through the overlapping openings 126, 152, 190, 226, 252, 290 of the saw blade 110, 210. The coupling rod 62 may then be lowered using the coupling lever 64 to lower the head of the coupling rod 62 onto the guide bar 111, 211 so the head of the coupling rod 62 presses against the top surface of the plate(s) 112, 114, 116, 212, 214, 216 that includes the key-hole shaped opening 126, 226. This press action holds the saw blade cartridge 110, 210 to the mount 18. When the saw blade cartridge 110, 210 is secured to the mount 18, the drive pins 78 and drive links 76 cooperate to urge the saw blade cartridge 110, 210 proximally. The proximal portion 118, 218 of the saw blade cartridge 110, 210 is seated adjacent the mount surface 66.

The saw blade cartridge 110, 210 may be actuated by actuating of the motor (not shown) of the surgical instrument 14. The actuation of the motor results in the back-and-forth oscillation of the drive pins 313. The movement of the drive pins 313 causes the drive links 162, 262 to engage in opposed back and forth reciprocation of the drive links 162, 262. The opposed back and forth motion of the drive links 162, 262 causes the saw blade 170, 270 to pivot back and forth around the head 158, 258 of the guide bar 111, 211. The pivoting action of the saw blade 170, 270 causes oscillation of the blade head 174, 274, and by extension the blade teeth 176, 178, 276, 278 causing the teeth to cut the tissue against which the saw blade cartridge 110, 210 is pressed.

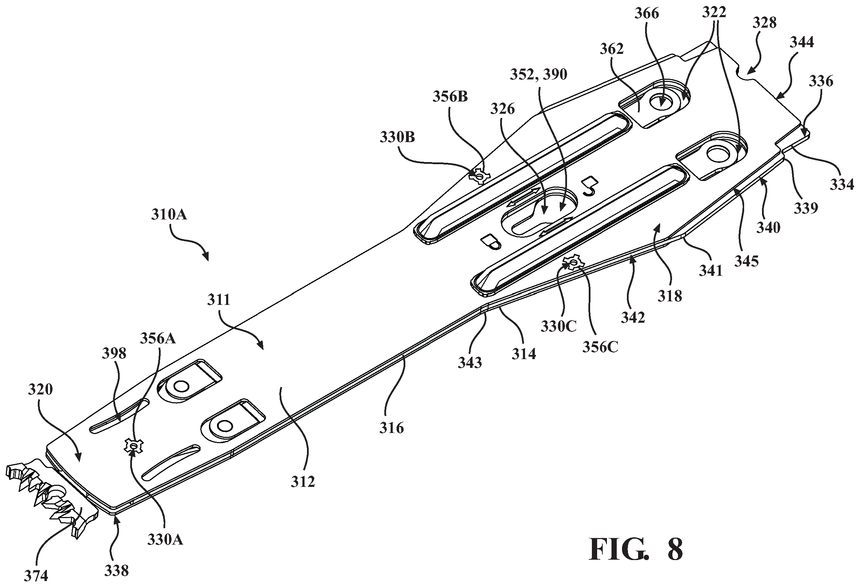

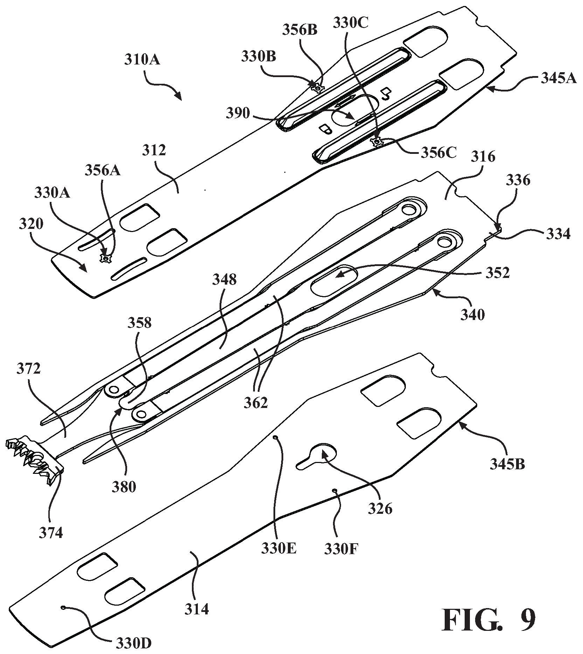

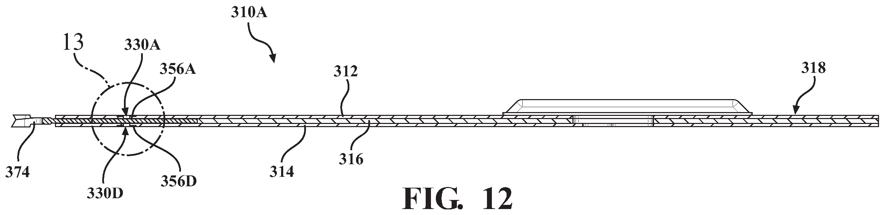

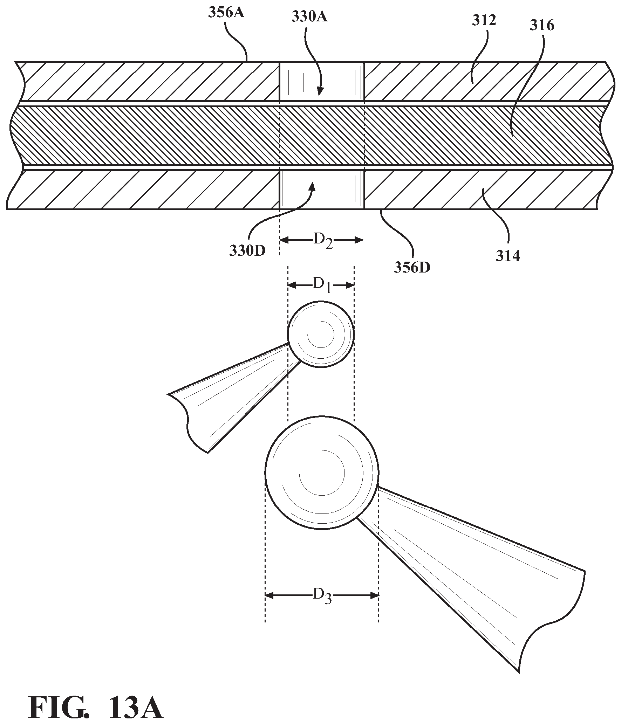

Referring to Figures 8 to 13B, a third configuration of a blade cartridge 310 for use with the surgical instrument 14 of the surgical system of Figure 1 is illustrated. It should be understood that the third configuration of a blade cartridge 310 may include any and/or all of the features of the blade cartridges 110, 210 described above. Furthermore, it should be understood that features of the blade cartridge 310 including the same based number may operate and/or function in the same manner as a component of the blade cartridges 110, 210 including the same base number.

The sagittal blade cartridge 310 may also be referred to as a sagittal blade cartridge, surgical blade cartridge, or cartridge. The blade cartridge 310 may comprises a guide bar 311. The guide bar 311 may comprises a plurality of plates 312, 314, 316, including a first plate 312, a second plate 314, and a third plate 316. The guide bar 311 may be configured such that the third plate 316 may be disposed between the first plate 312 and the second 314. As such, the first plate 312 may also be referred to as the bottom plate, the second plate 314 may be referred to as a top plate, and the third plate 316 may be referred to as an inner plate, center plate, inner member, center layer, and/or center member. While the guide bar 311 is illustrated as only including three plates layered in a laminate type arrangement, it is contemplated that the guide bar 311 may comprise any number of plates and/or layers.

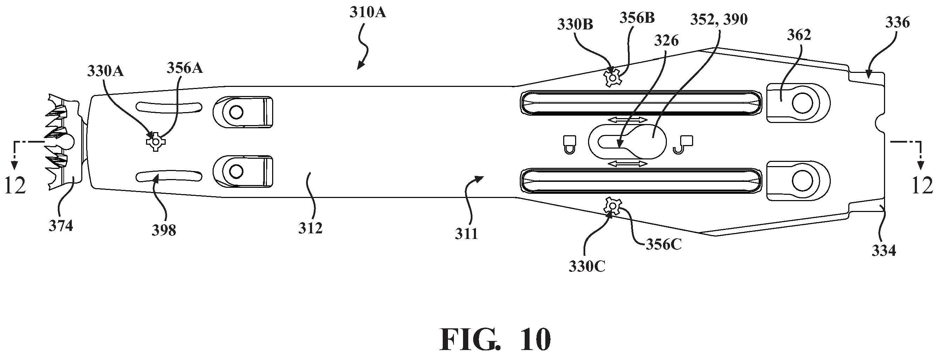

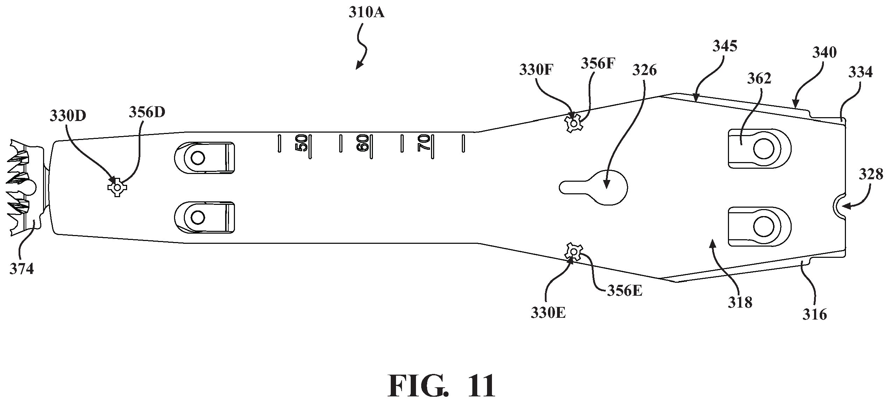

Collectively, the plates are formed so that the guide bar 311 has a proximal portion 318 that forms the proximal end of the plates 312, 314, 116. The proximal portion 318 of the guide bar 311 may be configured to initially taper outwardly as you move distally along the guide bar 311, and then eventually taper inwardly as you move further distally along the guide bar 311. The outward taper of the proximal portion 318 of the guide bar 311 may configured to as a mounting feature to assist in mounting and/or securing the saw blade cartridge 310 to a surgical instrument 14, such as a surgical robot or handheld instrument. For example, as illustrated in Figures 10 and 11, opposed outer edges of the proximal portion 318 of the guide bar 311 may define a contact surface with the tool mount 18. It is further contemplated that one or both of the proximal portion(s) 318 of the first and/or second plates 312, 314 may be shaped to define a recess 336 or include a recess or cutout 336 defined in the outer edge of the proximal portion 318 of the first and/or second plates 312, 314 that exposes a portion 334 of the proximal portion 318 of the third plate 316. The recess 336 may be sized and/or shaped to only expose a small portion 334 of the third plate, such as the tab 334 of the third plate 316 that is exposed by the recess 336 defined in the proximal portion 318 of the first plate 312 as illustrated in Figure 10. Alternatively, the recess 336 of defined by the proximal portion of the first plate 312 may include an entire section of the proximal portion 318 of the first plate 312 that is removed exposed a distal ledge of the third plate along the proximal edge of the proximal portion of the guide bar 311. In either configuration, the portion 334 of the proximal end 318 of the third plate 116 that is exposed by the recess 336 defined in the outer edge of the first or the second plate 312, 314 may be configured to be one of the portions 334 of the guide bar 311 that contacts and/or interacts with the mount 18 of the surgical instrument 14 for the purpose of mounting and/or aligning of the saw blade cartridge 310 relative to the surgical instrument 14. This will be described in greater detail below.

Referring to Figure 10 and 11, the opposed outer edges of the proximal portion 318 of the guide bar 311 may comprise a number of points 339, 341, 343 spaced along the perimeter edge of the proximal portion 318. The points 339, 341, 343 along the edge of the proximal portion 318 of the guide bar 311 may define a plurality of segments 340, 342 of the outer edge of the proximal portion 318 of the guide bar 311, each of which may comprise varying degrees of taper relative to a longitudinal axis L that extends from the proximal 318 to the distal end 320 of the guide bar 311. The taper and/or orientation of each of the segment 340 of the opposed outer edges of the proximal portion 318 of the guide bar 311 may be correspond to and/or be matched to an angle and/or taper of a surface of the bracket 68 of a mount 18 for securing the saw blade cartridge 310 to the surgical instrument 14, as will be described in greater detail below.

The proximal portion 318 of the guide bar 311 may further comprise a proximal edge 344 that is oriented to be generally perpendicular to the longitudinal axis of the guide bar 311. A recess 328 may be defined in the proximal edge 344 of the guide bar 311. The recess 328 may be formed in the proximal edge 344 of the guide bar 311 one side or the other of the longitudinal axis L of the guide bar 311. The recess 328 may serve as an alignment and/or positioning aide when manufacturing the guide bar 311, including manufacturing of the individual plates 312, 314, 316 forming the guide bar. It is further contemplated that the recess 328 formed in each of the first, second, and/or third plates 312, 314, 316 may vary in size and/or shape. For example, as illustrated in Figures 10 and 11, the recess 328 formed in the first plate and the third plate 312, 316 is cut deeper into the proximal portion the respective plates and has a larger radius that the recess 328 formed in the second plate 314. This may allow the manufacture of the guide bar 311 to distinguish between the first and second plates 312, 314, which may otherwise appear very similar to one another. The difference in the size and/or shape of the recess 128 formed in the first and second plates 312, 314 may also assist the user in properly mounting the guide bar to the mount of the surgical instrument 14.

Forward of the proximal portion 318 of the guide bar 311 is a middle portion and a distal portion 320. The plates 312, 314, 316 are formed so that the opposed sides of the guide bar that form the sides of the middle portion are generally parallel. The opposed sides of the distal portion 120 of the guide bar 311 may be tapered in some configurations. As illustrated in the figures, opposed edges 332, 399 of the distal portion 320 of the guide bar 311 may be tapered inwardly toward the center of the plates 312, 314, 316. It is also contemplated that the opposed edges of the distal portion 320 of the guide bar 311 may be generally parallel to one another. The width across the middle portion and/or the distal portion 320 of the guide bar 311 may be less that the width of the proximal portion 318 at the widest point between the opposed edges of the proximal portion 318 of the guide bar 311.

The guide bar 311 may comprise a plurality of openings 330A, 330B, 330C, 330D, 330E, 330F corresponding to a plurality of reference features 356A, 356B, 356C, 356D, 356E, 356F to allow the navigation system 32 to define a plane of the surgical saw blade cartridge 310. However, additional openings 330A, 330B, 330C, 330D, 330E, 330F and corresponding reference features 356A, 356B, 356C, 356D, 356E, 356F may be defined on the guide bar 311. The openings 330A, 330B, 330C, 330D, 330E, 330F may be arranged to provide access to a reference feature 356A, 356B, 356C, 356D, 356E, 356F defined on the inner plate 316. Alternatively, the openings 330A, 330B, 330C, 330D, 330E, 330F defined by the first and/or the second plate 312, 314 may themselves define the reference features 356A, 356B, 356C, 356D, 356E, 356F of the guide bare 311.

The openings 330A, 330B, 330C, 330D, 330E, 330F and corresponding reference features 356A, 356B, 356C, 356D, 356E, 356F may be spaced apart from another and arranged on the guide bar 311 such that a straight line cannot be drawn which intersects all of the reference features 356A, 356B, 356C, 356D, 356E, 356F of a single plate 312, 314, 316. As illustrated in Figures 8 to 13B, the reference features 356 may be arrange in a generally triangular shape on any of the respective plates 312, 314, 316 of the guide bar. Furthermore, as illustrated in Figures 8 to 13B the openings 330A, 330B, 330C, 330D, 330E, 330F and/or the reference features 356A, 356B, 356C, 356D, 356E, 356F defined on the plates 312, 314, 316 may be arrange on the respective plates 312, 314, 316 such that the each of the openings 330A, 330B, 330C, 330D, 330E, 330F and/or each of the reference features 356A, 356B, 356C, 356D, 356E, 356F of one plate 312, 314, 316 are concentrically aligned with an openings 330A, 330B, 330C, 330D, 330E, 330F and/or a reference features 356A, 356B, 356C, 356D, 356E, 356F of another plate 312, 314, 316 when the plates 312, 314, 316 are assigned as the guide bar 311.

It is further contemplated that the guide bar 311 may be configured to define openings 330A, 330B, 330C, 330D, 330E, 330F and/or reference features 356A, 356B, 356C, 356D, 356E, 356F on at least two different surfaces of the plates 312, 314, 316. This may include defining openings 330A, 330B, 330C, 330D, 330E, 330F and/or reference features 356A, 356B, 356C, 356D, 356E, 356F on opposed surfaces of a single plate such as the third plate 316. Alternatively, it may comprise defining openings 330A, 330B, 330C, 330D, 330E, 330F and/or reference features 356A, 356B, 356C, 356D, 356E, 356F on a surface of two different plates 312, 314, 316. For example, as is illustrated in Figures 8 to 12, the guide bar 311 may be configured to such that openings 330A, 330B, 330C, 330D, 330E, 330F and/or reference features 356A, 356B, 356C, 356D, 356E, 356F are defined on a surface of each of the first plate and the second plate 312, 314. The collection of references points may be configured to register the position of the guide bar 311 within the known coordinate system of the navigation system and/or to verify proper loading of the guide bar within the surgical manipulator 14.

The reference features 356A, 356B, 356C, 356D, 356E, 356F, and by extension the openings 330A, 330B, 330C, 330D, 330E, 330F, may be arranged on the guide bar 311 in a known location both with regard the specific location of the reference features 356A, 356B, 356C, 356D, 356E, 356F on the guide bar 311, as well as the location of each of the reference features 356A, 356B, 356C, 356D, 356E, 356F with regard to each of the other the reference features 356A, 356B, 356C, 356D, 356E, 356F. The known location of the reference features 356A, 356B, 356C, 356D, 356E, 356F on the guide bar 311 will be utilized to define a position of the guide bar 311 and/or the surgical saw blade cartridge 310 within a known coordinate system of the navigation system 32. The known location of the reference features 356A, 356B, 356C, 356D, 356E, 356F will be utilized by the navigation system 32 to calculate an expected location of each reference features 356A, 356B, 356C, 356D, 356E, 356F based on the geometric properties and the mechanical stack up of each of the surgical instrument 14 and the surgical saw blade cartridge 310 as part of the registration process, which will be described in greater detail below.

The the reference feature(s) 356 may be defined as a recess, indent, divot, indicia, or similar marking defined on any of the plates 312, 314, 316, of the guide bar 311. This may include a divot on a surface of any of the plates 312, 314, 316. It is further contemplated that the reference feature 356 may comprise a marking, such as a laser marker (without any physical recess, divot or aperture) defined on any of the plates 312, 314, 316. The marking may be realized as an etch, color, engraving, or similar optical marking.

Referring to Figueres 13A and 13B, an example configuration of two separate distal ends 59 of a pointer 57 are illustrated with regard to the reference features 356A, 356B, 356C, 356D, 356E, 356F and/or the openings 330A, 330B, 330C, 330D, 330E, 330F defined in the guide bar 311. The distal end 59 of a pointer 57 may be sized and/or shaped to fit within the opening 330 defined in the either the first or second plate 312, 314 of the guide bar 311 as is described above, such that the distal end 59 of the pointer 57 is not inhibited and/or prevented from contacting a reference feature 356 disposed on the third plate 316. For example, as illustrated in Figure 13A, the distal end of a first configuration of a distal end 59 of a pointer 57 includes a sphere-shaped tip including a first diameter D1. The opening may comprise a second dimension D2. The second dimension D2 may define a diameter of a circular or oval shaped opening. Alternatively, the second dimension D2 may comprise a length of a side of a triangle and/or the width of a square or rectangle. The combination of the distal end 59 of the pointer 57 and the opening 330 may be configured such that smallest second dimension D2 of the opening 330 is greater than the largest first diameter D1 of the distal end 59. The advantage of using a surgical saw blade cartridge 310, 210 that defines the reference feature 356 without using divots or similar physical alteration to the guide bar 311 is that the manufacturing process is simplified. In this instance, only the opening 330 needs to be machined into the second plate 314, without need to further machine a divot in the third plate 316. Machining divots suitable for navigation verification and registration is a costly and complex process. The sphere-shaped tip on the distal end 59 of the pointer 57 abuts the flat portion of the third plate 316. This abutment serves to register the position of the surgical blade within a known coordinate system of the navigation system.

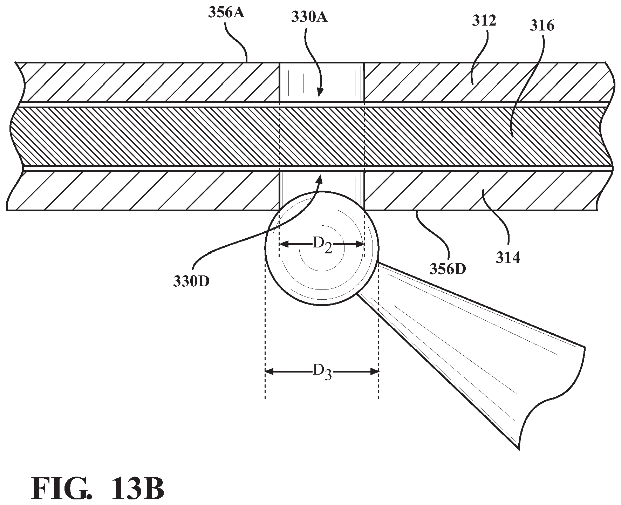

Alternatively, the distal end 59 of a pointer 57 may be sized and/or shaped to be larger than the opening 330 defined in the either the first or second plate 312, 314 of the guide bar 311, such that the distal end 59 of the pointer 57 is inhibited and/or prevented from contacting the third plate 316. In this configuration, of the guide bar 311, the opening 330 is configured to serve as the reference feature 356, where the distal end 59 of a pointer 57 is sized and/or configured to touch off on the opening 330. For example, as illustrated in Figure 13B, the distal end of a first configuration of a distal end 59 of a pointer 57 includes a sphere-shaped tip comprises a third dimensions D3 that is greater than the second dimension D2 of the opening 330. As is illustrated in Figure 13B, the distal end 59 of a pointer 57 may be manipulated to contact the opening 330 defined in the first and/or second plate 312, 314 without contacting the third plate 316. The first and/or second plate 312, 314 may also define a reference 356 such as recess, detent, or the like to be contacted by the distal end 59 of a pointer 57 to register the blade to the navigation system.

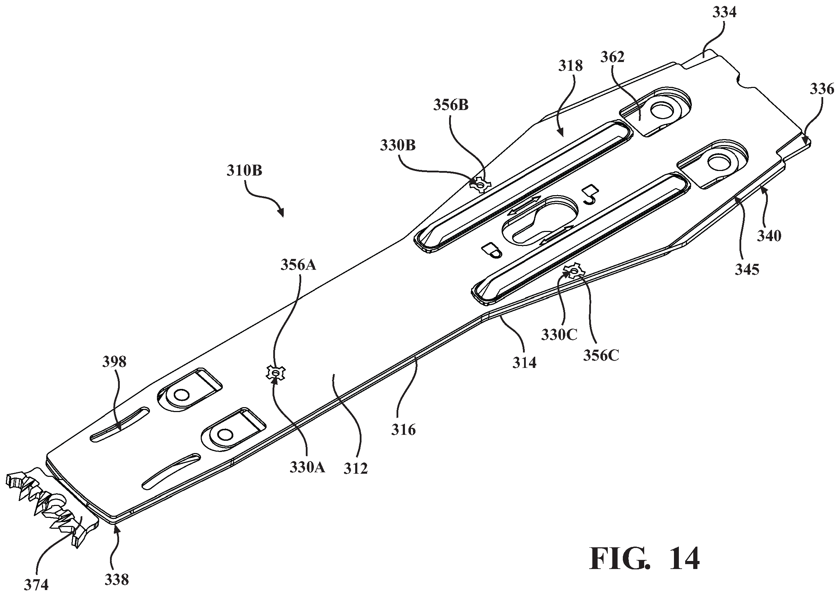

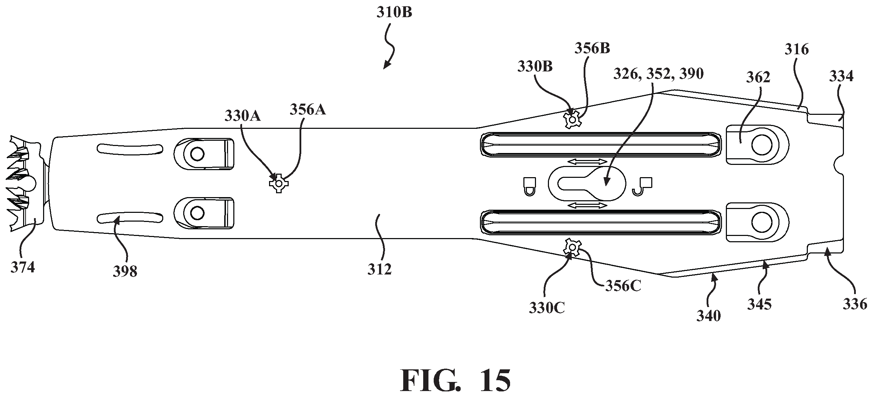

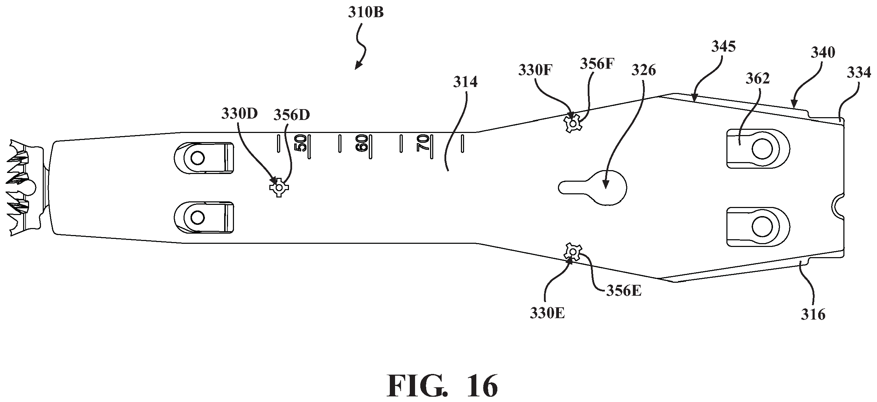

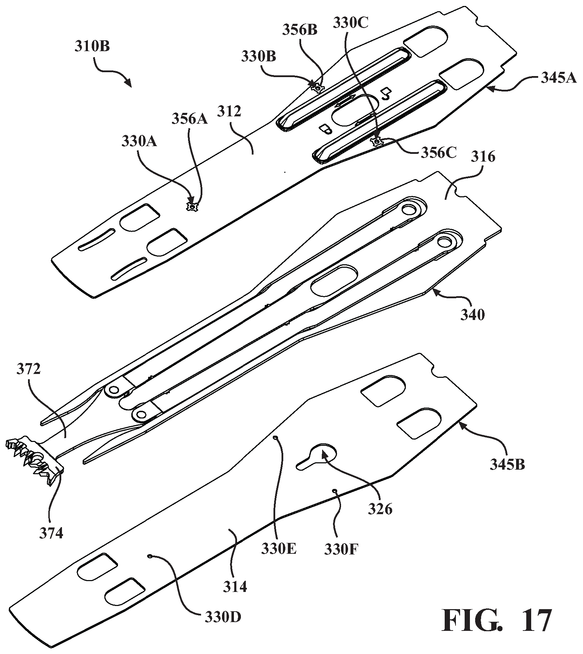

Referring to Figures 14 to 17, the third configuration of the surgical blade 310B is illustrated including an alternative arrangement of the references features 356 defined by the plates 312, 314, 316. As illustrated in Figures 14 to 17, the reference features 356 may be arrange in a generally triangular shape on any of the respective plates 312, 314, 316 of the guide bar. Furthermore, as illustrated in Figures 14 to 17 the openings 330A, 330B, 330C, 330D, 330E, 330F and/or the reference features 356A, 356B, 356C, 356D, 356E, 356F defined on the plates 312, 314, 316 may be arrange on the respective plates 312, 314, 316 such that the each of the openings 330A, 330B, 330C, 330D, 330E, 330F and/or each of the reference features 356A, 356B, 356C, 356D, 356E, 356F of one plate 312, 314, 316 are concentrically aligned with an openings 330A, 330B, 330C, 330D, 330E, 330F and/or a reference features 356A, 356B, 356C, 356D, 356E, 356F of another plate 312, 314, 316 when the plates 312, 314, 316 are assembled as the guide bar 311. In the configuration of the surgical blade 310B, the distal most opening 330A, 330D and/or reference feature 356A, 356D defined in the guide bar 311 are located distally on the guide bar 311 relative to the of the distal most opening 330A, 330D and/or reference feature 356A, 356D of the blade cartridge 310A of Figures 8 to 12.

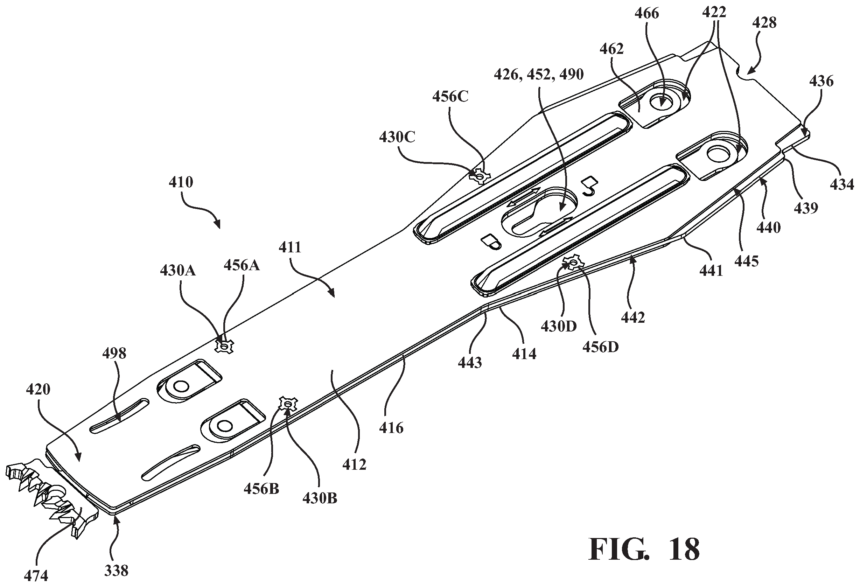

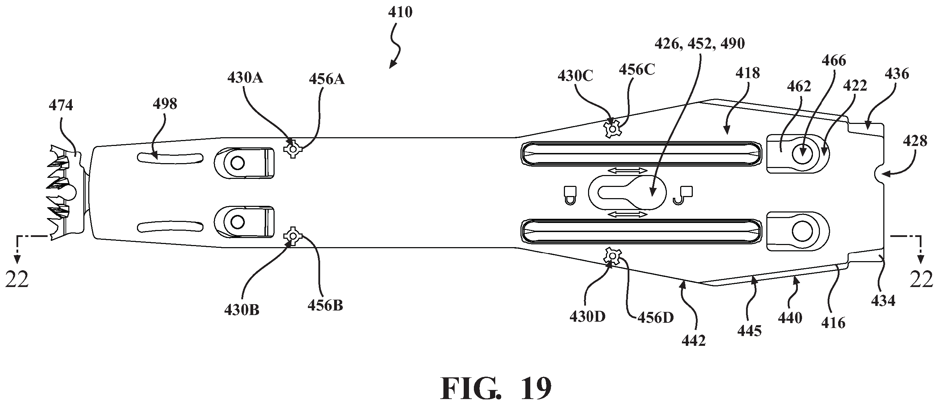

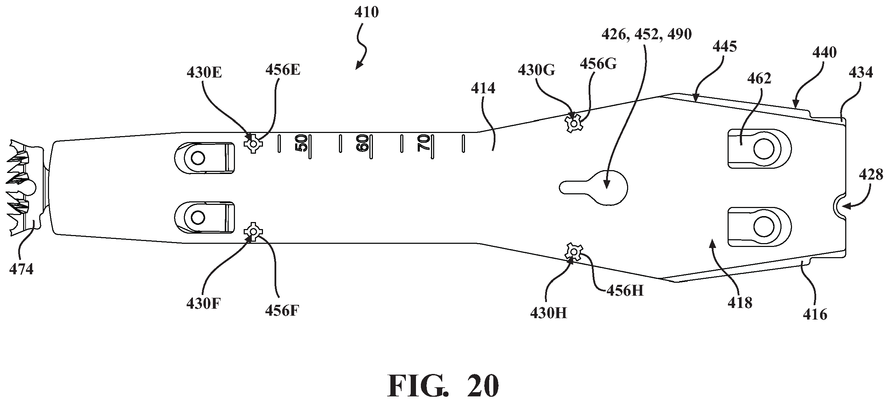

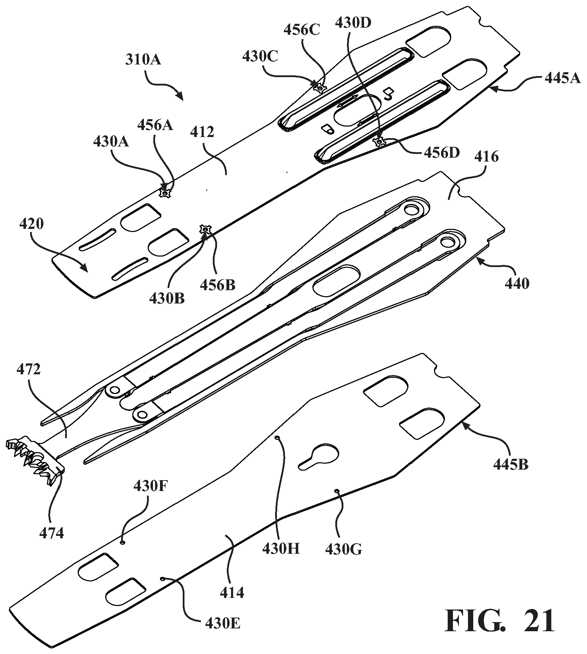

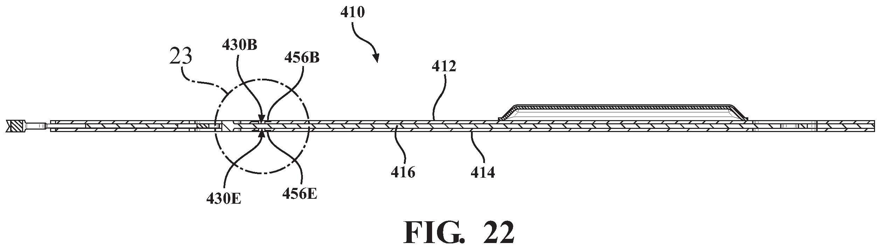

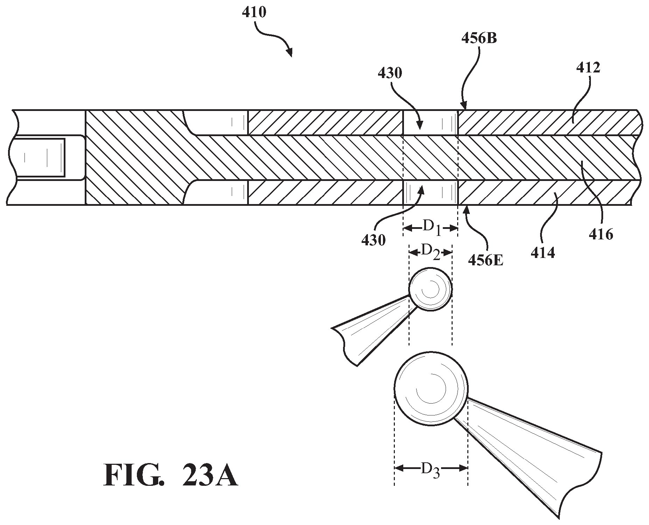

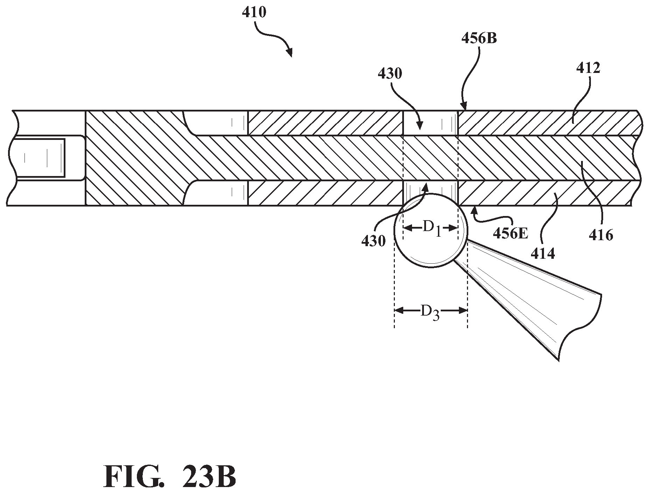

Referring to Figures 18 to 23B, a fourth configuration of a blade cartridge 410 for use with the surgical instrument 14 of the surgical system of Figure 1 is illustrated. The blade cartridge 410 includes may include any or all of the features of any of the configurations of blade cartridges 110, 210, 310 described above. Furthermore, it should be understood that features of the blade cartridge 410 including the same based number may operate and/or function in the same manner as a component of the blade cartridges 110, 210, 310 including the same base number.

The blade cartridge 410 illustrated in Figures 18 to 22 generally includes all of the features of the third configuration of the blade cartridge 410, with an alternative arrangement of the references features 456 defined by the plates 412, 414, 416. As illustrated in Figures 18 to 22, the openings 430 and/or reference features 456 may be arrange in a generally rectangular or trapezoid shape on any of the respective plates 412, 414, 416 of the guide bar 411. Furthermore, as illustrated in Figures 18 to 22 the openings 430A, 430B, 430C, 430D, 430E, 430F, 430 G, 430H and/or the reference features 456A, 456B, 456C, 456D, 456E, 456F, 456G, 456H defined on the plates 412, 414, 416 may be arrange on the respective plates 412, 414, 416 such that the each of the openings 430A, 430B, 430C, 430D, 430E, 430F, 430 G, 430H and/or each of the reference features 456A, 456B, 456C, 456D, 456E, 456F, 456G, 456H of one plate 412, 414, 416 are concentrically aligned with an openings 430A, 430B, 430C, 430D, 430E, 430F, 430 G, 430H and/or a reference features 456A, 456B, 456C, 456D, 456E, 456F, 456G, 456H of another plate 412, 414, 416 when the plates 412, 414, 416 are assembled as the guide bar 411.

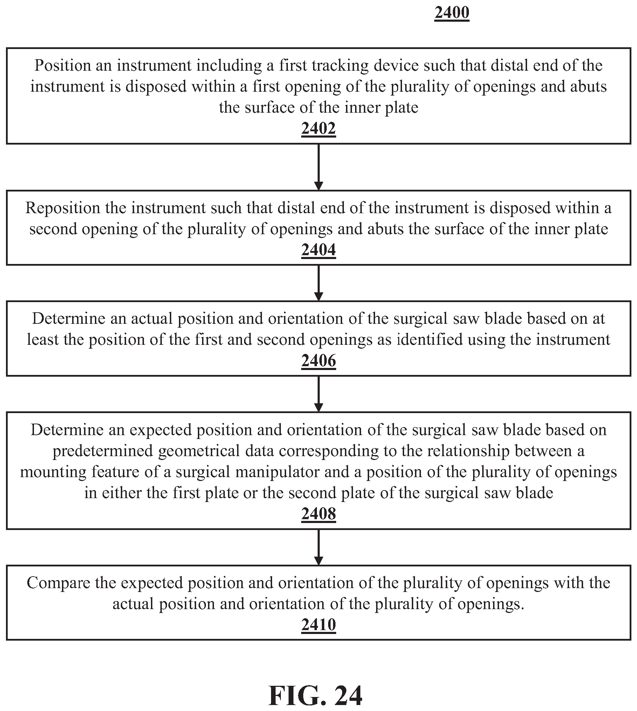

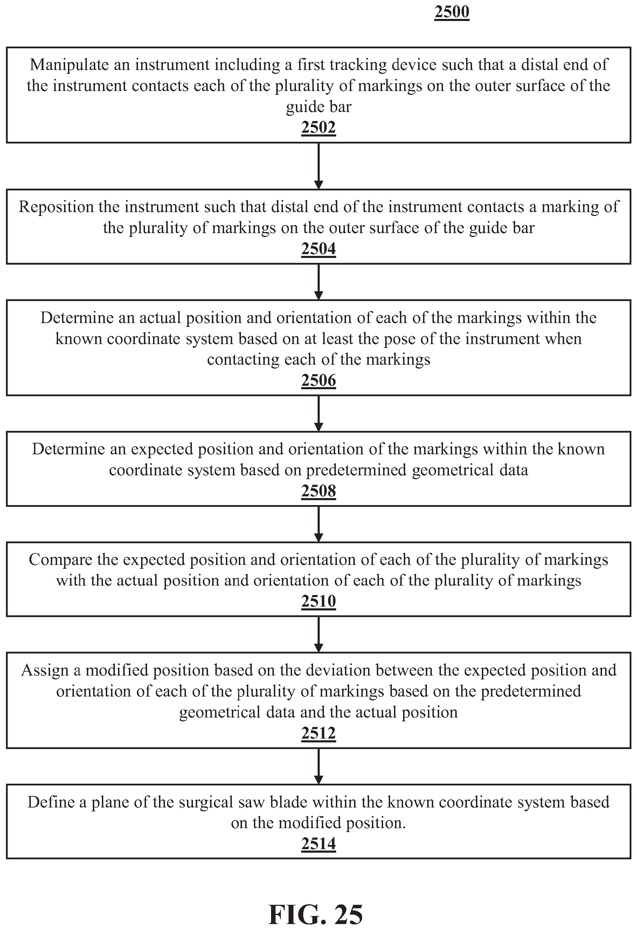

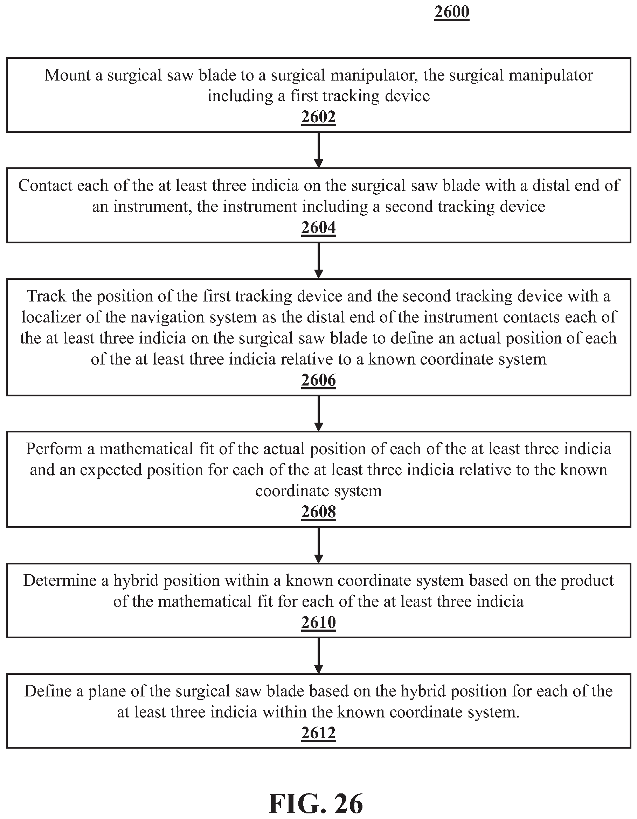

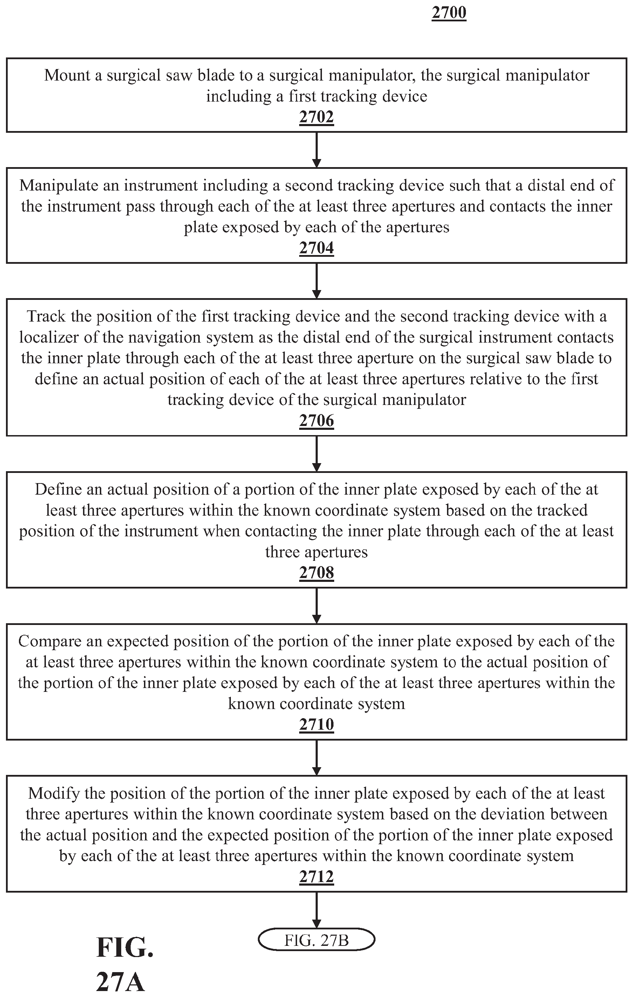

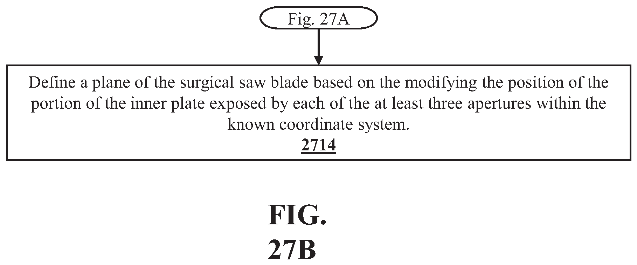

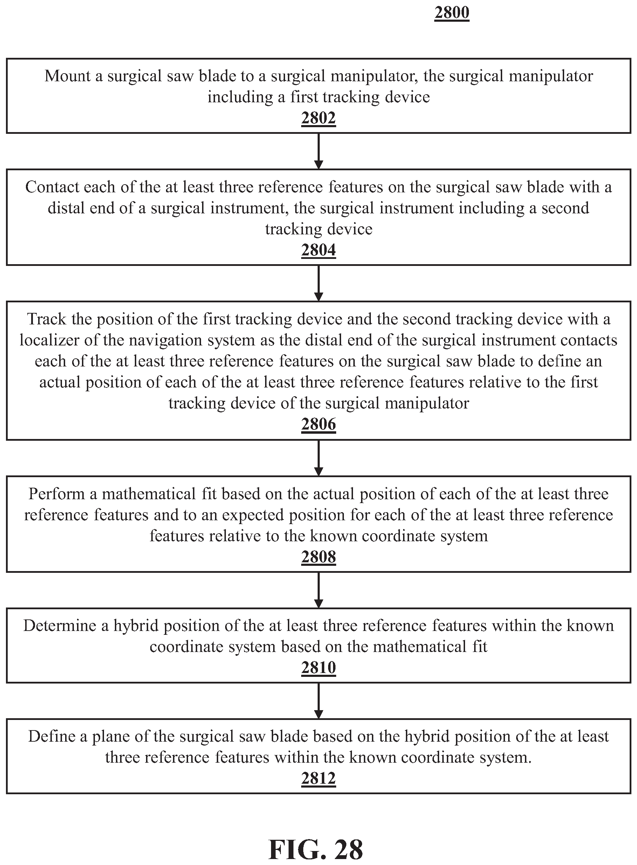

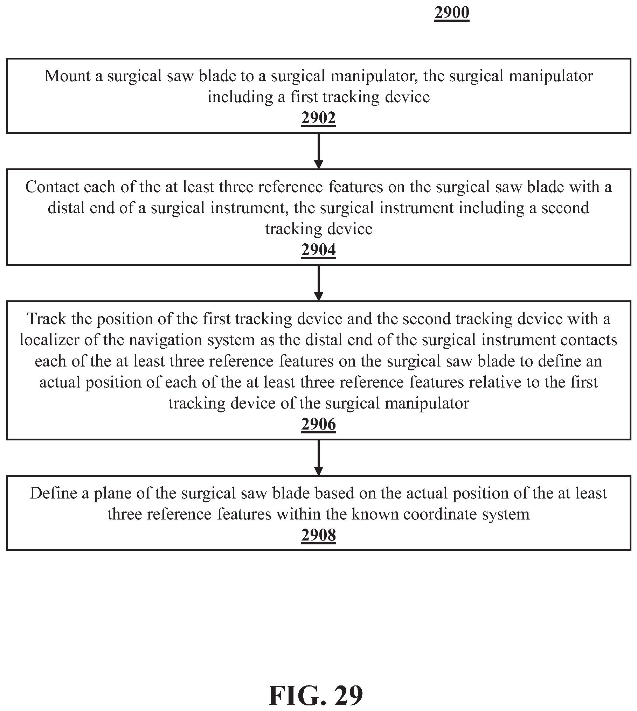

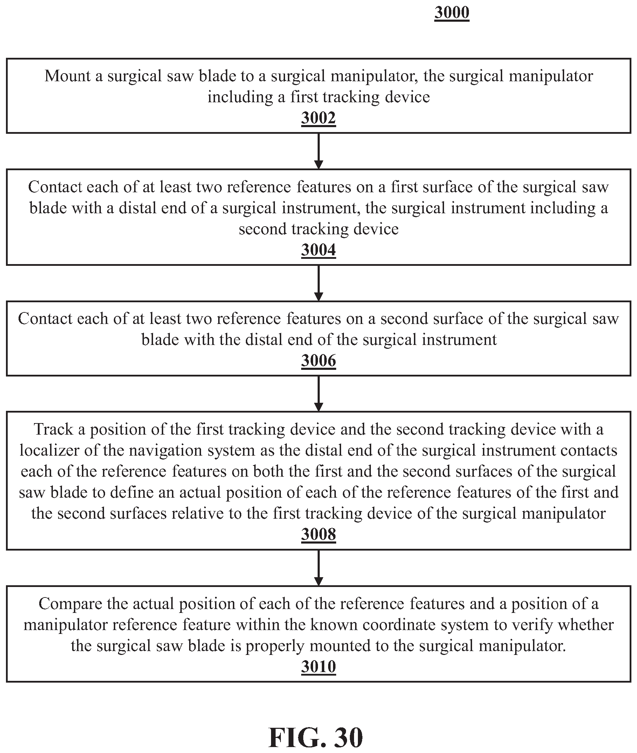

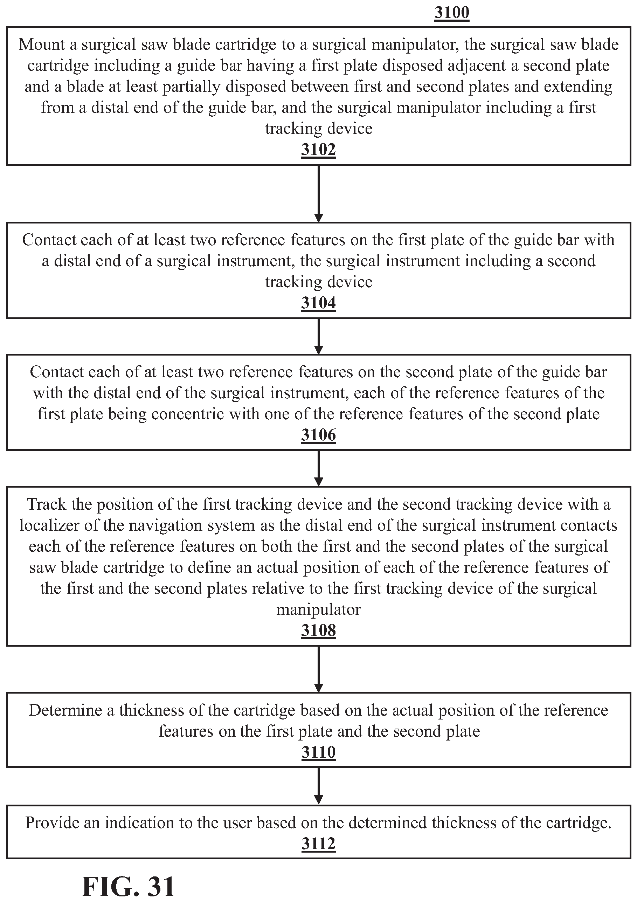

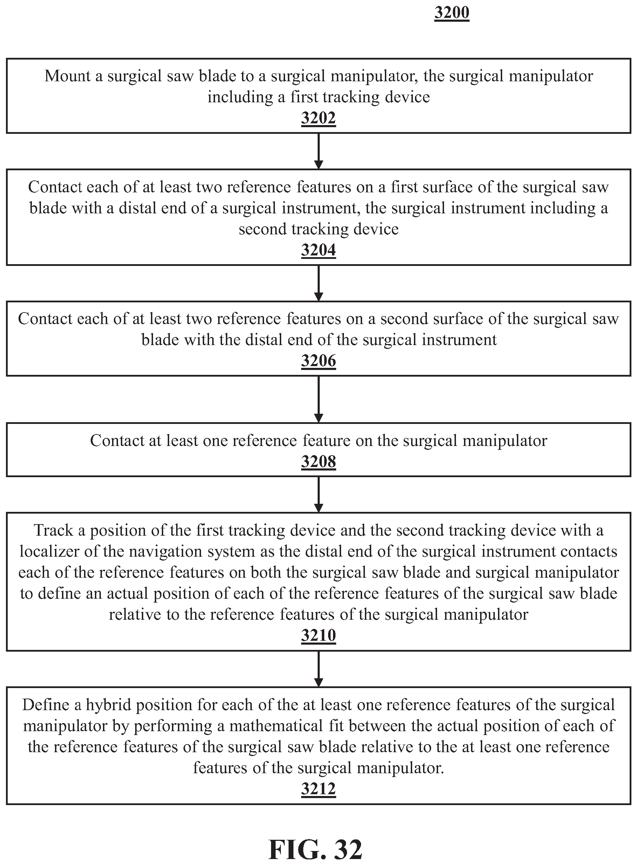

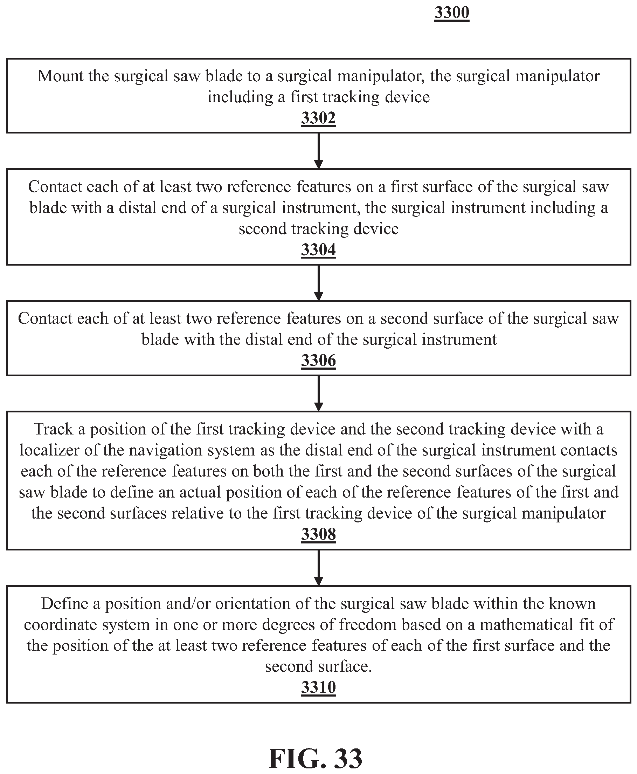

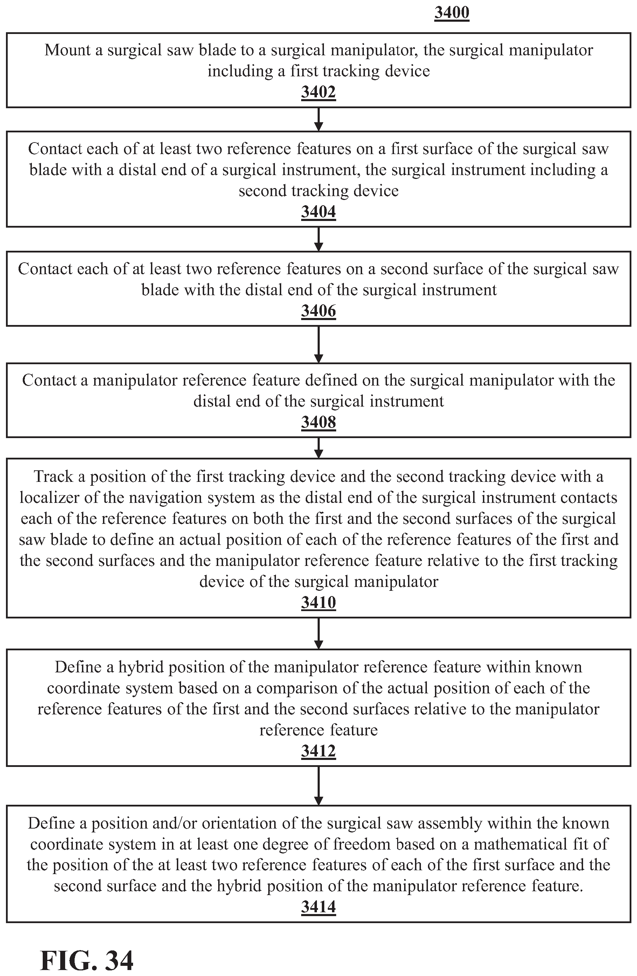

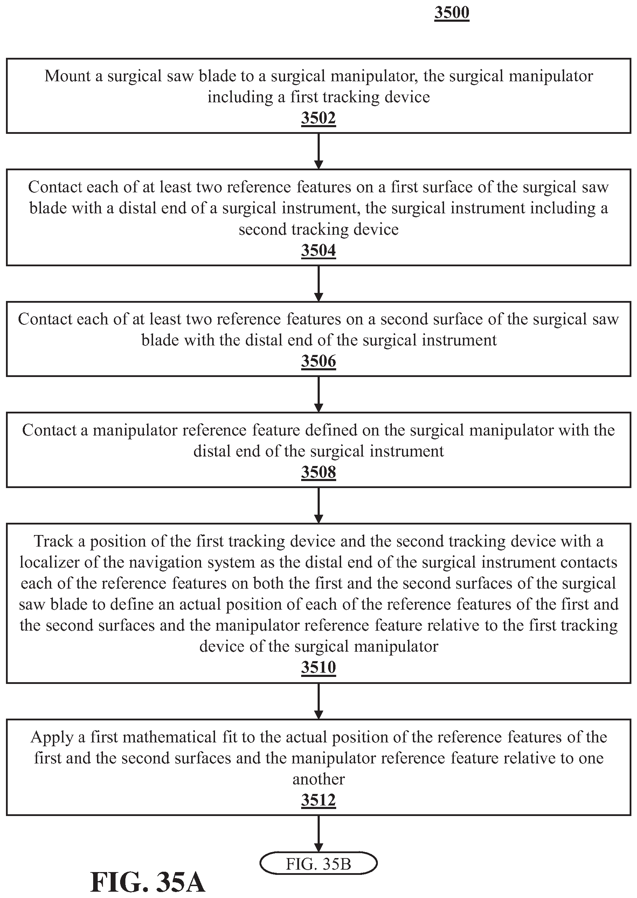

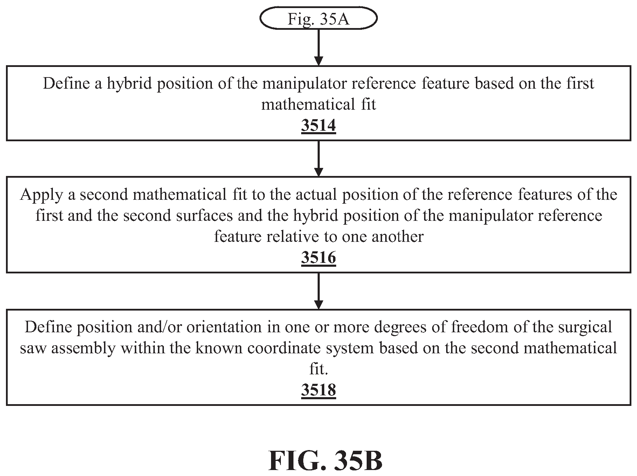

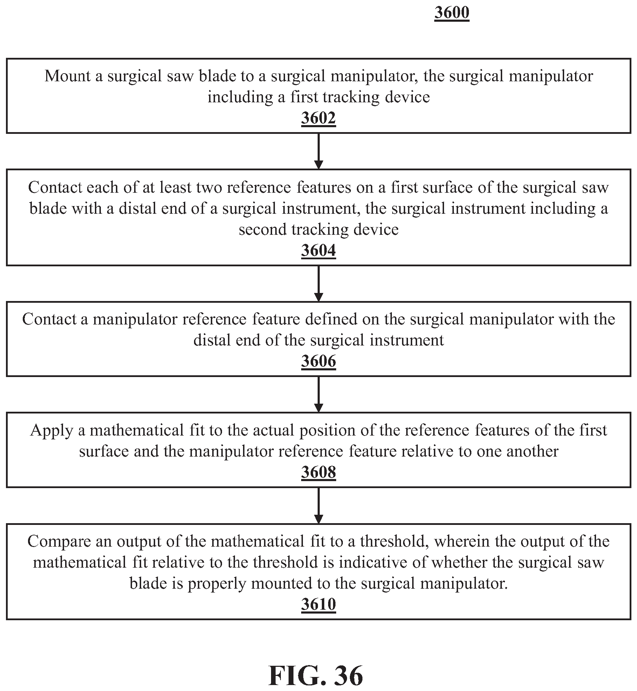

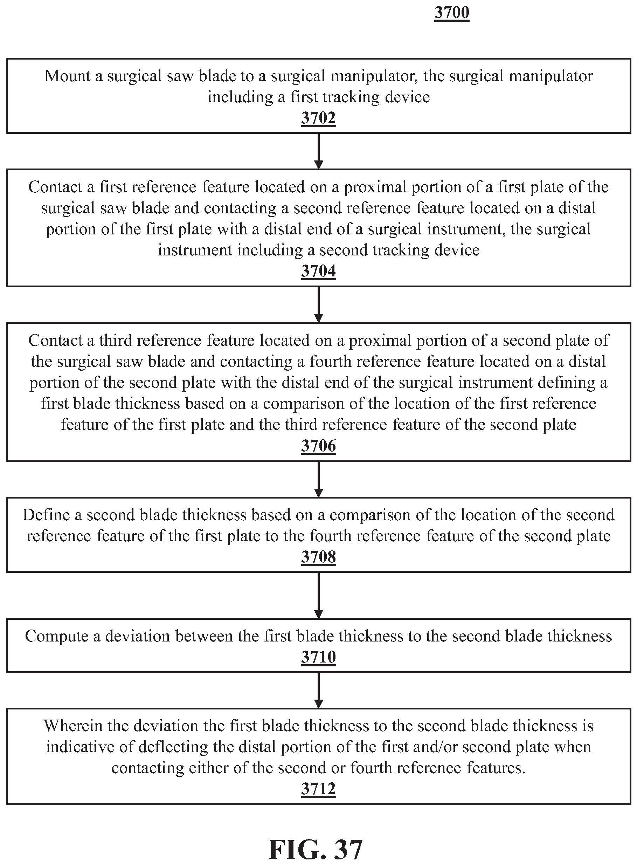

Referring to Figures 24 to 37, various methods of registration and/or verification are illustrated in flow chart form. The various registration and /or verification processes are described in more detail below.

### Registration Process:

Once the saw blade cartridge 110, 210, 310, 410 has been mounted to the mount 18 and/or the surgical instrument 14, the reference feature 156, 256, 356, 456 of the saw blade cartridge 110, 210, 310, 410 may be utilized to register and/or verify the pose of the saw blade cartridge 110, 210, 310, 410 with the navigation system 32. Referring back to Figures 6A to 6D various configurations of a system for registering the surgical saw blade cartridge 110 that is coupled to a surgical instrument 14 are illustrated.

The of registering the surgical saw blade cartridge 110, 210, 310, 410 within a known coordinate system defined by a navigation system 32 may comprise a step of determining an expected position of the reference feature 156, 256, 356, 456 based on predetermined geometrical data corresponding to the relationship between a mounting feature and the reference feature 156, 256, 356, 456 of the saw blade cartridge 110, 210 as described above. An expected position may be calculated and/or determined by the navigation system 32 based on the known geometrical data for the surgical instrument 14, the mount 18, and/or the surgical saw blade cartridge 110, 210, 310, 410 and the pose of the tracker 52 of disposed on the surgical instrument 14. Using the known geometric data or the various components and the pose of the tracker 52 of disposed on the surgical instrument 14, the navigation system may compute the transform to determine the expected position of the reference feature 156, 256, 356, 456.

The method of registering the surgical saw blade cartridge 110, 210 within a known coordinate system may further comprise the step of determining the actual position of the surgical saw blade cartridge 110, 210, 310, 410 within the known coordinate system defined by the navigation system 32. To determine the actual position of the surgical saw blade cartridge 110, 210, 310, 410 within the known coordinate system a pointer 57 may be utilized. The step may comprise touching a pointer 57 or instrument including a pointer tracker PT to one or more of the reference feature 156, 256, 356, 456 defined and/or disposed on the guide bar 111, 211, 311, 411. For example, in one configuration of the surgical saw blade cartridge 110 described above, the guide bar 111 may comprise an opening 130 defined in one or more of the plates 112, 114, of the guide bar 111 to provide access to the reference feature 156 disposed on the inner plate 116, 216. In this configuration touching the pointer 57 to the reference feature 156 may comprise inserting a distal end 59 of the pointer 57 within and/or through the openings 130 defined in at least one of the first plate 112, 212 or the second plate 114, 214 to contact the reference feature 156. The navigation system 32 may track the pose of the pointer 57 via the pointer tracker PT as the pointer 57 is manipulated to contact each of the reference feature(s) 156. While the distal end 59 of the pointer engages the reference feature 156, the navigation system may be configured to compute a transform using the pose of the pointer 57 within the known coordinate system to determine an actual location of each of the reference feature 156 within the known coordinate system.

Alternatively, with the second configuration of the surgical saw blade cartridge 210, where the reference feature 256 is defined on the surface of one of the first or second plates 212, 214, the step of determining the actual position of the surgical saw blade cartridge 210 within the known coordinate system may comprise manipulating the pointer 57 to contact each of the reference features 256 with the distal end 59 of the pointer 57. The navigation system 32 may track the pose of the pointer 57 via the pointer tracker PT as the pointer 57 is manipulated to contact each of the reference feature(s) 256. While the distal end 59 of the pointer 57 engages the reference feature 256, the navigation system 32 may be configured to compute a transform using the pose of the pointer 57 within the known coordinate system to determine an actual location of each of the reference feature(s) 256 within the known coordinate system.

In yet another configuration, the step of determining the actual position of the surgical saw blade cartridge 110 within the known coordinate system may comprise manipulating the pointer 57 to trace a reference feature 356 defined on the guide bar 111 with the distal end 59 of the pointer 57. The navigation system 32 may track the pose of the pointer 57 via the pointer tracker PT as the pointer 57 is manipulated to trace along a path defining the reference feature(s) 356. For example, the reference feature may comprise a path at least partially outlining a portion of the perimeter of the mount 18 on a surface of the guide bar 111. See Figures 6C and 6D. While the distal end 59 of the pointer 57 traces the path defining the reference feature 356, the navigation system 32 may be configured to compute a transform using the pose of the pointer 57 within the known coordinate system to determine an actual orientation of the plane of the surgical saw blade cartridge 110, or more specifically, the orientation of the plane defined by one or more of the first, second, and third plate, 112, 114, 116 in the known coordinate system, such as in the coordinate system of the tracker 52.

To determine the actual orientation of the plane of the cartridge, the navigation system may determine the actual position of the reference feature 156, 256, 356 defined and/or disposed on the guide bar 111, 211, 311, 411. The navigation system 32 may be further be configured to compare the actual position of each of the reference feature(s) 156, 256, 356 found using the pointer 57 (i.e., relative to the localizer) to the expected position of each of the reference feature(s) 156, 256, 356, 456 which is based on the stored information of the positions of those reference features relative to the tracker 52 that was determined during manufacturing. The comparison may include calculating a deviation between the actual position of each of the reference feature(s) 156, 256, 356 found using the pointer 57 to the expected position of each of the reference feature(s) 156, 256, 356, 456 based on the stored information. For example, a least squares model may be utilized to determine the deviation between the actual position of each of the reference feature(s) 156, 256, 356, 456 found using the pointer 57 to the expected position of each of the reference feature(s) 156, 256, 356, 456.

Using the calculated deviation between the actual position of each of the reference feature(s) 156, 256, 356, 456 found using the pointer 57 to the expected position of each of the reference feature(s) 156, 256, 356, 456, the navigation system 32 may further be comprised to adapt the position of each of the reference feature(s) 156, 256, 356, 456 within the known coordinate system and assign a hybrid location to each of the reference feature(s) 156, 256, 356, 456 based on the calculated deviation. The hybrid position will define the position of each of the reference feature(s) 156, 256, 356, 456 at location within the known coordinate system (such as relative the tracker 52 on the surgical instrument 14) that is at an intermediate position between the actual position of each of the reference feature(s) 156, 256, 356, 456 found using the pointer 57 and the expected position of each of the reference feature(s) 156, 256, 356.

The method may further comprise utilizing the hybrid position assigned to each of the reference feature(s) 156, 256, 356, 456 to define a plane of the guide bar 111, 211, 311, 411 and/or the surgical saw blade cartridge 110, 210, 310, 410 within the known coordinate system relative to the tracker 52 on the surgical instrument 14. The navigation system 32 may further be configured to navigate the surgical instrument 14, and by extension the surgical saw blade cartridge 110, 210, 310, 410 during a surgical procedure. This may be accomplished by tracking the position and/or orientation of the tracker 52 on the surgical instrument 14. Once the surgical saw blade cartridge 110, 210, 310, 410 is registered within the known coordinate system relative to the tracker 52 on the surgical instrument 14, the navigation system 32 will know the pose of the surgical saw blade cartridge 110, 210, 310, 410 based on the position of the tracker 52 on the surgical instrument 14 within the known coordinate system.

This approach to registration that defines the plane of the cartridge using the hybrid locations provides for a fast and effective method of registration for saw blade systems that do not control the positions of the cartridges in all six degrees of freedom, such as those robotic systems that provide control in only three degrees of freedom, as described in PCT/US2021/049440, which is hereby incorporated by reference in its entirety. For robotic systems that are handheld, the position of the saw blade relative to bone may not be controlled by actuators in all degrees of freedom, such as with respect the depth of the saw blade relative to the tool platform. For such a handheld robotic system, the distal end of the saw cartridge, I.e., the blade, need not be determinable with absolute precision.

In yet another configuration, a method of registering a saw blade 110, 210, 310, 410 within a known coordinate system of a navigation system, the method comprising: mounting a surgical saw blade 110, 210, 310, 410 to a surgical manipulator, the surgical manipulator including a first tracking device; contacting each of at least two reference features 156, 256, 356, 456 on a first surface or plate 112, 212, 312, 412 of the surgical saw blade 110, 210, 310, 410 with a distal end of a surgical instrument, the surgical instrument including a second tracking device; contacting each of at least two reference features 156, 256, 356, 456 on a second surface or plate 114, 214, 314, 414 of the surgical saw blade 110, 210, 310, 410 with the distal end of the surgical instrument; contacting at least one reference feature 60 on the surgical manipulator; tracking a position of the first tracking device and the second tracking device with a localizer of the navigation system as the distal end of the surgical instrument contacts each of the reference features 156, 256, 356, 456, 60 on both the surgical saw blade and surgical manipulator to define an actual position of each of the reference features 156, 256, 356, 456, 60 of the surgical saw blade relative to the reference features of the surgical manipulator; and defining a hybrid position for each of the at least one reference features 60 of the surgical manipulator by performing a mathematical fit between the actual position of each of the reference features 156, 256, 356, 456 of the surgical saw blade 110, 210, 310, 410 relative to the at least one reference features of the surgical manipulator 60.

The method of method of registering a saw blade 110, 210, 310, 410 within a known coordinate system may also comprise defining the hybrid position for the at least one reference feature of the surgical manipulator within the known coordinate system based on the mathematical fit is such that the hybrid position for each of the reference features is different from the actual position in only one degree of freedom.

The method of registering a saw blade 110, 210, 310, 410 within a known coordinate system may also comprise defining the hybrid position for the at least one reference feature of the surgical manipulator within the known coordinate system based on the mathematical fit cause the actual position of each of the at least one reference features of the surgical manipulator to be moved in only one degree of freedom.

The method of registering a saw blade 110, 210, 310, 410 within a known coordinate system may also comprise wherein the one degree of freedom is along the longitudinal axis of the surgical saw blade 110, 210, 310, 410.

In yet another implementation, a method of registering a surgical saw blade 110, 210, 310, 410 within a known coordinate system of a navigation system, the method comprising: mounting the surgical saw blade to a surgical manipulator, the surgical manipulator including a first tracking device; contacting each of at least two reference features on a first surface of the surgical saw blade with a distal end of a surgical instrument, the surgical instrument including a second tracking device; contacting each of at least two reference features on a second surface of the surgical saw blade with the distal end of the surgical instrument; tracking a position of the first tracking device and the second tracking device with a localizer of the navigation system as the distal end of the surgical instrument contacts each of the reference features on both the first and the second surfaces of the surgical saw blade to define an actual position of each of the reference features of the first and the second surfaces relative to the first tracking device of the surgical manipulator; defining a position and/or orientation of the surgical saw blade within the known coordinate system in one or more degrees of freedom based on a mathematical fit of the position of the at least two reference features of each of the first surface and the second surface.

The method of method of registering a saw blade 110, 210, 310, 410 within a known coordinate system may also comprise contacting a manipulator reference feature defined on the surgical manipulator with the distal end of the surgical instrument, and defining a hybrid position of the manipulator reference feature within known coordinate system based on a comparison of the actual position of each of the reference features of the first and the second surfaces relative to the manipulator reference feature where the tool center point is further defined based on a mathematical fit of the position of the at least two reference features of each of the first surface and the second surface and the hybrid position of the manipulator reference feature. It is contemplated that the tool center point may refer to an origin of a reference array specific to the surgical saw blade 110, 210, 310, 410, or may refer to an origin of a reference array of the system including at least the surgical saw blade 110, 210, 310, 410 and the surgical manipulator 14.

### Verification Process:

The combination of three of more openings 130A, 130B, 130C and the three or more reference features 156A, 156B, 156C may further be configured to verify the location and/or pose of the surgical saw blade 110, 210 relative to the tracker 52 of the surgical instrument 14 within the know coordinate system defined by the navigation system 32. It should be appreciated that the dimension of the openings 130A, 130B, 130C and corresponding reference features 156A, 156B, 156C may have a predetermined dimensional relationship with respect to the one or more mounting features. This enables the aforementioned registration and/or verification within the known coordinate system of the surgical navigation system 32 to be completed by touching the distal end 59 of the pointer 57 to one or more of the reference features 156A, 156B, 156C disposed on the guide bar 111. For example, the predetermined dimensional relationship may be stored on a memory unit of the surgical navigation system 32 based on the geometrical properties of a combination of the surgical saw blade cartridge 110, the mount 18, and the surgical instrument 14. Based on these known geometric properties, the navigation system may be configured to determine an expected location of each of the reference features 156A, 156B, 156C of the guide bar relative to the tracker 52 of the surgical instrument 14.

To complete the verification process, the pointer 57 may be manipulated to contact one or more of the reference features 156A, 156B, 156C while the navigation system 32 tracks the pose of the pointer 57 via the pointer tracker PT. The navigation system 32 may be configured to define the actual location of each of the reference features 156A, 156B, 156C within the known coordinate system based on the pose of the pointer 57 when in contact with each of the reference features 156A, 156B, 156C. The expected location of each of the reference features 156A, 156B, 156C may be compared against the actual location of the reference features 156A, 156B, 156C. The navigation system 32 may be configured to define an acceptable amount of deviation between the expected location of each of the reference features 156A, 156B, 156C and the actual location of the reference features 156A, 156B, 156C. If the deviation between the expected location of each of the reference features 156A, 156B, 156C and the actual location of the reference features 156A, 156B, 156C is with the accepted amount of deviation, the navigation system 32 may be configured to allow operation and/or navigation of the surgical instrument 14 during the surgical procedure. By contrast, ff the deviation between the expected location of each of the reference features 156A, 156B, 156C and the actual location of the reference features 156A, 156B, 156C is greater than the accepted amount of deviation, the navigation system 32 may be configured to provide a warning to the user and/or prevent operation and/or navigation of the surgical instrument 14.

A similar verification process may be completed for one of the alternate configurations of the surgical saw blade cartridge 110, 210, 310, 410 including one of the alternative types of the reference features 156, 256, 356, 456 such as the laser markings and/or tracing of a pattern or outline on the guide bar 111, 211, 311, 411.

The combination of openings 130, 230, 330, 430 and/or the reference features 156, 256, 356, 456 defined by the guide bar 111, 211, 311, 411 may also be configured to verify the surgical saw blade cartridge 110, 210, 310, 410 is properly loaded. In operation, the method may comprise manipulating the pointer 57 to contact one or more of the reference features 156, 256, 356, 456 while the navigation system 32 tracks the pose of the pointer 57 via the pointer tracker PT. The navigation system 32 may be configured to define the actual position of each of the reference features 156, 256, 356, 456 within the known coordinate system based on the pose of the pointer 57 when in contact with each of the reference features 156, 256, 356, 456. The pointer 57 may also be manipulated to contact one or more of the manipulator reference features 60 while the navigation system 32 tracks the pose of the pointer 57 via the pointer tracker PT. The navigation system 32 may be configured to define the actual position of manipulator reference features 60. The method may further comprises applying a mathematical fit to the actual position of the reference features 156, 256, 356, 456 compared to the actual position of manipulator reference features 60 whether the blade cartridge 110, 210, 310, 410 is properly mounted to the surgical manipulator based on comparison of the actual position of the manipulator reference feature to the actual position of each of the reference features of the first and the second surfaces based on defined deviation being within an accepted threshold/range.

The combination of openings 130, 230, 330, 430 and/or the reference features 156, 256, 356, 456 defined by the guide bar 111, 211, 311, 411 may also be configured to verify that the process of registering the surgical saw blade cartridge 110, 210, 310, 410 with the navigation system was successfully completed. The method may comprise mounting a surgical saw blade cartridge to a surgical manipulator, the surgical saw blade cartridge including a guide bar having a first plate disposed adjacent a second plate and a blade at least partially disposed between first and second plates and extending from a distal end of the guide bar, and the surgical manipulator including a first tracking device. The method may further comprise contacting each of at least two reference features on the first plate of the guide bar with a distal end of a surgical instrument, the surgical instrument including a second tracking device and contacting each of at least two reference features on the second plate of the guide bar with the distal end of the surgical instrument, each of the reference features of the first plate being concentric with one of the reference features of the second plate. While contacting the reference features, tracking the position of the first tracking device and the second tracking device with a localizer of the navigation system as the distal end of the surgical instrument contacts each of the reference features on both the first and the second plates of the surgical saw blade cartridge to define an actual position of each of the reference features of the first and the second plates relative to the first tracking device of the surgical manipulator. The method may then comprise determining a thickness of the cartridge based on the actual position of the reference features on the first plate and the second plate, and providing an indication to the user based on the determined thickness of the cartridge.

The various shapes of the elements may also vary from what has been described.

Accordingly, it is an object of the appended claims to cover all such variations and modifications that cover the true spirit and scope of the surgical saw blade cartridge 110, 210, 310, 410.
The following examples are also encompassed by the present disclosure and may fully or partly be incorporated into embodiments.
1. A saw blade cartridge for use with a surgical device, the saw blade cartridge comprising:
   a guide bar comprising:
   a first plate and a second plate, each of the first plate and the second plate having a distal portion and a proximal portion;
   an inner plate at least partially disposed between the first and the second plates, the inner plate defining a pivot surface;
   a blade at least partially disposed between the first and the second plates, the blade comprising:
      a blade body including a proximal end and a distal end; and
      a blade head disposed on the distal end of the blade body, the blade head formed with teeth, wherein the proximal end of the blade body abuts the pivot surface;
      wherein the proximal portion of each of the first and second plates each define a perimeter edge,
      wherein a portion of the inner plate extends beyond the perimeter edge defined by the proximal portion of at least one of the first and second plates.
2. The saw blade cartridge of example 1, wherein the perimeter edge defined by the proximal portion of at least one of the first and second plates is disposed over the portion of the inner plate that extends beyond the proximal portion of the at least one of the first and second plates to define a shelf to assist in mounting and alignment of the saw blade cartridge with the surgical device.
3. The saw blade cartridge of example 1, wherein the perimeter edge defined by the proximal portion of only one of the first and second plates is disposed over the portion of the inner plate that extends beyond the proximal portion of other of the at least one of the first and second plates to define a shelf to assist in mounting and alignment of the saw blade cartridge with the surgical device.
4. The saw blade cartridge of example 1, wherein the perimeter edge defined by the proximal portion of at least one of the first and second plates comprises a recess to expose the portion of the inner plate to define a shelf to assist in mounting and alignment of the saw blade cartridge with the surgical device.
5. The saw blade cartridge of example 1, wherein the perimeter edge defined by the proximal portion of only one of the first and second plates comprises a recess to expose the portion of the inner plate to define a shelf to assist in mounting and alignment of the saw blade cartridge with the surgical device.
6. The saw blade cartridge of anyone of examples 1 to 5, wherein the first plate defines a keyhole shape for mounting of the blade, and wherein the inner plate extends beyond the perimeter edge defined by the second plate.
7. The saw blade cartridge of anyone of examples 1 to 5, wherein the inner plate defines a recess in the side of the inner plate that faces away from the longitudinal axis of the cartridge, and wherein the first plate defines a recess that aligns with the recess of the inner plate.
8. The saw blade cartridge of any of examples 1 to 5, wherein the inner plate further comprises a tine extending from a proximal end of the guide bar, the tine defining the pivot surface.
9. The saw blade cartridge of example 8, wherein the tine is an inner tine, and the inner plate further including a first outer tine and a second outer tine, the inner tine being disposed between the first and second outer tines.
10. The saw blade cartridge of example 8, wherein the tine is an inner tine, and further comprising a first outer tine and a second outer tine, the first and second outer tine being positioned between the first and second plates and the inner tine being disposed between the first and second outer tines.
11. The saw blade cartridge of example 9 or 10, wherein the first and second outer tine extend closer to the distal end of the guide bar than the inner tine.
12. The saw blade cartridge of example 9 or 10, wherein the first and second outer tines each comprises a distal end;
   wherein the guide bar is configured such that the distal end of each of the first and second outer tines is proximal to a distal end of the first and the second plates; and
   wherein a side defined by the first and the second plates defines an aperture proximal to the distal end of the first and the second plates and distal to the distal end of each of the first and second outer tines, the aperture provides for debris exit.
13. The saw blade cartridge of example 10, wherein the first and second outer tines each comprises a distal tip;
   wherein the guide bar is configured such that the distal tip of each of the first and second outer tines is adjacent to a distal end of the first and the second plates.
14. The saw blade cartridge of any of examples 1 to 13, wherein the pivot surface is an arcuate surface.
15. The saw blade cartridge of example 14, wherein the proximal end of the blade body defining a recess that abuts the pivot surface, such that the blade body is configured to pivot about the pivot surface defined by the inner plate when the blade body is actuated.
16. The saw blade cartridge of example 14 or 15, wherein the teeth define a thickness of the blade head that is equal to or greater than a thickness of the guide bar to allow the insertion of the guide bar into a kerf cut by the blade head.
17. The saw blade cartridge of any one of examples 1 to 16, wherein at least one of the plates defines a reference feature configured to facilitate determination of a position and/or orientation of the surgical blade with a navigation system.
18. The saw blade cartridge of example 17, wherein the reference feature is selected from the group comprising an optical marking, a divot, or an aperture.
19. The saw blade cartridge of any one of the preceding examples, further comprising at least one drive link moveably disposed between the first and second plates,
   the at least one drive link having opposed proximal and distal ends, the proximal end adapted to be attached to a drive element of the surgical device, and the distal end being connected to the proximal end of the blade body so that reciprocation of the at least one drive link results in pivoting of the blade around the pivot surface.
20. The saw blade cartridge of example 19, wherein the at least one drive link comprises at least two drive links.
21. The saw blade cartridge of example 20, wherein the at least two drive links are movably coupled to the blade head.
22. A saw blade cartridge of a surgical device, the saw blade cartridge comprising:
   a guide bar comprising:
   a first plate and a second plate, each of the first plate and the second plate having a distal portion and a proximal portion;
   an inner plate disposed partially between the first and the second plates;
   wherein the guide bar defines a pivot surface;
   a blade at least partially disposed between the first and the second plates, the blade comprising:
      a blade body including a proximal end and a distal end; and
      a blade head disposed on the distal end of the blade body, the blade head formed with teeth, wherein the proximal end of the blade body abuts the pivot surface;
      wherein the proximal portion of each of the first and second plates each define a perimeter edge,
      wherein a portion of the inner plate extends beyond the perimeter edge defined by the proximal portion of at least one of the first and second plates.
23. A saw blade cartridge a surgical device and a navigation system, the saw blade cartridge comprising:
   a guide bar including a distal portion and a proximal portion, the guide bar comprising:
   a first plate;
   a second plate; and
   an inner plate positioned between the first and the second plates;
   a blade at least partially disposed between the first and the second plates of the guide bar, the blade comprising:
      a blade body including a proximal end and a distal end, the proximal end of the blade body abuts a pivot defined by the guide bar; and
      a blade head disposed on the distal end of the blade body, the blade head formed with teeth;
      at least three indicia disposed on an outer surface of one of the first plate or the second plate to define a plane of the guide bar within a coordinate system of the navigation system.
24. The saw blade cartridge of example 23, wherein each of the at least three indicia comprise an optical marking defined on one of the first plate or the second plate to facilitate determination of a position and/or orientation of the surgical blade with a navigation system.
25. The saw blade cartridge of example 23 or 24, wherein the blade head further defines a cut-out with an opening defined between two adjacent teeth.
26. The saw blade cartridge of example 25, wherein the cut-out defines a tear-drop shape opening in the blade head.
27. The saw blade cartridge of any one of examples 23 to 26, wherein the proximal portion of each of the first and second plates each define a perimeter edge,
   wherein a portion of the proximal portion of the inner plate extends beyond the perimeter edge defined by the proximal portion of at least one of the first and second plates.
28. A saw blade cartridge a surgical device and a navigation system, the saw blade cartridge comprising:
   a guide bar including a distal portion and a proximal portion, the guide bar comprising:
   a first plate;
   a second plate; and
   an inner plate disposed partially between the first and the second plates, the inner plate defining a pivot surface;
   a blade at least partially disposed between the first and the second plates, the blade comprising:
      a blade body including a proximal end and a distal end, the proximal end of the blade body abuts the pivot surface; and
      a blade head disposed on the distal end of the blade body, the blade head formed with teeth;
      at least three openings defined in one of the first plate or the second plate, the at least three of the openings to provide access to an outer surface of the inner plate at the location of each of the at least three openings.
29. The saw blade cartridge of example 28, wherein the proximal portion of each of the first and second plates each define a perimeter edge,
   wherein a portion of the proximal portion of the inner plate extends beyond the perimeter edge defined by the proximal portion of at least one of the first and second plates.
30. A saw blade cartridge for a surgical device, the saw blade cartridge comprising:
   a guide bar including a distal end and a proximal end, the guide bar comprising:
   a first plate;
   a second plate; and
   an inner plate disposed partially between the first and the second plates, the inner plate defining a pivot surface;
   a recess defined in the proximal end of at least one of the first plate, the second plate, or the inner plate;
   a blade at least partially disposed between the first and the second plates, the blade comprising:
      a blade body including a proximal end and a distal end, the proximal end of the blade body abuts the pivot surface; and
      a blade head disposed on the distal end of the blade body, the blade head formed with teeth.
31. The saw blade cartridge of example 30, wherein the blade head further defines a cut-out with an opening defined between two adjacent teeth.
32. The saw blade cartridge of example 30 or 31, wherein the cut-out defines a tear-drop shape opening in the blade head.
33. The saw blade cartridge of any one of examples 30 to 32, wherein a longitudinal axis extends between the distal end and a proximal end of the guide bar, the longitudinal axis bisecting the guide bar into a first lateral portion and a second lateral portion; and
   wherein the recess is defined in the proximal end of at least one of the first plate, the second plate, or the inner plate and is positioned on one of the first lateral portion or the second lateral portion.
34. The saw blade cartridge of any one of examples 30 to 33, wherein each of the first plate, the second plate, or the inner plate comprises an opposed outer edge; and
   wherein each of the opposed outer edges of each of the first plate, the second plate, and the inner plate mirror one another.
35. The saw blade cartridge of any one of examples 30 to 34, wherein at least one of the plates defines a reference feature configured to facilitate determination of a position and/or orientation of the guide bar within a coordinate system of a navigation system.
36. The saw blade cartridge of example 35, wherein the reference feature is selected from the group comprising an optical marking, a divot, or an aperture.
37. A surgical saw system, the system comprising:
   a surgical device including a mount, the mount defining a mount surface, and the mount comprising:
   a first wall and a second wall extending above the mounting surface, the first and second wall positioned on opposing sides of a longitudinal axis of the mount;
   the first and second wall are tapered relative to the longitudinal axis of the mount;
   at least one tab disposed on each of the first and second walls, each of the at least one tabs positioned above and extending over the mounting surface;
   a saw blade cartridge removably couplable to the mount of the surgical device, the saw blade cartridge comprising:
      a guide bar including a distal portion and a proximal portion, the guide bar comprising:
      a first plate;
      a second plate; and
      an inner plate disposed partially between the first and the second plates,
      wherein the proximal portion of each of the first and second plates each define a perimeter edge,
      wherein a portion of the proximal portion of the inner plate extends beyond the perimeter edge defined by the proximal portion of at least one of the first and second plates;
      wherein each of the first and second walls engage the perimeter edge of at least one of the first plate or the second plate to facilitate alignment of the saw blade cartridge along the longitudinal axis and each of the at least one tabs engages a surface of the portion of the proximal portion of the inner plate that extends beyond the perimeter edge defined by the proximal portion of at least one of the first and second plates.
38. The system of example 37, wherein the mount further comprises a mount indicia; and
   the guide bar comprises a bar indicia, the combination of the mount indicia and bar indicia configured to define a plane of the guide bar within a coordinate system of a navigation system.
39. The system of example 37, wherein at least one of the plates defines a reference feature configured to facilitate determination of a position and/or orientation of the saw blade cartridge with a navigation system.
40. The system of example 39, wherein the reference feature is selected from the group comprising an optical marking, a divot, or an aperture.
41. The system of example 37, wherein the surgical device further comprises a tracking device for use with a navigation system to track the pose the surgical instrument.
42. The system of example 41, wherein the surgical device further comprises one or more markers configured to register the surgical device within the known coordinate system defined by the navigation system relative to the tracking device of the surgical device.
43. A method of confirming a position and/or orientation of a surgical saw blade with a navigation system, the surgical saw blade including a guide bar comprising an inner plate partially disposed between a first plate and a second plate, either of the first plate or the second plate including a plurality of openings to provides access to a surface of the inner plate, the method comprising:
   positioning an instrument including a first tracking device such that distal end of the instrument is disposed within a first opening of the plurality of openings and abuts the surface of the inner plate;
   repositioning the instrument such that distal end of the instrument is disposed within a second opening of the plurality of openings and abuts the surface of the inner plate;
   determining an actual position and orientation of the surgical saw blade based on at least the position of the first and second openings as identified using the instrument;
   determining an expected position and orientation of the surgical saw blade based on predetermined geometrical data corresponding to the relationship between a mounting feature of a surgical manipulator and a position of the plurality of openings in either the first plate or the second plate of the surgical saw blade; and
   comparing the expected position and orientation of the plurality of openings with the actual position and orientation of the plurality of openings.
44. The method of example 43, further comprising the step of removably securing the surgical saw blade to the mounting feature of the surgical manipulator.
45. The method of example 43 or 44, further comprising the step of repositioning the instrument such that distal end of the instrument contacts a reference feature disposed on at least one of the mounting feature or the surgical manipulator to facilitate determination of a position and orientation of the surgical saw blade relative to the mounting feature or the surgical manipulator.
46. A method of confirming a position and/or orientation of a surgical saw blade within a known coordinate system defined by a navigation system, the surgical saw blade coupled to a surgical manipulator by a mounting feature and including a guide bar having a plurality of markings on an outer surface of the guide bar, the method comprising:
   manipulating an instrument including a first tracking device such that a distal end of the instrument contacts each of the plurality of markings on the outer surface of the guide bar;
   repositioning the instrument such that distal end of the instrument contacts a marking of the plurality of markings on the outer surface of the guide bar;
   determining an actual position and orientation of each of the markings within the known coordinate system based on at least the pose of the instrument when contacting each of the markings;
   determining an expected position and orientation of the markings within the known coordinate system based on predetermined geometrical data;
   comparing the expected position and orientation of each of the plurality of markings with the actual position and orientation of each of the plurality of markings;
   assigning a modified position based on the deviation between the expected position and orientation of each of the plurality of markings based on the predetermined geometrical data and the actual position; and
   defining a plane of the surgical saw blade within the known coordinate system based on the modified position.
47. The method of example 46, wherein each of the plurality of the markings are disposed on the outer surface of the guide bar such the plurality of markings outline a portion of an outer perimeter of the mounting feature of the surgical manipulator on the outer surface of the guide bar; and
   wherein the step of repositioning the distal end of the instrument from a first marking to a second marking further comprises maneuvering the distal end of the instrument along the outer surface of the guide bar from the first marking to the second marking tracing the portion of the outer perimeter of the mounting feature onto the outer surface of the guide bar.
48. The method of example 47, wherein the plurality of markings are connected by a path defined by an optical marking on the outer surface of the guide bar; and
   wherein the step of dragging the distal end of the instrument along the outer surface of the guide bar from the first marking to the second marking further comprises dragging the distal end along the outer surface of the guide bar along a path defined.
49. The method of any one of examples 46 to 48, further comprising the step of removably securing the surgical saw blade to the mounting feature of the surgical manipulator.
50. The method of any one of examples 46 to 49, further comprising the step of repositioning the instrument such that distal end of the instrument contacts a reference feature disposed on at least one of the mounting feature or the surgical manipulator to facilitate determination of a position and orientation of the surgical saw blade relative to the mounting feature or the surgical manipulator.
51. The method of any one of examples 46 to 50, wherein the step of determining the expected position and orientation of the first and second markings within the known coordinate system includes utilizing predetermined geometrical data the corresponds to the relationship between the mounting feature of the surgical manipulator and the guide bar.
52. A method of operating a saw blade with a navigation system, the surgical saw blade including a guide bar comprising three plates and at least three indicia disposed on the guide bar, the method comprising:
   mounting a surgical saw blade to a surgical manipulator, the surgical manipulator including a first tracking device;
   contacting each of the at least three indicia on the surgical saw blade with a distal end of an instrument, the instrument including a second tracking device;
   tracking the position of the first tracking device and the second tracking device with a localizer of the navigation system as the distal end of the instrument contacts each of the at least three indicia on the surgical saw blade to define an actual position of each of the at least three indicia relative to a known coordinate system;
   performing a mathematical fit of the actual position of each of the at least three indicia and an expected position for each of the at least three indicia relative to the known coordinate system; and
   determining a hybrid position within a known coordinate system based on the product of the mathematical fit for each of the at least three indicia; and
   defining a plane of the surgical saw blade based on the hybrid position for each of the at least three indicia within the known coordinate system.
53. The method of example 52, further comprising contacting a reference point on the surgical manipulator with a distal end of an instrument to define an actual position of the reference point relative to the first tracking device of the surgical manipulator;
   wherein the step of performing the least squares fit further comprises including the actual position of the reference point in combination with the actual position of each of the at least three indicia compared to an expected position for each of the at least three indicia and the reference point relative to the first tracking device of the surgical manipulator; and
   modifying the position of the at least three indicia within a known coordinate system based on the product of the least squares fit.
54. The method of example 52, wherein the at least three indicia comprise at least three apertures, each of the apertures defined in an outer plate of the guide bar to provide access to a surface an inner plate; and
   wherein the step of contacting each of the at least three indicia on the surgical saw blade comprises placing the distal end of the instrument within each of the three apertures to contact the surface of the inner plate at the position of the each of the apertures.
55. The method of example 54, wherein the distal end of the instrument comprises a ball shaped tip, the ball shaped tip sized to fit within each of the apertures defined in an outer plate of the guide bar.
56. The method of example 54, wherein the distal end of the instrument comprises a tip having a first dimension, the first dimension less than a second dimension of each of the apertures defined in an outer plate of the guide bar.
57. The method of example 52, further comprising comparing the product of the least squares fit against a target least squares fit;
   wherein if the deviation between the product of the least squares fit from the target least squares fit exceeds an acceptable margin as defined by the navigation system, the surgical manipulator will be rendered inoperable.
58. A method of registering a saw blade within a known coordinate system of a navigation system, the surgical saw blade including a guide bar comprising three plates and at least three apertures defined in an outer plate of the guide bar to provide access to an inner plate, the method comprising:
   mounting a surgical saw blade to a surgical manipulator, the surgical manipulator including a first tracking device;
   manipulating an instrument including a second tracking device such that a distal end of the instrument pass through each of the at least three apertures and contacts the inner plate exposed by each of the apertures;
   tracking the position of the first tracking device and the second tracking device with a localizer of the navigation system as the distal end of the surgical instrument contacts the inner plate through each of the at least three aperture on the surgical saw blade to define an actual position of each of the at least three apertures relative to the first tracking device of the surgical manipulator;
   defining an actual position of a portion of the inner plate exposed by each of the at least three apertures within the known coordinate system based on the tracked position of the instrument when contacting the inner plate through each of the at least three apertures;
   comparing an expected position of the portion of the inner plate exposed by each of the at least three apertures within the known coordinate system to the actual position of the portion of the inner plate exposed by each of the at least three apertures within the known coordinate system;
   modifying the position of the portion of the inner plate exposed by each of the at least three apertures within the known coordinate system based on the deviation between the actual position and the expected position of the portion of the inner plate exposed by each of the at least three apertures within the known coordinate system; and
   defining a plane of the surgical saw blade based on the modifying the position of the portion of the inner plate exposed by each of the at least three apertures within the known coordinate system.
59. A method of registering a saw blade within a known coordinate system of a navigation system, the saw blade including a guide bar comprising three plates and at least three reference features disposed on the guide bar, the method comprising:
   mounting a surgical saw blade to a surgical manipulator, the surgical manipulator including a first tracking device;
   contacting each of the at least three reference features on the surgical saw blade with a distal end of a surgical instrument, the surgical instrument including a second tracking device;
   tracking the position of the first tracking device and the second tracking device with a localizer of the navigation system as the distal end of the surgical instrument contacts each of the at least three reference features on the surgical saw blade to define an actual position of each of the at least three reference features relative to the first tracking device of the surgical manipulator;
   performing a mathematical fit based on the actual position of each of the at least three reference features and to an expected position for each of the at least three reference features relative to the known coordinate system; and
   determining a hybrid position of the at least three reference features within the known coordinate system based on the mathematical fit; and
   defining a plane of the surgical saw blade based on the hybrid position of the at least three reference features within the known coordinate system.
60. The method of example 59, wherein the at least three reference features are defined on an outer surface of the guide bar; and
   wherein the step of contacting each of the at least three reference features on the surgical saw blade with a distal end of a surgical instrument comprises touching the distal end of the surgical instrument to each of the at least three reference features on the outer surface of the guide bar.
61. The method of example 59, wherein the at least three reference feature are defined on a surface of an inner plate of the guide bar and an outer plate of the guide bar comprises an aperture corresponding to the position of each of the at least three reference features on the inner plate, each of the apertures terminating at a location of one of each of the at least three reference features on the inner plate to provide access to the reference feature; and
   wherein the step of contacting each of the at least three reference features on the surgical saw blade with a distal end of a surgical instrument comprises inserting the distal end of the surgical instrument through each of the apertures of the outer plate to contact each of the at least three reference features of the inner plate of the guide bar.
62. A method of controlling a hand-held medical robotic system for use with a surgical saw blade, the surgical saw blade including a guide bar comprising an inner plate partially disposed between a first plate and a second plate, either of the first plate or the second plate including a plurality of openings to provides access to a surface of the inner plate, the method comprising:
   registering the surgical saw blade to a navigation system, the step of registering the surgical saw blade comprising manipulating an instrument including a first tracking device to insert a distal end of the instrument within each of the plurality of openings to contact the surface of the inner plate at each location of the plurality of openings to define a plane of the surgical saw blade within a first coordinate system relative to a second tracking device that is coupled to the hand-held medical robotic system; and
   navigating the surgical saw blade based on the position and orientation of the second tracking device.
63. A method of controlling a hand-held medical robotic system for use with a surgical saw blade, the surgical saw blade including two reference features disposed on an outer surface of a guide bar of the surgical saw blade and an optical marking on the surface of the guide bar extending between the two references features, the method comprising:
   registering the surgical saw blade to a navigation system, the step of registering the surgical saw blade comprising manipulating an instrument including a first tracking device to use a distal end of the instrument to contact the surface of the guide bar to trace the optical marking extending between the two reference features to define a plane of the surgical saw blade within a first coordinate system relative to a second tracking device that is coupled to the hand-held medical robotic system; and
   navigating the surgical saw blade based on the position and orientation of the second tracking device.
64. A surgical saw system, the system comprising:
   a surgical device including a mount, the mount defining a mount surface, and the mount comprising:
   a first wall and a second wall extending above the mounting surface, the first and second wall positioned on opposing sides of a longitudinal axis of the mount;
   the first and second wall, each having a proximal portion and a distal portion, the proximal portion oriented at a first interior taper angle relative to the longitudinal axis of the mount and the distal portion orient at a second interior taper angle relative to the longitudinal axis of the mount;
   wherein the second interior taper angle is greater than the first interior taper angle relative to the longitudinal axis of the mount rather than the first taper;
   at least one tab disposed on each of the first and second walls, each of the at least one tabs positioned above and extending over the mounting surface;
   a saw blade cartridge removably couplable to the mount of the surgical device, the saw blade cartridge comprising:
      a guide bar including a distal portion and a proximal portion, the guide bar comprising:
      a first plate;
      a second plate;
      an inner plate disposed partially between the first and the second plates;
      wherein one of the first and second plates defines an outer edge of a proximal portion of the guide bar;
      wherein the proximal portion of each of the first and second walls engage the outer edge of the proximal portion of the guide bar to facilitate alignment of the saw blade cartridge along the longitudinal axis;
      wherein the distal portion of the wall is spaced away from the outer edge of the proximal portion of the guide bar; and
      an under surface of each of the at least one tabs is disposed over a top surface of the inner plate.
65. A surgical saw system, the system comprising:
   a surgical device including a mount, the mount defining a mount surface, and the mount comprising:
   a first wall and a second wall extending above the mounting surface, the first and second wall positioned on opposing sides of a longitudinal axis of the mount;
   the first and second wall are tapered relative to the longitudinal axis of the mount;
   at least one tab disposed on each of the first and second walls, each of the at least one tabs positioned above and extending over the mounting surface;
   a saw blade cartridge removably couplable to the mount of the surgical device, the saw blade cartridge comprising:
      a guide bar including a distal portion and a proximal portion, the guide bar comprising:
      a first plate and a second plate, at least one of the first and second plate defining a first outer edge of the proximal portion of the guide bar; and
      an inner plate disposed partially between the first and the second plates, the inner plate defining a pivot surface and a second outer edge of a portion of the proximal portion of the guide bar;
      wherein the second outer edge of the inner plate extends beyond the first outer edge defined by the first plate, the second outer edge configured to engage the first and second walls of the surgical device to assist in aligning the saw blade cartridge within the mount of the surgical device.
66. A saw blade cartridge for use with a surgical device, the saw blade cartridge comprising:
   a guide bar including a distal portion and a proximal portion, the guide bar comprising:
   a first plate;
   an second plate disposed adjacent to the first plate, the second plate defining a pivot surface;
   a blade extending distally from the distal portion of the guide bar, the blade comprising:
      a blade body including a proximal end and a distal end; and
      a blade head disposed on the distal end of the blade body, the blade head formed with teeth, wherein the proximal end of the blade body abuts the pivot surface;
      wherein the proximal portion defines a proximal end;
      wherein the first plate defines a first outer edge,
      wherein the second plate defines a second outer edge, the second outer edge extends beyond the first outer edge defined by the first plate and wherein the second outer edge tapers outwardly moving distally from the proximal end of the proximal portion toward the distal end of the proximal portion such that the second outer edge assists in aligning the saw blade cartridge with the surgical device.
67. A saw blade cartridge for use with a surgical device, the saw blade cartridge comprising:
   a guide bar including a distal portion and a proximal portion, the guide bar comprising:
   a first plate defining a first outer edge of the proximal portion;
   a second plate disposed adjacent to the first plate, the second plate defining a pivot surface and a second outer edge of the proximal portion of the guide bar,
   wherein the second outer edge extends beyond the first outer edge defined by the first plate, the second outer edge configured to engage the surgical device to assist in aligning the saw blade cartridge with the surgical device
   a blade extending distally of the distal portion of the guide bar, the blade comprising:
      a blade body including a proximal end configured to abut the pivot surface.
68. The saw blade cartridge of example 67, wherein the guide bar further comprises a third plate, the second plate at least partially disposed between the first and third plates.
69. The saw blade cartridge of example 67 or 68, wherein the first plate defines a reference feature configured to facilitate determination of a position and/or orientation of the surgical blade with a navigation system.
70. The saw blade cartridge of example 68, wherein one of the first or the third plates define a reference feature configured to facilitate determination of a position and/or orientation of the surgical blade with a navigation system.
71. The saw blade cartridge of example 68, wherein each of the first or the third plates define at least one reference feature, the combination of reference features configured to facilitate determination of a position and/or orientation of the surgical blade within a known coordinate system of a navigation system.
72. The saw blade cartridge of example 71, wherein the reference features are positioned on each of the first and third plates such that the references features of the first and third plates mirror each other.
73. The saw blade cartridge of example 71, wherein the reference features are positioned on each of the first and third plates such that the references features are concentrically aligned with each other.
74. The saw blade cartridge of example 69 or 70, wherein the reference feature is defined by an aperture having a first dimension, the first dimension configured to be smaller than a second dimension of a tip of surgical instrument associated with the navigation system for engaging the reference feature to facilitate determination of a position and/or orientation of the surgical blade to the navigation system.
75. The saw blade cartridge of example 67 or 68, wherein at least one of the second plate or the blade body further comprise a reference feature configured to facilitate determination of a position and/or orientation of the surgical blade with a navigation system; and
   the first plate is shaped to provide access to the reference feature of at least one of the second plate or the blade body.
76. The saw blade cartridge of example 69 or 75, wherein the reference feature is selected from the group comprising an optical marking, a divot, or an aperture.
77. A saw blade cartridge for use with a surgical device guided by a navigation system, the saw blade cartridge comprising:
   a guide bar including a distal portion and a proximal portion, the guide bar comprising:
   a first plate defining a first aperture;
   a second plate defining a second aperture; and
   an inner plate disposed partially between the first and the second plates, the inner plate defining a pivot surface;
   wherein the first and second apertures are configured to facilitate determination of a position and/or orientation of the guide bar with a navigation system;
   a blade extending distally from the distal portion of the guide bar, the blade comprising:
      a blade body including a proximal end configured to abut the pivot surface;
      wherein the proximal portion defines a proximal end;
      wherein the first plate and second plate are positioned such that the first aperture and second aperture are concentrically aligned.
78. The saw blade cartridge of example 77, wherein the first aperture and second aperture are defined on opposed sides of the inner plate.
79. The saw blade cartridge of example 77 or 78, wherein the first aperture and second aperture are defined on opposed sides of the guide bar.
80. The saw blade cartridge of any one of examples 77 to 79, wherein each of the apertures defines a reference feature that is identifiable by the navigation system to facilitate determination of a position and/or orientation of the surgical blade within a known coordinate system.
81. The saw blade cartridge of any one of examples 77 to 80, wherein each of the apertures further comprises a marking identifying the aperture as a reference feature, the marking including at least one of a laser marking, etch, color, engraving, symbol or bevel.
82. The saw blade cartridge of any one of examples 77 to 81, wherein the guide bar includes a longitudinal axis extending between the distal portion and the proximal portion of the guide bar bisecting the guide bar; and
   wherein each of the first and second plates define a plurality of apertures with at least one of the plurality of apertures defined by each of the first and second plates are located on opposed sides of the longitudinal axis of the guide bar.
83. The saw blade cartridge of any one of examples 77 to 81, wherein the guide bar includes a longitudinal axis extending between the distal portion and the proximal portion of the guide bar bisecting the guide bar; and
   Wherein each of the first and second apertures are positioned on the longitudinal axis of the respective plate of the guide bar.
84. A saw blade cartridge for use with a surgical device including a tracker identifiable by/associated with a navigation system, the saw blade cartridge comprising:
   a guide bar having a distal portion and a proximal portion, the guide bar comprising:
   a first plate defining a first reference feature in the distal portion and a second reference feature in the proximal portion;
   a second plate defining a third reference feature in the distal portion and a fourth reference feature in the proximal portion; and
   an inner plate at least partially disposed between the first and the second plates,
   wherein the combination of reference features are usable to register the saw blade cartridge within the known coordinate system defined by the navigation system relative to the tracker of the surgical device
   a blade at least partially disposed between the first and the second plates, the blade comprising:
      a blade body including a proximal end and a distal end; and
      a blade head disposed on the distal end of the blade body, the blade head formed with teeth
85. The saw blade cartridge of example 84, wherein the first and third reference features are located in the distal portion of the guide bar and the second and fourth reference features are located in the proximal portion of the guide bar.
86. The saw blade cartridge of example 84, wherein the guide bar includes a longitudinal axis extending between the distal portion and the proximal portion of the guide bar and bisecting the guide bar; and
   wherein the first and second reference features of the first plate are located on opposed sides of the longitudinal axis; and
   wherein the third and fourth reference features of the second plate are located on opposed sides of the longitudinal axis.
87. The saw blade cartridge of example 84, wherein the guide bar includes a longitudinal axis extending between the distal portion and the proximal portion of the guide bar and bisecting the guide bar; and
   wherein at least one of the first and second reference features of the first plate are disposed on the longitudinal axis; and
   wherein at least one of the third and fourth reference features of the second plate are disposed on the longitudinal axis.
88. A surgical saw assembly for use with a navigation system, the assembly comprising:
   a surgical instrument/device including an instrument reference feature;
   a tracker disposed on the surgical instrument, the tracker identifiable by and/or associated with the navigation system;
   a saw blade cartridge removably couplable to the surgical device, the saw blade cartridge comprising:
      a guide bar having a distal portion and a proximal portion, the guide bar comprising:
      a first plate defining a first reference feature in the distal portion and a second reference feature in the proximal portion;
      a second plate defining a third reference feature in the distal portion and a fourth reference feature in the proximal portion; and
      an inner plate at least partially disposed between the first and the second plates, the inner plate defining a pivot surface;
      wherein the combination of reference features are usable to facilitate registration of the saw blade cartridge within a known coordinate system defined by the navigation system relative to the tracker of the surgical device.
      a blade extending from the distal portion of the guide bar.
89. The surgical saw assembly of example 88, wherein the first plate comprises a second reference feature; and
   wherein the second plate comprises a fourth reference feature.
90. The surgical saw assembly of example 89, wherein the first and third reference features are located in the distal portion of the guide bar and the second and fourth reference features are located in the proximal portion of the guide bar.
91. The surgical saw assembly of example 89, wherein the guide bar includes a longitudinal axis extending between the distal portion and the proximal portion of the guide bar and bisecting the guide bar; and
   wherein the first and second reference features of the first plate are located on opposed sides of the longitudinal axis; and
   wherein the third and fourth reference features of the second plate are located on opposed sides of the longitudinal axis.
92. The surgical saw assembly of example 89, wherein the guide bar includes a longitudinal axis extending between the distal portion and the proximal portion of the guide bar and bisecting the guide bar; and
   wherein at least one of the first and second reference features of the first plate is disposed on the longitudinal axis; and
   wherein at least one of the third and fourth reference features of the second plate is disposed on the longitudinal axis.
93. A method of registering a saw blade within a known coordinate system of a navigation system, the surgical saw blade including a guide bar comprising three plates and at least three reference features are defined on the guide bar, the method comprising:
   mounting a surgical saw blade to a surgical manipulator, the surgical manipulator including a first tracking device;
   contacting each of the at least three reference features on the surgical saw blade with a distal end of a surgical instrument, the surgical instrument including a second tracking device;
   tracking the position of the first tracking device and the second tracking device with a localizer of the navigation system as the distal end of the surgical instrument contacts each of the at least three reference features on the surgical saw blade to define an actual position of each of the at least three reference features relative to the first tracking device of the surgical manipulator;
   defining a plane of the surgical saw blade based on the actual position of the at least three reference features within the known coordinate system; and
94. The method of example 93, further comprising the step of controlling the surgical manipulator based on the defined plane and a target cutting plane.
95. The method of example 93 or 94, wherein the defined plane is defined without a prior knowledge of the expected position of the blade relative to the surgical manipulator.
96. A method of registering a saw blade within a known coordinate system of a navigation system, the method comprising:
   mounting a surgical saw blade to a surgical manipulator, the surgical manipulator including a first tracking device;
   contacting each of at least two reference features on a first surface of the surgical saw blade with a distal end of a surgical instrument, the surgical instrument including a second tracking device;
   contacting each of at least two reference features on a second surface of the surgical saw blade with the distal end of the surgical instrument;
   tracking a position of the first tracking device and the second tracking device with a localizer of the navigation system as the distal end of the surgical instrument contacts each of the reference features on both the first and the second surfaces of the surgical saw blade to define an actual position of each of the reference features of the first and the second surfaces relative to the first tracking device of the surgical manipulator;
   comparing the actual position of each of the reference features and a position of a manipulator reference feature within the known coordinate system to verify whether the surgical saw blade is properly mounted to the surgical manipulator.
97. The method of example 96, wherein the step of contacting each of at least two reference features on the first surface comprises contacting a first reference feature located on a proximal portion of the first surface and contacting a second reference feature located on a distal portion of the first surface; and
   wherein the step of contacting each of at least two reference features on the second surface comprises contacting a third reference feature located on a proximal portion of the second surface and contacting a fourth reference feature located on a distal portion of the second surface.
98. The method of example 96, wherein the surgical saw blade comprises a distal portion and a proximal portion with a longitudinal that extends between the distal portion and the proximal portion and bisects the surgical saw blade;
   wherein the step of contacting each of at least two reference features on the first surface comprises contacting a first reference feature located on a first portion of the first surface and contacting a second reference feature located on a second portion of the first surface, such that the first and second portions are on opposed sides of the longitudinal axis; and
   wherein the step of contacting each of at least two reference features on the second surface comprises contacting a third reference feature located on a third portion of the second surface and contacting a fourth reference feature located on a fourth portion of the second surface, such that the third and fourth portions are on opposed sides of the longitudinal axis.
99. The method of any one of examples 96 to 98, further comprising the steps of:
   contacting a manipulator reference feature on the surgical manipulator with the distal end of the surgical instrument; and
   tracking the position of the first tracking device and the second tracking device with a localizer of the navigation system as the distal end of the surgical instrument contacts the manipulator reference feature to define an actual position of the manipulator reference feature within the known coordinate system relative to the first tracking device of the surgical manipulator.
100. The method of example 99, further comprising the step of comparing the actual position of the manipulator reference feature within the known coordinate system to the actual position of each of the reference features of the first and the second surfaces within the known coordinate system to define a deviation between the actual position of the manipulator reference feature and the actual position of each of the reference features of the first and the second surfaces; and
   determining whether the surgical saw blade is properly mounted to the surgical manipulator based on comparison of the actual position of the manipulator reference feature to the actual position of each of the reference features of the first and the second surfaces based on defined deviation being within an accepted threshold/range.
101. The method of any one of examples 96 to 100, further comprising defining a tool center point of the surgical saw blade based on the actual position for each of the reference features within the known coordinate system.
102. The method of example 101, further comprising the step of navigating the surgical saw blade via the navigation system based on the defined tool center point of the surgical saw blade.
103. The method of any one of examples 96 to 100, further comprising defining a plane of the surgical saw blade based on the actual position for each of the reference features within the known coordinate system.
104. The method of any one of examples 96 to 100, further comprising defining a hybrid position for the manipulator reference feature within the known coordinate system based on a mathematical fitting of the actual position of reference features of the surgical saw blade and the manipulator reference point; wherein the step of defining the hybrid position of the manipulator reference feature within the known coordinate system based on the mathematical fit is such that the hybrid position of the manipulator reference feature is different from the actual position of the manipulator reference feature in only one degree of freedom.
105. The method of example 104, wherein the one degree of freedom is along the longitudinal axis of the surgical saw blade.
106. A method of registering a saw blade within a known coordinate system of a navigation system, the method comprising:
   mounting a surgical saw blade cartridge to a surgical manipulator, the surgical saw blade cartridge including a guide bar having a first plate disposed adjacent a second plate and a blade at least partially disposed between first and second plates and extending from a distal end of the guide bar, and the surgical manipulator including a first tracking device;
   contacting each of at least two reference features on the first plate of the guide bar with a distal end of a surgical instrument, the surgical instrument including a second tracking device;
   contacting each of at least two reference features on the second plate of the guide bar with the distal end of the surgical instrument, each of the reference features of the first plate being concentric with one of the reference features of the second plate;
   tracking the position of the first tracking device and the second tracking device with a localizer of the navigation system as the distal end of the surgical instrument contacts each of the reference features on both the first and the second plates of the surgical saw blade cartridge to define an actual position of each of the reference features of the first and the second plates relative to the first tracking device of the surgical manipulator; and
   determining a thickness of the cartridge based on the actual position of the reference features on the first plate and the second plate; and
   providing an indication to the user based on the determined thickness of the cartridge.
107. The method of example 106, wherein the step of contacting each of at least two reference features on the first plate comprises contacting a first reference feature located on a proximal portion of the first plate and contacting a second reference feature located on a distal portion of the first plate; and
   wherein the step of contacting each of at least two reference features on the second plate comprises contacting a third reference feature located on a proximal portion of the second plate and contacting a fourth reference feature located on a distal portion of the second plate.
108. The method of example 107, wherein the step of determining a thickness of the surgical blade further comprises the step of defining a first blade thickness based on a comparison of the location of the first reference feature of the first plate and the third reference feature of the second plate.
109. The method of example 108, wherein the step of determining a thickness of the surgical blade further comprises defining a second blade thickness based on a comparison of the location of the second reference feature of the first plate to the fourth reference feature of the second plate.
110. The method of example 109, further comprising computing a deviation between the first blade thickness to the second blade thickness; and
   accepting registration of the surgical blade within the known coordinate system of a navigation system based on the deviation between the first blade thickness to the second blade thickness being within an accepted threshold/range.
111. The method of example 110, wherein the deviation the first blade thickness to the second blade thickness is indicative of deflecting the distal portion of the first and/or second plate when contacting either of the second or fourth reference features.
112. The method of any one of examples 106 to 111, wherein the step of providing the indication comprises at least one of a visual cue, an auditory cue, and/or a haptic cue to the user.
113. The method of any one of examples 106 to 112, wherein the step of providing the indication indicates to the user that the determined thickness is out of range or beyond a defined threshold.
114. The method of any one of examples 106 to 112, wherein the step of providing the indication indicates that user needs to replace the surgical saw blade and/or re-register the surgical saw blade to the navigation system.
115. The method of example 106, wherein the surgical saw blade comprises a distal portion and a proximal portion with a longitudinal axis that extends between the distal portion and the proximal portion and bisects the surgical saw blade;
   wherein the step of contacting each of at least two reference features on the first plate comprises contacting a first reference feature located on a first portion of the first plate and contacting a second reference feature located on a second portion of the first plate, such that the first and second portions are on opposed sides of the longitudinal axis; and
   wherein the step of contacting each of at least two reference features on the second plate comprises contacting a third reference feature located on a third portion of the second plate and contacting a fourth reference feature located on a fourth portion of the second plate, such that the third and fourth portions are on opposed sides of the longitudinal axis.
116. The method of any one of examples 106 to 115, further comprising the steps of:
   contacting a manipulator reference feature on the surgical manipulator with the distal end of the surgical instrument; and
   tracking the position of the first tracking device and the second tracking device with a localizer of the navigation system as the distal end of the surgical instrument contacts the manipulator reference feature to define an actual position of the manipulator reference feature within the known coordinate system relative to the first tracking device of the surgical manipulator.
117. The method of example 116, further comprising the step of comparing the actual position of the manipulator reference feature within the known coordinate system to the actual position of each of the reference features of the first and the second plates within the known coordinate system to determine whether the surgical saw blade is properly mounted to the surgical manipulator.
118. The method of example 116, further comprising the step of comparing the actual position of the manipulator reference feature within the known coordinate system and the actual position of each of the reference features of the first and the second plates within the known coordinate system to the expected position of the manipulator reference feature and the expected position of the of the reference features of the first and second plates to define a deviation; and
   determining whether the surgical saw blade is properly mounted to the surgical manipulator based on the deviation and a deviation threshold.
119. The method of any one of examples 106 to 118, further comprising the step of controlling a plurality of actuators of the surgical manipulator based on the defined plane and a target cutting plane.
120. A method of registering a saw blade within a known coordinate system of a navigation system, the method comprising:
   mounting a surgical saw blade to a surgical manipulator, the surgical manipulator including a first tracking device;
   contacting each of at least two reference features on a first surface of the surgical saw blade with a distal end of a surgical instrument, the surgical instrument including a second tracking device;
   contacting each of at least two reference features on a second surface of the surgical saw blade with the distal end of the surgical instrument;
   contacting at least one reference feature on the surgical manipulator;
   tracking a position of the first tracking device and the second tracking device with a localizer of the navigation system as the distal end of the surgical instrument contacts each of the reference features on both the surgical saw blade and surgical manipulator to define an actual position of each of the reference features of the surgical saw blade relative to the reference features of the surgical manipulator; and
   defining a hybrid position for each of the at least one reference features of the surgical manipulator by performing a mathematical fit between the actual position of each of the reference features of the surgical saw blade relative to the at least one reference features of the surgical manipulator.
121. The method of example 120, wherein the step of defining the hybrid position for the at least one reference feature of the surgical manipulator within the known coordinate system based on the mathematical fit is such that the hybrid position for each of the reference features is different from the actual position in only one degree of freedom.
122. The method of example 120, wherein the step of defining the hybrid position for the at least one reference feature of the surgical manipulator within the known coordinate system based on the mathematical fit cause the actual position of each of the at least one reference features of the surgical manipulator to be moved in only one degree of freedom.
123. The method of example 121 or 122, wherein the one degree of freedom is along the longitudinal axis of the surgical saw blade.
124. The method of any one of examples 120 to 123, further comprising:
   comparing the actual position of the at least one reference features of the manipulator to the actual position of each of the reference features of the surgical saw blade; and
   determining whether the surgical saw blade is properly mounted to the surgical manipulator based on a deviation the actual position of the at least one reference features of the manipulator to the actual position of each of the reference features of the surgical saw blade being within an accepted threshold/range.
125. The method of any one of examples 120 to 123, further comprising:
   rejecting the registration of the surgical saw blade within the known coordinate system of a navigation system based on a deviation the actual position of the at least one reference features of the manipulator to the actual position of each of the reference features of the surgical saw blade being within an accepted threshold/range.
126. The method of example 124 or 125, wherein the accepted threshold for deviation between the actual position of the at least one reference features of the manipulator to the actual position of each of the reference features of the surgical saw blade is plus or minus one millimeter (+/- 1 mm.).
127. The method of any one of examples 120 to 123, further comprising:
   comparing the hybrid position of the at least one reference features of the manipulator to the actual position of each of the reference features of the surgical saw blade; and
   determining whether the surgical saw blade is properly mounted to the surgical manipulator based on a deviation the hybrid position of the at least one reference features of the manipulator to the actual position of each of the reference features of the surgical saw blade being within an accepted threshold/range.
128. The method of any one of examples 120 to 123, further comprising:
   rejecting the registration of the surgical saw blade within the known coordinate system of a navigation system based on a deviation the hybrid position of the at least one reference features of the manipulator to the actual position of each of the reference features of the surgical saw blade being within an accepted threshold/range.
129. The method of example 127 or 128, wherein the accepted threshold for deviation between the hybrid position of the at least one reference features of the manipulator to the actual position of each of the reference features of the surgical saw blade is plus or minus one millimeter (+/- 1 mm.).
130. A method of registering a surgical saw blade within a known coordinate system of a navigation system, the method comprising:
   mounting the surgical saw blade to a surgical manipulator, the surgical manipulator including a first tracking device;
   contacting each of at least two reference features on a first surface of the surgical saw blade with a distal end of a surgical instrument, the surgical instrument including a second tracking device;
   contacting each of at least two reference features on a second surface of the surgical saw blade with the distal end of the surgical instrument;
   tracking a position of the first tracking device and the second tracking device with a localizer of the navigation system as the distal end of the surgical instrument contacts each of the reference features on both the first and the second surfaces of the surgical saw blade to define an actual position of each of the reference features of the first and the second surfaces relative to the first tracking device of the surgical manipulator;
   defining a position and/or orientation of the surgical saw blade within the known coordinate system in one or more degrees of freedom based on a mathematical fit of the position of the at least two reference features of each of the first surface and the second surface.
131. The method of example 130, further comprising the step of contacting a manipulator reference feature defined on the surgical manipulator with the distal end of the surgical instrument; and
   defining a hybrid position of the manipulator reference feature within known coordinate system based on a comparison of the actual position of each of the reference features of the first and the second surfaces relative to the manipulator reference feature; and
   wherein the tool center point is further defined based on a mathematical fit of the position of the at least two reference features of each of the first surface and the second surface and the hybrid position of the manipulator reference feature.
132. The method of example 130 or 131, wherein the step of defining the position and/or orientation of the surgical saw blade within the known coordinate system in one or more degrees of freedom comprises defining the position and/or orientation of the surgical saw blade within the known coordinate system in up to six (6) degrees of freedom.
133. A method of registering a surgical saw assembly within a known coordinate system of a navigation system, the method comprising:
   mounting a surgical saw blade to a surgical manipulator, the surgical manipulator including a first tracking device;
   contacting each of at least two reference features on a first surface of the surgical saw blade with a distal end of a surgical instrument, the surgical instrument including a second tracking device;
   contacting each of at least two reference features on a second surface of the surgical saw blade with the distal end of the surgical instrument;
   contacting a manipulator reference feature defined on the surgical manipulator with the distal end of the surgical instrument; and
   tracking a position of the first tracking device and the second tracking device with a localizer of the navigation system as the distal end of the surgical instrument contacts each of the reference features on both the first and the second surfaces of the surgical saw blade to define an actual position of each of the reference features of the first and the second surfaces and the manipulator reference feature relative to the first tracking device of the surgical manipulator;
   defining a hybrid position of the manipulator reference feature within known coordinate system based on a comparison of the actual position of each of the reference features of the first and the second surfaces relative to the manipulator reference feature; and
   defining a position and/or orientation of the surgical saw assembly within the known coordinate system in at least one degree of freedom based on a mathematical fit of the position of the at least two reference features of each of the first surface and the second surface and the hybrid position of the manipulator reference feature.
134. The method of example 133, wherein the step of defining the position and/or orientation of the surgical saw blade within the known coordinate system in one or more degrees of freedom comprises defining the position and/or orientation of the surgical saw blade within the known coordinate system in up to six (6) degrees of freedom.
135. A method of registering a saw assembly within a known coordinate system of a navigation system, the method comprising:
   mounting a surgical saw blade to a surgical manipulator, the surgical manipulator including a first tracking device;
   contacting each of at least two reference features on a first surface of the surgical saw blade with a distal end of a surgical instrument, the surgical instrument including a second tracking device;
   contacting each of at least two reference features on a second surface of the surgical saw blade with the distal end of the surgical instrument;
   contacting a manipulator reference feature defined on the surgical manipulator with the distal end of the surgical instrument; and
   tracking a position of the first tracking device and the second tracking device with a localizer of the navigation system as the distal end of the surgical instrument contacts each of the reference features on both the first and the second surfaces of the surgical saw blade to define an actual position of each of the reference features of the first and the second surfaces and the manipulator reference feature relative to the first tracking device of the surgical manipulator;
   applying a first mathematical fit to the actual position of the reference features of the first and the second surfaces and the manipulator reference feature relative to one another;
   defining a hybrid position of the manipulator reference feature based on the first mathematical fit; and
   applying a second mathematical fit to the actual position of the reference features of the first and the second surfaces and the hybrid position of the manipulator reference feature relative to one another;
   defining position and/or orientation in one or more degrees of freedom of the surgical saw assembly within the known coordinate system based on the second mathematical fit.
136. The method of example 135, wherein the step of defining the position and/or orientation in one or more degrees of freedom comprises defining the position and/or orientation in up to six (6) degrees of freedom
137. A method of verification of mounting of a surgical saw blade to a surgical manipulator, the method comprising:
   mounting a surgical saw blade to a surgical manipulator, the surgical manipulator including a first tracking device;
   contacting each of at least two reference features on a first surface of the surgical saw blade with a distal end of a surgical instrument, the surgical instrument including a second tracking device;
   contacting a manipulator reference feature defined on the surgical manipulator with the distal end of the surgical instrument; and
   applying a mathematical fit to the actual position of the reference features of the first surface and the manipulator reference feature relative to one another;
   comparing an output of the mathematical fit to a threshold, wherein the output of the mathematical fit relative to the threshold is indicative of whether the surgical saw blade is properly mounted to the surgical manipulator.
138. The method of example 137, further comprising the step of contacting each of at least two reference features on a second surface of the surgical saw blade with the distal end of the surgical instrument; and
   wherein the step of applying the mathematical fit comprises applying a mathematical fit to the actual position of the reference features of the second surface in addition to the reference features of the first surface and the manipulator reference feature relative to each another.
139. A method of verifying a registration of a surgical saw blade within a known coordinate system of a navigation system, the method comprising:
   mounting a surgical saw blade to a surgical manipulator, the surgical manipulator including a first tracking device;
   contacting a first reference feature located on a proximal portion of a first plate of the surgical saw blade and contacting a second reference feature located on a distal portion of the first plate with a distal end of a surgical instrument, the surgical instrument including a second tracking device; and
   contacting a third reference feature located on a proximal portion of a second plate of the surgical saw blade and contacting a fourth reference feature located on a distal portion of the second plate with the distal end of the surgical instrument
   defining a first blade thickness based on a comparison of the location of the first reference feature of the first plate and the third reference feature of the second plate;
   defining a second blade thickness based on a comparison of the location of the second reference feature of the first plate to the fourth reference feature of the second plate;
   computing a deviation between the first blade thickness to the second blade thickness; and
   wherein the deviation the first blade thickness to the second blade thickness is indicative of deflecting the distal portion of the first and/or second plate when contacting either of the second or fourth reference features.

## Claims

1. A saw blade cartridge (110, 210, 310) for use with a surgical device (14) guided by a navigation system, the saw blade cartridge (110, 210, 310) comprising:
a guide bar (111, 211, 311) including a distal portion and a proximal portion, the guide bar (111, 211, 311) comprising:
a first plate (112, 212, 312) defining a first aperture (130A, 330A, 330B, 330C);
a second plate (114, 214, 314) defining a second aperture (130B, 330D, 330E, 330F); and
an inner plate (116, 216, 316) disposed partially between the first and the second plates (112, 212, 312, 114, 214, 314), the inner plate defining a pivot surface (158, 258, 358);
wherein the first and second apertures (130A, 330A, 330B, 330C, 130B, 330D, 330E, 330F) are configured to facilitate determination of a position and/or orientation of the guide bar (111, 211, 311) with a navigation system (32);
a blade (170, 270, 370) extending distally from the distal portion of the guide bar (111, 211, 311), the blade comprising:
a blade body (172, 272, 372) including a proximal end configured to abut the pivot surface (158, 258, 358);
wherein the first plate and second plate (112, 212, 312, 114, 214, 314) are positioned such that the first aperture and second aperture (130A, 330A, 330B, 330C, 130B, 330D, 330E, 330F) are concentrically aligned.

2. The saw blade cartridge (110, 210, 310) of claim 1, wherein the first aperture and second aperture (130A, 330A, 330B, 330C, 130B, 330D, 330E, 330F) are defined on opposed sides of the guide bar (111, 211, 311).

3. The saw blade cartridge (110, 210, 310) of claim 1 or 2, wherein each of the first and second apertures (130A, 330A, 330B, 330C, 130B, 330D, 330E, 330F) define a reference feature (156, 256, 256A, 256B, 256C, 256D, 356) that is identifiable by the navigation system (32) to facilitate determination of a position and/or orientation of the surgical blade cartridge (110, 210, 310) within a known coordinate system.

4. The saw blade cartridge (110, 210, 310) of any one of claims 1 to 3, wherein each of the first and second apertures (130A, 330A, 330B, 330C, 130B, 330D, 330E, 330F) further comprises a marking identifying the aperture as a reference feature (156, 256, 256A, 256B, 256C, 256D, 356), the marking including at least one of a laser marking, etch, color, engraving, symbol or bevel.

5. The saw blade cartridge (110, 210, 310) of any one of claims 1 to 4, wherein the guide bar (111, 211, 311) includes a longitudinal axis extending between the distal portion and the proximal portion of the guide bar (111, 211, 311) that bisects the guide bar (111, 211, 311); and
wherein each of the first and second plates (112, 212, 312, 114, 214, 314) further define a plurality of apertures with at least one of the plurality of apertures (130A, 330A, 330B, 330C, 130B, 330D, 330E, 330F) defined by each of the first and second plates (112, 212, 312, 114, 214, 314) being located on opposed sides of the longitudinal axis of the guide bar (111, 211, 311).

6. The saw blade cartridge (110, 210, 310) of any one of claims 1 to 4, wherein the guide bar (111, 211, 311) includes a longitudinal axis extending between the distal portion and the proximal portion of the guide bar (111, 211, 311) that bisects the guide bar (111, 211, 311); and
wherein each of the first and second apertures (130A, 330A, 330B, 330C, 130B, 330D, 330E, 330F) are positioned on the longitudinal axis of the respective plate of the guide bar (111, 211, 311).

7. A method of registering a saw blade cartridge (110, 210, 310) within a known coordinate system of a navigation system, the method comprising:
mounting a surgical saw blade cartridge (110, 210, 310) to a surgical manipulator (14), the surgical manipulator (14) including a first tracking device (52);
contacting each of at least two reference features (130A, 330A, 330B, 330C,) on a first surface of the surgical saw blade cartridge (110, 210, 310) with a distal end (59) of a surgical instrument (57), the surgical instrument (57) including a second tracking device (PT);
contacting each of at least two reference features (130B, 330D, 330E, 330F) on a second surface of the surgical saw blade cartridge (110, 210, 310) with the distal end (59) of the surgical instrument (57);
tracking a position of the first tracking device (52) and the second tracking device (PT) with a localizer of the navigation system (32) as the distal end of the surgical instrument (57) contacts each of the reference features (130A, 330A, 330B, 330C, 130B, 330D, 330E, 330F) on both the first and the second surfaces (112, 212, 312, 114, 214, 314) of the surgical saw blade cartridge (110, 210, 310) to define an actual position of each of the reference features (130A, 330A, 330B, 330C, 130B, 330D, 330E, 330F) of the first and the second surfaces (112, 212, 312, 114, 214, 314) relative to the first tracking device (52) of the surgical manipulator (14);
comparing the actual position of each of the reference features (130A, 330A, 330B, 330C, 130B, 330D, 330E, 330F) and a position of a manipulator reference feature (60A, 60B, 60C, 60D) within the known coordinate system to verify whether the surgical saw blade cartridge (110, 210, 310) is properly mounted to the surgical manipulator (14).

8. The method of claim 7, wherein the step of contacting each of at least two reference (130A, 330A, 330B, 330C) features on the first surface (112, 212, 312) comprises contacting a first reference feature (130A, 330A, 330B, 330C) located on a proximal portion of the first surface (112, 212, 312) and contacting a second reference feature (130A, 330A, 330B, 330C) located on a distal portion of the first surface (112, 212, 312); and
wherein the step of contacting each of at least two reference features (130B, 330D, 330E, 330F) on the second surface (114, 214, 314) comprises contacting a third reference feature (130B, 330D, 330E, 330F) located on a proximal portion of the second surface (114, 214, 314) and contacting a fourth reference feature (130B, 330D, 330E, 330F) located on a distal portion of the second surface (114, 214, 314).

9. The method of claim 7, wherein the surgical saw blade cartridge (110, 210, 310) comprises a distal portion and a proximal portion with a longitudinal axis that extends between the distal portion and the proximal portion and bisects the surgical saw blade cartridge (110, 210, 310);
wherein the step of contacting each of at least two reference features (130A, 330A, 330B, 330C) on the first surface (112, 212, 312) comprises contacting a first reference feature (130A, 330A, 330B, 330C) located on a first portion of the first surface (112, 212, 312) and contacting a second reference feature (130A, 330A, 330B, 330C) located on a second portion of the first surface (112, 212, 312), such that the first and second portions are on opposed sides of a longitudinal axis of the surgical saw blade cartridge (110, 210, 310); and
wherein the step of contacting each of at least two reference features (130B, 330D, 330E, 330F) on the second surface comprises contacting a third reference feature (130B, 330D, 330E, 330F) located on a third portion of the second surface (114, 214, 314) and contacting a fourth reference feature (130B, 330D, 330E, 330F) located on a fourth portion of the second surface (114, 214, 314), such that the third and fourth portions are on opposed sides of the longitudinal axis.

10. The method of any one of claims 7 to 9, further comprising the steps of:
contacting a manipulator reference feature (60A, 60B, 60C, 60D) on the surgical manipulator (14) with the distal end of the surgical instrument (57); and
tracking the position of the first tracking device (52) and the second tracking device (PT) with a localizer of the navigation system (32) as the distal end of the surgical instrument (57) contacts the manipulator reference feature (60A, 60B, 60C, 60D) to define an actual position of the manipulator reference feature (60A, 60B, 60C, 60D) within the known coordinate system relative to the first tracking device (52) of the surgical manipulator (14).

11. The method of claim 10, further comprising the step of comparing the actual position of the manipulator reference feature (60A, 60B, 60C, 60D) within the known coordinate system to the actual position of each of the reference features (130A, 330A, 330B, 330C, 130B, 330D, 330E, 330F) of the first and the second surfaces within the known coordinate system to define a deviation between the actual position of the manipulator reference feature (60A, 60B, 60C, 60D) and the actual position of each of the reference features (130A, 330A, 330B, 330C, 130B, 330D, 330E, 330F) of the first and the second surfaces (112, 212, 312, 114, 214, 314); and
determining whether the surgical saw blade cartridge (110, 210, 310) is properly mounted to the surgical manipulator (14) based on comparison of the actual position of the manipulator reference feature (60A, 60B, 60C, 60D) to the actual position of each of the reference features (130A, 330A, 330B, 330C, 130B, 330D, 330E, 330F) of the first and the second surfaces (112, 212, 312, 114, 214, 314) based on defined deviation being within an accepted threshold/range.

12. The method of any one of claims 7 to 11, further comprising defining a tool center point of the surgical saw blade cartridge (110, 210, 310) based on the actual position for each of the reference features (130A, 330A, 330B, 330C, 130B, 330D, 330E, 330F) within the known coordinate system.

13. The method of claim 12, further comprising the step of navigating the surgical saw blade cartridge (110, 210, 310) via the navigation system based on the defined tool center point of the surgical saw blade cartridge (110, 210, 310).

14. The method of any one of claims 7 to 11, further comprising defining a plane of the surgical saw blade cartridge (110, 210, 310) based on the actual position for each of the reference features (130A, 330A, 330B, 330C, 130B, 330D, 330E, 330F) within the known coordinate system.

15. The method of any one of claims 7 to 11, further comprising defining a hybrid position for the manipulator reference feature (60A, 60B, 60C, 60D) within the known coordinate system based on a mathematical fitting of the actual position of reference features (130A, 330A, 330B, 330C, 130B, 330D, 330E, 330F) of the surgical saw blade cartridge (110, 210, 310) and the manipulator reference feature (60A, 60B, 60C, 60D); and
wherein the step of defining the hybrid position of the manipulator reference feature (60A, 60B, 60C, 60D) within the known coordinate system based on the mathematical fit is such that the hybrid position of the manipulator reference feature (60A, 60B, 60C, 60D) is different from the actual position of the manipulator reference feature (60A, 60B, 60C, 60D) in only one degree of freedom.
